(19) Europäisches Patentamt
European Patent Office
Office européen des brevets

(11) Veröffentlichungsnummer : **0 349 609 B1**

(12) # EUROPÄISCHE PATENTSCHRIFT

(45) Veröffentlichungstag der Patentschrift :
**05.08.92 Patentblatt 92/32**

(51) Int. Cl.$^5$ : **C07D 307/60,** C07D 407/12, A61K 31/365, A61K 31/40

(21) Anmeldenummer : **88909981.8**

(22) Anmeldetag : **04.11.88**

(86) Internationale Anmeldenummer :
**PCT/DE88/00684**

(87) Internationale Veröffentlichungsnummer :
**WO 89/04310 18.05.89 Gazette 89/11**

(54) **AMINOSÄUREESTER, VERFAHREN ZU IHRER HERSTELLUNG UND IHRE VERWENDUNG.**

(30) Priorität : **04.11.87 DE 3737399**

(43) Veröffentlichungstag der Anmeldung :
**10.01.90 Patentblatt 90/02**

(45) Bekanntmachung des Hinweises auf die Patenterteilung :
**05.08.92 Patentblatt 92/32**

(84) Benannte Vertragsstaaten :
**AT BE CH DE FR GB IT LI NL SE**

(56) Entgegenhaltungen :
**ZA-A- 8 702 867**

(73) Patentinhaber : **Dr. Willmar Schwabe GmbH & Co.**
**Dr. Willmar-Schwabe-Strasse 4**
**W-7500 Karlsruhe 41 (DE)**

(72) Erfinder : **KLESSING, Klaus**
**Rheingoldstrasse 3**
**W-7505 Ettlingen 5 (DE)**
Erfinder : **CHATTERJEE, Shyam, Sunder**
**Stettiner Strasse 1**
**W-7500 Karlsruhe 1 (DE)**

(74) Vertreter : **Bunke, Holger, Dr.rer.nat. Dipl.-Chem. et al**
**Patentanwälte Prinz, Leiser, Bunke & Partner**
**Manzingerweg 7**
**W-8000 München 60 (DE)**

EP 0 349 609 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Ester, deren Säurekomponente ein Aminosäurerest und deren alkoholische Komponente der Rest eines 4-Alkoxy-5-arylhydroxymethyl-2-(5H)-furanons ist, nämlich Aminosäureester der allgemeinen Formel I,

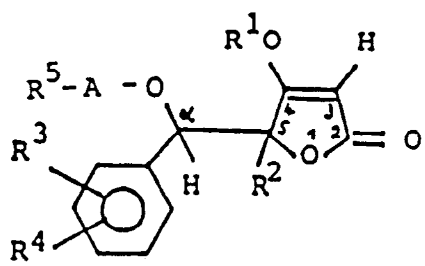

(I)

worin die Sauerstoffatome an C-5 und C-α relativ zueinander die threo-Position einnehmen und worin
$R^1$ eine Methyl- oder Ethylgruppe,
$R^2$ ein Wasserstoffatom oder eine Methylgruppe,
$R^3$ und $R^4$ unabhängig voneinander ein Wasserstoff-, Fluor-, Chlor- oder Bromatom, eine Niederalkylgruppe mit 1 bis 3 C-Atomen, eine Perfluorniederalkylgruppe mit 1 bis 3 C-Atomen, die Difluormethoxy- oder Nitrogruppe,
A den Acylrest einer der folgenden Aminosäuren:

Alanin, Leucin, Isoleucin, Norleucin, Valin, Norvalin, Phenylglycin, Phenylalanin, Prolin, 5-Oxoprolin, Glutaminsäure, Glutamin, Asparaginsäure, Asparagin, Methionin, Glycin, ß-Alanin, 4-Aminobuttersäure, 2-Methylalanin oder eine 1-Amino-1-cycloalkancarbonsäure mit einem 3-7-gliedrigen Cycloalkylrest,

wobei jeweils ein Wasserstoffatom der Aminogruppe dieser Aminosäuren durch den Rest $R^5$ ersetzt ist, und

$R^5$ ein Wasserstoffatom, einen Niederalkanoyl-Rest mit 2 bis 5 C-Atomen, den Benzyloxycarbonyl-Rest (Z) oder den tert.-Butoxycarbonyl-Rest (BOC) bedeuten,
sowie deren pharmakologisch verträgliche Säureadditionssalze.

Die Erfindung betrifft ferner Verfahren zur Herstellung der vorgenannten Aminosäureester, wobei diese durch unterschiedliche Kombination der einzelnen Verfahrensschritte in Form von Racematen, Gemischen von Diastereomeren oder Enantiomeren oder in Form reiner Diastereomere oder Enantiomere, je nach Wunsch, erhalten werden können. (Zur Definition der Begriffe "Racemat", "Diastereomer" und "Enantiomer" vergl. Römpps Chemie-Lexikon, 8. Aufl., Bd. 2, Seite 928/929, Franckh'sche Verlagshandlung, Stuttgart 1981.)

Die Erfindung betrifft ferner Arzneimittel, die mindestens eine der erfindungsgemäßen Verbindungen enthalten, sowie die erfindungsgemäßen Verbindungen zur Vervendung als therapeutische Wirkstoffe und bei der Behandlung von Konvulsionen und Erkrankungen des epileptischen Formenkreises bei warmblütigen Tieren und beim Menschen sowie die Verwendung dieser Verbindungen bei der Herstellung von Antikonvulsiva und Antiepileptika.

Die Verbindungen der allgemeinen Formel I enthalten in ihrer alkoholischen Komponente, also der 5-Arylhydroxymethyl-2(5H)-furanon-Teilstruktur, zwei Asymmetriezentren an den Kohlenstoffatomen C-5 und C-α, bei denen infolge der threo-Konfiguration der angrenzenden Sauerstoffatome zwar die relative Konfiguration festgelegt ist, nicht aber die absolute Konfiguration. Es können daher die beiden folgenden Isomeren der allgemeinen Formeln Ia und Ib auftreten:

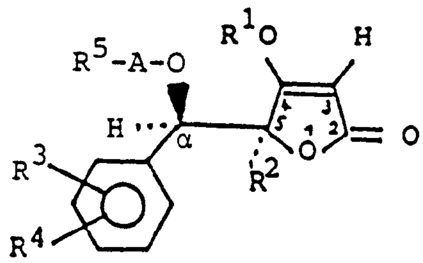

5-S, α-R
Ia

und

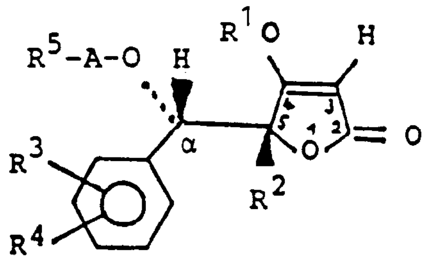

5-R, α-S
Ib

Die entsprechenden erythro-Verbindungen mit 5-S, α-S-bzw. 5-R, α-R-Konfiguration sind nicht Gegenstand dieser Erfindung.

Wenn es sich bei den Verbindungen der allgemeinen Formel I um Ester einer achiralen Aminosäure handelt, so sind die Isomeren Ia und Ib enantiomere Verbindungen. Sind die Verbindungen der allgemeinen Formel I aber Ester einer chiralen L- oder D-Aminosäure, dann tritt (mindestens) ein neues Asymmetriezentrum hinzu, was zu (mindestens) 4 Diastereomeren führt, nämlich zu (L)-Ia und (L)-Ib sowie (D)-Ia und (D)-Ib, je nachdem, ob zur Veresterung eine L- oder D-Aminosäure verwendet wird.

Zum Gegenstand der Erfindung gehören alle Verbindungen der allgemeinen Formeln Ia, Ib, (L)-Ia, (L)-Ib, (D)-Ia und (D)-Ib, sowie die jeweiligen Gemische von Ia mit Ib, von (L)-Ia mit (L)-Ib und von (D)-Ia mit (D)-Ib, nicht jedoch Gemische von L-Aminosäureestern mit D-Aminosäureestern.

Die Verbindungen der allgemeinen Formel I sind neu und zeichnen sich durch antikonvulsive und antiepileptische Wirksamkeit aus.

Es sind bereits eine ganze Reihe von Verbindungen unterschiedlichster chemischer Konstitution mit antikonvulsiver und antiepileptischer Wirksamkeit bekannt (vgl. z.B. Ehrhart/Ruschig, Arzneimittel, Bd. 1, S. 177 ff., Verlag Chemie, Weinheim, 1972), zu denen insbesondere die Wirkstoffe Carbamazepin, Diazepam, Diphenylhydantoin, Ethosuximid, Phenobarbital und Valproinsäure gehören. Alle diese bekannten Antikonvulsiva/Antiepileptika weisen chronisch-toxische Nebenwirkungen in unterschiedlichem Maße auf, zu denen Exantheme, depressive Verstimmungen, Paranoia, Megaloblasten-Anämie, Schädigungen des blutbildenden Knochenmarks, Leberschädigungen und anderes gehören. Viele dieser Wirkstoffe sind in Wasser schlecht löslich; dies führt zu Schwierigkeiten, eine intravenös oder parenteral applizierbare Darreichungsform herzustellen, und bedingt das Risiko einer ungenügenden Bioverfügbarkeit nach oraler Applikation.

Es bestand deshalb ein Bedürfnis nach der Bereitstellung neuer pharmazeutischer Mittel mit antikonvulsiver und antiepileptischer Wirksamkeit und mit gleichzeitig verbesserter Löslichkeit in Wasser und wäßrigen Lösungsmittelsystemen, weil der Arzt nur so in die Lage versetzt wird, aus einem größeren Arzneimittelschatz Mittel auszuwählen, deren Wirkungs- und Nebenwirkungspektren den physischen und psychischen Bedürfnissen des Patienten am ehesten gerecht werden.

Die der Erfindung zugrundeliegende Aufgabe besteht darin, dieses Bedürfnis nach der Bereitstellung neuer, besser wasserlöslicher Verbindungen mit antikonvulsiver/antiepileptischer Wirksamkeit zu befriedigen. Der Erfindung liegt ferner die Aufgabe zugrunde, Verfahren zur Herstellung solcher Verbindungen mit antikonvulsiver/antiepileptischer Wirksamkeit zu schaffen, mit denen es gelingt, stereoselektiv und gezielt die threo-Verbindungen der allgemeinen Formel I in Form ihrer Racemate, Diastereomere, Enantiomere oder ihrer Diastereomeren- oder Enantiomerengemische zu synthetisieren.

Die Lösung dieser Aufgabe besteht in der Schaffung und Bereitstellung der erfindungsgemäßen Stoffe mit antikonvulsiver/antiepileptischer Wirksamkeit sowie der diese Verbindungen enthaltenden Arzneimittel und in der Schaffung der erfindungsgemäßen Verfahren zur Herstellung der neuen Stoffe.

Gegenstand der Erfindung sind insbesondere diejenigen Verbindungen der allgemeinen Formel I, worin die Sauerstoffatome an C-5 und C-α relativ zueinander die threo-Position einnehmen und worin
$R^1$ eine Methylgruppe,
$R^2$ ein Wasserstoffatom,
einer der beiden Reste $R^3$ und $R^4$ ein Wasserstoffatom und der andere ein in 2′-Stellung befindliches Fluor-, Chlor- oder Bromatom oder eine in 2′-Stellung befindliche Methyl- oder Trifluormethylgruppe
oder einer der beiden Reste $R^3$ und $R^4$ ein Fluor-, Chloroder Bromatom oder eine Trifluormethylgruppe, jeweils in 2′-Stellung, und der andere ein Chlor- oder Bromatom oder eine Trifluormethylgruppe, jeweils in 4′-, 5′- oder 6′-Stellung,
A den Acylrest einer der folgenden Aminosäuren:

Glycin, D- oder L-Alanin, 4-Aminobuttersäure, D- oder L-Phenylglycin, D- oder L-Phenylalanin, D- oder L-Prolin, D- oder L-5-Oxoprolin, D- oder L-Glutaminsäure, D- oder L-Glutamin, D- oder L-Methionin, 2-Methylalanin oder 1-Amino-1-cyclohexancarbonsäure,
wobei jeweils ein Wasserstoffatom der Aminogruppe dieser Aminosäuren durch den Rest $R^5$ ersetzt ist, und $R^5$ ein Wasserstoffatom, einen Niederalkanoyl-Rest mit 2 bis 5 C-Atomen, den Benzyloxycarbonyl-Rest (Z) oder den tert.-Butoxycarbonyl-Rest (BOC) bedeuten,
sowie deren pharmakologisch verträgliche Säureadditionssalze.

Wegen ihrer guten antikonvulsiven/antiepileptischen Wirksamkeit besonders bevorzugt sind diejenigen Verbindungen der allgemeinen Formel I, bei denen $R^5$ ein Wasserstoffatom oder den Niederalkanoylrest mit 2 bis 5 C-Atomen bedeutet.

Bevorzugt sind weiterhin diejenigen Verbindungen der allgemeinen Formel I, bei denen der Aminosäurerest, falls chiral, L-Konfiguration besitzt.

Besonders bevorzugt sind schließlich diejenigen Ester der allgemeinen Formel I, deren durch den threo-5-Arylhydroxymethyl-2(5H)-furanon-Rest gebildete alkoholische Komponente, für sich allein gesehen, optisch rechtsdrehend ist, also als (+)-Enantiomer vorliegt.

Gegenstand der Erfindung ist weiterhin ein Verfahren zur Herstellung der Verbindungen der allgemeinen Formel I, das - je nach dem gewünschten Substitutionsmuster bezüglich der Reste $R^1$, $R^2$, $R^3$, $R^4$, $R^5$ und A und je nach der gewünschten Konfiguration der Endprodukte - durch eine oder mehrere der nachfolgend beschriebenen Verfahrensstufen (A) bis (H) gekennzeichnet ist:

(A) Ein racemisches threo-4-Alkoxy-5-arylhydroxymethyl-2(5H)-furanon der allgemeinen Formel II,

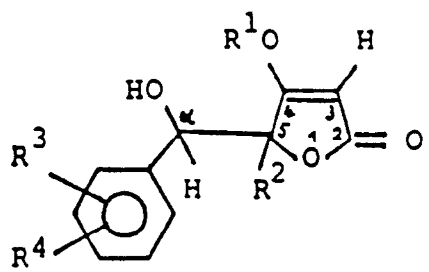

(II)

worin $R^1$, $R^2$, $R^3$, und $R^4$ die im Zusammenhang mit der Erläuterung der allgemeinen Formel I genannten Bedeutungen besitzen, wird mit einem reaktiven Aminosäurederivat der allgemeinen Formel III

$$R^5\text{-A-X} \quad \text{(III)}$$

umgesetzt, worin $R^5$ die im Zusammenhang mit der Erläuterung der Formel I genannten Bedeutungen besitzt oder eine sonstige in der Peptidchemie gebräuchliche, sauer oder hydrogenolytisch abspaltbare Schutzgruppe für Aminogruppen von Aminosäuren bedeutet,
A die im Zusammenhang mit der Erläuterung der allgemeinen Formel I genannte Bedeutung besitzt und
X entweder OH, Cl, Br oder die Gruppe O-A-$R^5$ ist,
wobei die entsprechende Verbindung der allgemeinen Formel I in Form eines Diastereomerengemischs entsteht, worin $R^1$, $R^2$, $R^3$, $R^4$, $R^5$ und A die genannten Bedeutungen besitzen.
Der Rest $R^5$ ist stets an das Stickstoffatom der Aminogruppe der entsprechenden Aminosäure gebunden, d.h. im Falle der Aminosäuren Glutamin und Asparagin nicht an das Stickstoffatom der Amidgruppe.
Die reaktiven Aminosäurederivate der allgemeinen Formel III können an der Aminogruppe durch den Rest $R^5$ substituierte Aminosäuren (X = OH), deren Säurehalogenide (X = Cl, Br) oder deren Säureanhydride (X = O-A-$R^5$) sein. Im Falle des Glycins, beispielsweise, kann es sich also um folgende Verbindungen der allgemeinen Formel III handeln: $R^5$-NH-$CH_2$-CO-OH, $R^5$-NH-$CH_2$-CO-Cl (oder: -Br) oder ($R^5$-NH-$CH_2$-CO)$_2$O.
(B) Falls für die Veresterung in Stufe (A) eine chirale Aminosäure eingesetzt wurde, wird das entstandene Diasteromerengemisch in an sich bekannter Weise in die beiden Diastereomeren der allgemeinen Formeln VIIa und VIIb aufgetrennt:

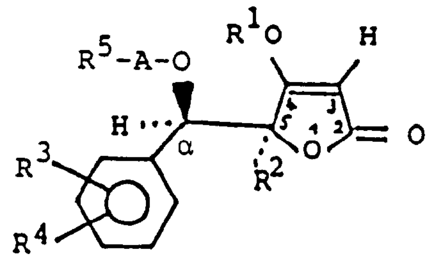

(VIIa)

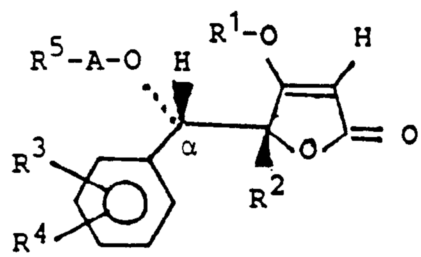

(VIIb)

(C) Der an das Stickstoffatom der Aminogruppe gebundene Rest $R^5$ der in Stufe (A) und/oder (B) erhaltenen Aminosäureester wird acidolytisch oder hydrogenolytisch abgespalten und durch ein Wasserstoffatom ersetzt, wodurch die eine freie Aminogruppe aufweisenden Aminosäureester der allgemeinen Formeln VIII, VIIIa und VIIIb entstehen:

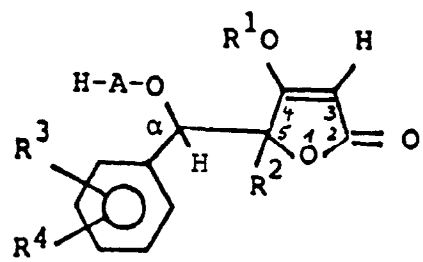

(VIII)

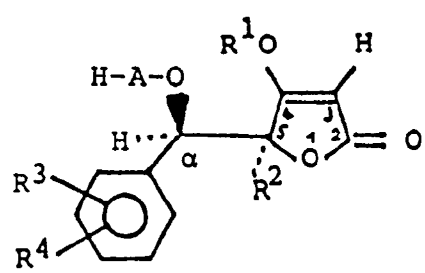

(VIIIa)

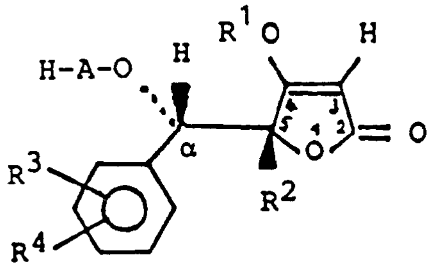

(VIIIb)

(D) Falls in Stufe (A) eine chirale Aminosäure eingesetzt wurde und insbesondere, wenn die Diastereomerentrennung in Stufe (B) nicht oder nicht vollständig durchgeführt wurde, wird das Gemisch der Diastereomeren VIIIa und VIIIb in die Einzelkomponenten aufgetrennt.

(E) Die diastereomeren Ester der allgemeinen Formeln VIIIa und VIIIb werden getrennt voneinander unter Bildung der enantiomeren Furanone der allgemeinen Formeln IIa und IIb

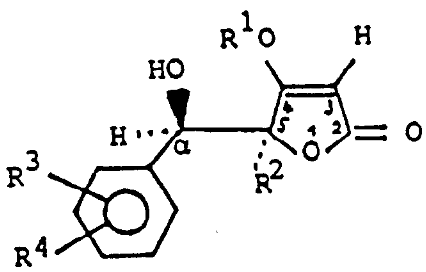

(IIa)

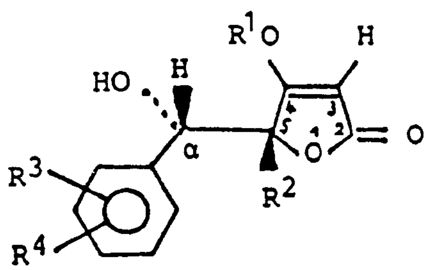

(IIb)

verseift.

(F) Die einzelnen Enantiomeren der allgemeinen Formeln IIa und IIb werden analog zur Stufe (A) mit einem an der Aminogruppe durch den Rest $R^5$ geschützten achiralen Aminosäurederivat der allgemeinen Formel III

$$R^5\text{-A-X} \quad \text{(III)}$$

zu den Verbindungen der allgemeinen Formeln VIIa und VIIb, die in diesem Falle Enantiomere sind, verestert.

(G) Die Verbindungen der allgemeinen Formeln VIIa und VIIb werden analog zur Stufe (C) unter Abspaltung des Restes $R^5$, der durch ein Wasserstoffatom ersetzt wird, zu den eine freie Aminogruppe aufweisenden Aminosäureestern der allgemeinen Formeln VIIIa und VIIIb, die in diesem Falle Enantiomere sind, gespalten.

(H) Die in Stufe (C) oder (G) erhaltenen Verbindungen der allgemeinen Formeln VIII, VIIIa oder VIIIb werden mit einem reaktiven Acylderivat der allgemeinen Formeln

$$R^5\text{-Y} \qquad \text{oder} \qquad R^5\text{-O-}R^5$$

umgesetzt, worin Y ein Chlor- oder Bromatom und $R^5$ einen Niederalkanoylrest mit 2 bis 5 C-Atomen bedeuten, oder mit einem Keten der Formel $R^7{=}C{=}O$ umgesetzt, worin $R^7$ ein Alkylidenrest mit 1 bis 4 C-Atomen ist, wodurch Verbindungen der allgemeinen Formeln I, Ia oder Ib, worin $R^5$ einen Niederalkanoylrest mit 2 bis 5 C-Atomen bedeutet, entstehen.

Das erfindungsgemäße Verfahren kann schematisch durch das folgende Fließbild dargestellt werden, in welchem die römischen Zahlen die Verbindungen gemäß den entsprechend gekennzeichneten allgemeinen Formeln bezeichnen und die in Klammern stehenden Versalien den entsprechend benannten Verfahrensstufen entsprechen:

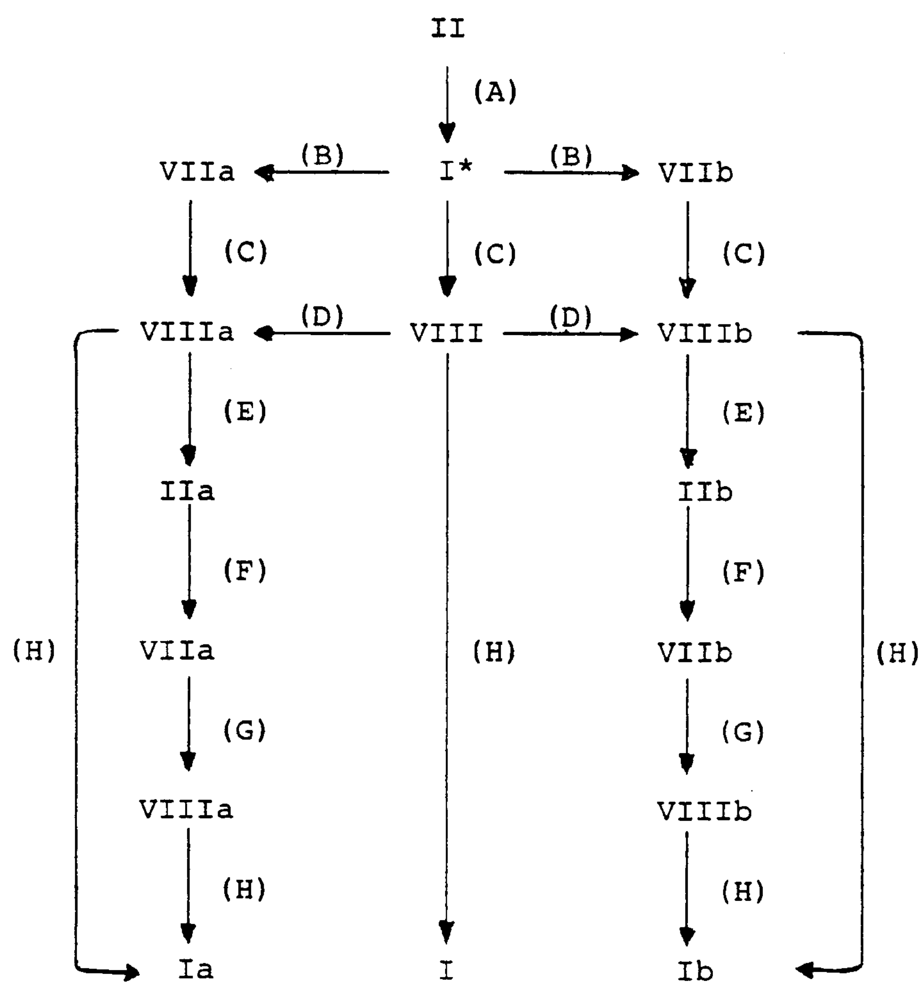


I* = Verbindungen der allgemeinen Formel I, worin $R^5 \neq H$.

Das erfindungsgemäße Verfahren erlaubt die Herstellung aller Verbindungen, die unter die allgemeine Formel I fallen, und zwar wahlweise in Form von Racematen, reinen Diastereomeren oder Enantiomeren oder in Form von Gemischen von Diastereomeren oder Enantiomeren, je nach der Kombination der gewählten Verfahrensstufen. Nachfolgend werden die unterschiedlichen Kombinationsmöglichkeiten der Stufen (A) bis (H) zur Herstellung der jeweils gewünschten Verbindungen ("Zielverbindungen") der allgemeinen Formel I näher erläutert:

Fall 1:

Zielverbindung ist ein Racemat oder ein Diastereomerengemisch einer Verbindung der allgemeinen Formel I, worin $R^1$, $R^2$, $R^3$, $R^4$, $R^5$ und A die eingangs beschriebenen Bedeutungen haben. Zur Herstellung genügt die Anwendung entweder

a) nur der Stufe (A), wenn solche N-geschützte Aminosäurederivate $R^5$-A-X der allgemeinen Formel III verwendet werden, die als Schutzgruppe bereits denjenigen Rest $R^5$ besitzen, der auch für die Zielverbindung

vorgesehen ist, mit der Einschränkung, daß $R^5$ nicht Wasserstoff ist ($R^5 \neq H$); oder
b) nur der Stufen (A) und (C), wenn bei der Zielverbindung $R^5 = H$; oder
c) nur der Stufen (A), (C) und (H), wenn bei der Zielverbindung der Rest $R^5$ nicht Wasserstoff und von dem in Stufe (A) als Aminoschutzgruppe verwendeten Rest $R^5$ verschieden ist.

Fall 2:

Zielverbindungen sind die einzelnen Diastereomeren der allgemeinen Formeln Ia und Ib, worin $R^1$, $R^2$, $R^3$, $R^4$, $R^5$ die eingangs beschriebenen Bedeutungen besitzen und A der Acylrest einer der eingangs beschriebenen chiralen D- oder L-Aminosäuren ist. Zur Herstellung genügt die Anwendung entweder
a) der Stufen (A) und (B), wenn $R^5 \neq H$, oder
b) der Stufen (A), (B) und (C) oder der Stufen (A), (C) und (D), wenn $R^5 = H$, oder
c) der Stufen (A), (B), (C) und (H) oder der Stufen (A), (C), (D) und (H), wenn $R^5 \neq H$ und $R^5$ von dem in Stufe (A) als Aminoschutzgruppe verwendeten Rest $R^5$ verschieden ist.

Fall 3:

Zielverbindungen sind die einzelnen Enantiomeren der allgemeinen Formeln Ia und Ib, worin $R^1$, $R^2$, $R^3$, $R^4$ und $R^5$ die eingangs beschriebenen Bedeutungen haben und A der Acylrest einer der eingangs beschriebenen achiralen Aminosäuren ist. Zur Herstellung genügt die Anwendung entweder
a) der Stufen (A), (B), (C), (E) und (F) oder der Stufen (A), (C), (D), (E) und (F), wenn in Stufe (F) ein Aminosäurederivat der allgemeinen Formel III eingesetzt wird, bei dem $R^5 \neq H$, oder
b) der Stufen (A), (B), (C), (E), (F) und (G) oder der Stufen (A), (C), (D), (E), (F) und (G), wenn $R^5 = H$, oder
c) der Stufen (A), (B), (C), (E), (F), (G) und (H) oder der Stufen (A), (C), (D), (E), (F), (G) und (H), wenn $R^5 \neq H$ und von dem in Stufe (F) als Aminoschutzgruppe verwendeten Rest $R^5$ verschieden ist.

Bei allen Ausführungsformen des Falles 3 muß in Stufe (A) ein chirales D- oder L-Aminosäurederivat der allgemeinen Formel III eingesetzt werden, um über die Stufen (B) bis (E) eine Racematspaltung unter Bildung der Verbindungen der allgemeinen Formeln IIa und IIb durchführen zu können; die in der Zielverbindung enthaltene achirale Aminosäure kann erst in Stufe (F) des Verfahrens eingeführt werden, also erst dann, wenn die Furanone der allgemeinen Formeln IIa und IIb bereits in enantiomerer Form vorliegen und voneinander getrennt sind.

Fall 4:

Zielverbindungen sind diejenigen Verbindungen der allgemeinen Formel I, worin $R^1$, $R^2$, $R^3$ und $R^4$ die eingangs beschriebenen Bedeutungen besitzen, $R^5 = H$ und A den Acylrest des D- oder L-5-Oxoprolins bedeutet. Zur Herstellung des Diastereomerengemischs der Zielverbindung genügt die Anwendung der Stufe (A), wobei als Aminosäure D- oder L-5-Oxoprolin oder eines seiner reaktiven Derivate der allgemeinen Formel III ohne Schutzgruppe ($R^5 = H$) zur Veresterung eingesetzt wird. Zur Isolierung der einzelnen Diastereomeren Ia und Ib werden anschließend die Stufen (B) und/oder (D) durchgeführt.

Die einzelnen Verfahrensstufen (A) bis (H) werden, soweit erforderlich, nachstehend noch weiter erläutert.

Stufe A:

Die am Amin-Stickstoffatom geschützten Aminosäurederivate der allgemeinen Formel III werden mit den threo-4-Alkoxy-5-arylhydroxymethyl-2(5H)-furanonen der allgemeinen Formel II verestert, und zwar vorzugsweise in Gegenwart eines aliphatischen Carbodiimids wie N,N′-Dicyclohexylcarbodiimid als Kondensationsmittel und vorzugsweise in Gegenwart eines 4-Dialkylaminopyridins wie 4-Dimethylamino- oder 4-Pyrrolidinopyridin als Acylierungskatalysator, vorzugsweise in einem wasserfreien aprotischen Lösungsmittel, z.B. einem niederen Halogenkohlenwasserstoff wie Chloroform oder Dichlormethan, oder einem niederen aliphatischen Dialkylether oder cyclischen Ether, oder Acetonitril oder Dimethylformamid, bei Temperaturen zwischen -30 und +30°C, vorzugsweise zwischen -10 und +10°C.

Diese Verfahrensweise eignet sich besonders zur racemisierungsfreien Veresterung von chiralen D- oder L-Aminosäuren oder deren Derivaten der allgemeinen Formel III. Es können jedoch auch andere aus der Peptidchemie geläufige Kondensationsmittel verwendet werden, insbesondere dann, wenn achirale Aminosäuren oder deren Derivate der allgemeinen Formel III zur Veresterung eingesetzt werden. Die verwendeten threo-4-Alkoxy-5-arylhydroxymethyl-2(5H)-furanone der allgemeinen Formel II werden im allgemeinen als Racemate

eingesetzt.

Besonders bevorzugte Verbindungen der allgemeinen Formel II sind: threo-5-(2-Fluorphenylhydroxymethyl)-, threo-5-(2-Chlorphenylhydroxymethyl)-, threo-5-(2-Bromphenylhydroxymethyl)-, threo-5-(2-Trifluormethylphenylhydroxymethyl)-, threo-5-(2-Methylphenylhydroxymethyl)-, threo-5-(2,4-Dichlorphenylhydroxymethyl)- und threo-5-(2,5-Dichlorphenylhydroxymethyl)-4-methoxy-2(5H)-furanon.

Als Aminosäuren werden für die Veresterung in Stufe (A) vorzugsweise Alanin, Phenylalanin, Phenylglycin, Prolin, 5-Oxoprolin, Glutaminsäure, Glutamin, Methionin, Glycin, 2-Methylalanin, 1-Amino-1-cyclohexancarbonsäure und 4-Aminobuttersäure oder deren reaktive Derivate der allgemeinen Formel III eingesetzt. Wie bereits erwähnt, kann 5-Oxoprolin auch ohne Schutzgruppe $R^5$ eingesetzt werden. Wenn der Rest $R^5$ ein Niederalkanoylrest mit 2 bis 5 C-Atomen ist, handelt es sich nicht um eine "Schutzgruppe", sondern um einen Acylrest, der sich nicht sauer oder hydrogenolytisch abspalten läßt.

Aus der Gruppe der chiralen Aminosäuren sind die jeweiligen L-Formen besonders bevorzugt.

Als andere, in der Peptidchemie gebräuchliche, sauer oder hydrogenolytisch abspaltbare Schutzgruppen für Aminogruppen von Aminosäuren können z.B. 3,5-Dimethoxyphenylisopropyloxycarbonyl (Ddz), 2-(4-Biphenyl)-isopropyloxycarbonyl (Bpoc), 2-Furylmethyloxycarbonyl (Foc), p-Brombenzyloxycarbonyl [Z(Br)] oder p-Methoxybenzyloxycarbonyl [Z(OMe)] verwendet werden.

Die derartig geschützten Aminosäurederivate der allgemeinen Formel III sind entweder bekannt oder können nach literaturbekannten Methoden hergestellt werden (vgl. O. Keller et al., Org. Syntheses 63, 160 (1985); W.J. Paleveda et al., Org. Syntheses 63, 171 (1985); W. Grassmann und E. Wünsch, Chem. Ber. 91, 462 (1958); E. Wünsch in "Methoden der organischen Chemie", Bd. XV/1, Georg Thieme Verlag, Stuttgart, 1974. Bevorzugte Schutzgruppen sind BOC und Z.

Stufe (B):

Setzt man in Stufe (A) eine chirale D- oder L-Aminosäure zur Veresterung der racemischen threo-5-Arylhydroxymethyl-4-alkoxy-2(5H)-furanone ein, so erhält man ein Gemisch der diastereomeren Ester VIIa und VIIb. Diese können durch fraktionierte Kristallisation aus Lösungsmitteln oder Lösungsmittelgemischen getrennt werden. Geeignete Lösungsmittel sind niedere Alkohole mit 1 bis 5 C-Atomen, z.B. Methanol, Ethanol, Isopropanol, Butanol, niedere aliphatische oder cycloaliphatische Ether, z.B. Diethylether, tert.-Butylmethylether und Tetrahydrofuran, niedere aliphatische Ketone, z.B. Aceton oder Butanon, oder niedere aliphatische Carbonsäureester, jeweils allein oder in Mischungen miteinander oder in Mischungen mit Kohlenwasserstoffen mit 5 bis 10 C-Atomen, z.B. Pentan, Hexan, Heptan und Cyclohexan.

Zur Trennung löst man das Gemisch in einem der oben angeführten Lösungsmittel in der Wärme, kühlt ab, läßt bei Temperaturen zwischen -20 und +30°C stehen und trennt das zunächst ausfallende, schwerer lösliche Diastereomere durch Filtration oder Zentrifugation ab. Man kann auch in der Kälte oder bei Raumtemperatur lösen und vor dem Stehenlassen die Lösung durch Verdampfen eines Teils des Lösungsmittels konzentrieren, um so die Kristallisation des schwerer löslichen Diastereomeren zu erreichen. Das nach Abtrennen des Kristallisats verbleibende Filtrat wird weiter konzentriert, stehengelassen und erneut filtriert, um eine möglichst quantitative Abtrennung des schwerer löslichen Diastereomeren zu gewährleisten. Das leichter lösliche Diastereomere wird durch Eindampfen der Mutterlaugen und Umkristallisation aus einem zweiten Lösungsmittel oder Lösungsmittelgemisch gewonnen oder stark angereichert.

Beispielsweise verwendet man zunächst Ethanol als Lösungsmittel zur Abtrennung des ersten Diastereomeren und reinigt das andere in den Mutterlaugen verbliebene Diastereomere durch Umkristallisation aus Isopropanol oder Butanol, gegebenenfalls unter Zumischung von z.B. Pentan, Hexan oder tert.-Butylmethylether.

Die oben beschriebenen Reinigungsoperationen können, je nach gewünschter Diastereomeren-Reinheit, beliebig oft wiederholt werden. Die Reinheit läßt sich in bekannter Weise durch NMR-Spektroskopie, dünnschichtchromatographisch, durch Hochdruckflüssigkeitschromatographie, Polarimetrie oder durch vergleichbare übliche Methoden überprüfen.

Statt mittels fraktionierter Kristallisation kann man die Diastereomerentrennung auch mit Hilfe chromatographischer Methoden in an sich bekannter Weise, beispielsweise durch Säulenchromatographie mit z.B. Chloroform/Methanol oder Toluol/Aceton als Eluens an Kieselgel, durchführen.

Stufe (C):

Die Abspaltung der Schutzgruppe $R^5$ aus den Verbindungen der allgemeinen Formeln I, VIIa oder VIIb kann entweder acidolytisch oder bei Schutzgruppen, die der Arylmethyloxycarbonyl-Reihe angehören, auch durch Hydrogenolyse in Gegenwart eines Katalysators erfolgen.

Für die Acidolyse geeignete Säuren sind Salzsäure, Brom- oder Jodwasserstoffsäure, Essigsäure, Trifluoressigsäure und Ameisensäure. Art und Konzentration der jeweiligen Säure werden so gewählt, daß eine selektive Abspaltung der Schutzgruppe stattfindet, ohne die Esterbindung zu gefährden. Üblicherweise führt man die Spaltung in einem Lösungsmittel durch. Geeignete Lösungsmittel sind niedere Alkancarbonsäureester, beispielsweise Ethylacetat, Propylacetat, Ethylpropionat oder dergleichen, sowie die bei Stufe (B) beschriebenen Ether.

Die Lösungen der Verbindungen I, VIIa oder VIIb in dem jeweiligen Lösungsmittel werden nach Versetzen mit der zur Spaltung benötigten Menge Säure bei Temperaturen zwischen -20 und +40°C stehengelassen, bis die Acidolyse vollständig abgelaufen ist, und die Verbindungen werden in Form der entsprechenden Säureadditionssalze isoliert. Besonders bevorzugt sind Lösungen in Ethylacetat, denen eine etwa 1- bis 4-fach molare Menge, bezogen auf den Ester der allgemeinen Formel I mit $R^5 \neq H$, einer Lösung von Brom- oder Chlorwasserstoff in Eisessig zugefügt wird. Man erhält die gewünschten Verbindungen der allgemeinen Formeln VIII, VIIIa oder VIIIb in Form gut kristallisierender Hydrochloride oder Hydrobromide, die gewünschten- falls durch Umkristallisation aus einem niederen Alkohol, Ether, Ester oder Gemischen derselben, gegebenenfalls unter Zusatz eines Kohlenwasserstoff als Lösungsmittel gereinigt werden können.

Alternativ wird die Abspaltung der Schutzgruppe $R^5$ durch Hydrogenolyse der Verbindungen der allgemeinen Formeln I mit $R^5 \neq H$, VIIa oder VIIb, bei denen $R^5$ beispielsweise Z, Z(Br), Z(OMe), Ddz, Bpoc oder Foc bedeutet, in an sich bekannter Weise, vorzugsweise unter Verwendung eines Palladiumkatalysators, in einem geeigneten Lösungsmittel wie z.B. Ethylacetat, Dioxan, Ethanol oder Isopropanol bewirkt. Die Verbindungen der allgemeinen Formeln VIII, VIIIa oder VIIIb werden in Form ihrer freien Basen isoliert und gewünschtenfalls in ein Säureadditionssalz übergeführt, beispielsweise in das entsprechende Hydrochlorid, Hydrobromid, Sulfat, Hydrogensulfat, Methansulfonat, Fumarat, Maleat, Benzoat, Citrat oder dergleichen.

Stufe (D):

Falls die Ester chiraler D- oder L-Aminosäuren nicht bereits in Stufe (B) in die Diastereomeren aufgetrennt wurden oder die Trennung unvollständig war, trennt man die freien Aminosäureester der Formeln VIIIa und VIIIb geeigneterweise in Form ihrer oben genannten Säureadditionssalze in die Diastereomeren auf. Die Trennung erfolgt analog zur Stufe (B) durch fraktionierte Kristallisation oder auf chromatographischem Wege. Besonders geeignete Kristallisationsmedien sind niedere Alkohole, beispielsweise Methanol, Ethanol, Propanol, Isopropanol oder Butanol, aliphatische oder cyclische Ether, niedere Ketone, wobei die Verbindungen VIIIa oder VIIIb auch in Form der mit den Ketonen gebildeten Azomethine oder Enamine anfallen können, und niedere Alkancarbonsäureester, gegebenenfalls unter Zusatz von Kohlenwasserstoffen mit 5 bis 10 C-Atomen.

Stufe (E):

Zur Herstellung von Estern der allgemeinen Formeln Ia oder Ib, bei denen der Aminosäurerest achiral ist, also von Estern des Glycins, ß-Alanins, 2-Methylalanins, der 4-Aminobuttersäure oder der 1-Amino-1-cycloalkancarbonsäuren benötigt man als Ausgangsprodukte die enantiomeren reinen threo-5-Arylhydroxymethyl-4-alkoxy-2(5H)-furanone der allgemeinen Formeln IIa oder IIb. Diese werden durch sauer oderalkalisch katalysierte Spaltung der Ester der allgemeinen Formeln VIIIa oder VIIIb erhalten. Bevorzugte Ester sind dabei Ester des D- oder L-Prolins und des D- oder L-Phenylglycins, die eine besonders effektive Trennung der Diastereomeren VIIa und VIIb in Stufe (B) des Verfahrens oder der Diastereomeren VIIIa und VIIIb in Stufe (D) des Verfahrens erlauben.

Die Spaltung der Ester VIIIa bzw. VIIIb kann sauer oder basisch katalysiert erfolgen. Vorzugsweise werden dabei folgende Bedingungen eingehalten:

Saure Spaltung:

Die Verbindungen der allgemeinen Formeln VIIIa bzw. VIIIb werden in wäßriger Lösung oder Suspension, gegebenenfalls im Gemisch mit niederen Alkoholen, unter Zufügen mindestens äquivalenter Mengen von Chlor- oder Bromwasserstoffsäure, Schwefel- oder Phosphorsäure, einer Sulfonsäure, beispielsweise Methan- oder p-Toluolsulfonsäure, oder einer starken organischen Säure wie Oxalsäure auf Temperaturen zwischen 50 und 120°C, vorzugsweise zwischen 70 und 110°C, erhitzt, bis die Spaltung vollständig ist.

Alkalisch katalysierte Spaltung (Umesterung):

Lösungen oder Suspensionen der Verbindungen VIIIa bzw. VIIIb in einem niederen wasserfreien Alkohol, vorzugsweise Methanol oder Ethanol, werden mit einem wasserfreien Alkalicarbonat, vorzugsweise Lithiumcarbonat, versetzt und bei Temperaturen zwischen -20 und +40°C, vorzugsweise zwischen 0 und 20°C, gerührt, bis die Spaltung vollständig ist.

Stufe (F):

Die in Stufe (E) erhaltenen Enantiomeren der Formeln IIa bzw. IIb werden analog zur Stufe (A) mit den an der Aminogruppe durch den Rest $R^5$ substituierten Aminosäurederivaten der allgemeinen Formel III von Glycin, ß-Alanin, 2-Methylalanin, 4-Aminobuttersäure oder 1-Amino-1-cycloalkancarbonsäuren zu den Verbindungen der allgemeinen Formeln VIIa bzw. VIIb umgesetzt, wobei VIIa und VIIb optische Antipoden sind.

Stufe (G):

Die in Stufe (F) erhaltenen Verbindungen VIIa bzw. VIIb können, falls gewünscht, analog zur Stufe (C) zu den freien Aminosäureestern der Formeln VIIIa bzw. VIIIb, die ebenfalls optische Antipoden darstellen, gespalten werden. Die Reaktionsbedingungen sind analog. Die Verbindungen der allgemeinen Formeln VIIIa bzw. VIIIb werden ebenfalls vorzugsweise in Form ihrer Säureadditionssalze isoliert.

Stufe (H):

In einigen Fällen wird es vorgezogen, zur Herstellung der Verbindungen der allgemeinen Formeln I, Ia oder Ib, worin $R^1$, $R^2$, $R^3$, $R^4$, $R^5$ und A die im Zusammenhang mit der Erläuterung der allgemeinen Formel I beschriebenen Bedeutungen besitzen, mit der Einschränkung, daß $R^5$ nur Niederalkanoyl mit 2 bis 5 C-Atomen bedeutet, zunächst diejenigen Verbindungen der allgemeinen Formeln VIIa und/oder VIIb zu synthetisieren, bei denen $R^5$ eine andere Bedeutung als Niederalkanoyl besitzt, beispielsweise BOC, Z, Foc oder Bpoc ist. Hieraus können nach Abspaltung der Schutzgruppe analog zur Stufe (C) oder (G) aus den dann vorliegenden Verbindungen der allgemeinen Formeln VIII, VIIIa oder VIIIb durch Acylierung mit einem reaktiven Niederalkanoyl-Derivat der allgemeinen Formeln $R^5$-Y oder $R^5$-O-$R^5$, worin $R^5$ Niederalkanoyl mit 2 bis 5 C-Atomen, vorzugsweise Acetyl, und Y ein Chlor- oder Bromatom bedeuten, die gewünschten Verbindungen der Formeln I, Ia oder Ib erhalten werden.

Die Acylierung wird in einem aprotischen Lösungsmittel, vorzugsweise einem niederen Halogenkohlenwasserstoff, z.B. Dichlormethan oder Chloroform, oder in einem aliphatischen oder cycloaliphatischen Ether in Gegenwart einer Hilfsbase, vorzugsweise Pyridin oder Triethylamin, gegebenenfalls in Gegenwart eines Acylierungskatalysators, vorzugsweise 4-Dimethylamino- oder 4-Pyrrolidinopyridin, bei Temperaturen zwischen -30 und +50°C, vorzugsweise zwischen 0 und 30°C, durchgeführt. Es können jedoch auch andere, dem Fachmann geläufige Acylierungsverfahren angewandt werden, beispielsweise die Acylierung mit einem Keten der Formel $R^7$=C=O, worin $R^7$ eine Alkylidengruppe mit 1 bis 4 C-Atomen bedeutet.

Wie bereits erwähnt, gehören zum Gegenstand der Erfindung ferner Arzneimittel, die gegebenenfalls zusammen mit einem pharmazeutisch inerten Excipiens eine oder mehrere der Verbindungen der allgemeinen Formel I enthalten.

Diese Arzneimittel und pharmazeutischen Zubereitungen können als Antikonvulsiva in der Human- und Veterinärmedizin und als Antiepileptika in der Humanmedizin verwendet werden. Die wirksame Dosis, in der die Verbindungen verabreicht werden können, liegt sowohl in der Humanmedizin als auch in der Veterinärmedizin zwischen etwa 0,05 und 10 mg/kg. Es können 5 bis 500 mg der Wirksubstanz ein- oder mehrmals täglich appliziert werden. Als pharmakologisch inerte, übliche Träger- und Zusatzstoffe können beispielsweise Wasser, pflanzliche Öle, Polyethylenglycole, Glycerinester, Gelatine, Kohlenhydrate wie Lactose oder Stärke, Magnesiumstearat, Talk, Vaseline, Konservierungsmittel, Stabilisatoren, Gleitmittel, Netzmittel, Emulgatoren, physiologisch unbedenkliche Salze, Puffersubstanzen, Farbstoffe, Geschmacksstoffe und Aromastoffe verwendet werden. Die Auswahl des pharmakologisch inerten Excipiens hängt davon ab, ob die Wirkstoffe enteral, parenteral oder topikal appliziert werden sollen. Die Verbindungen können auch im Gemisch mit anderen Wirkstoffen, beispielsweise Vitaminen oder anderen bekannten Antikonvulsiva oder Antiepileptika, verabreicht werden.

Jede der in den nachfolgenden Beispielen genannten erfindungsgemäßen Verbindungen stellt ein zur Herstellung pharmazeutischer Zubereitungen besonders geeignetes Mittel dar.

Bezüglich der Beispiele ist folgendes zu beachten:

Temperaturen sind in °C angegeben und unkorrigiert. Drücke sind in mbar angegeben, wobei 1 mbar ≙ $10^2$ Pa. Optische Drehungen sind als spezifische Drehungen [α], gemessen bei der Wellenlänge λ = 489 nm der D-Linie des Natriumspektrums und, falls nicht anders angegeben, bei 20°C. In Klammern sind die Konzentration c[g/100 ml] und das verwendete Lösungsmittel angegeben. Die $^1H$ und $^{13}C$-Kernresonanzspektren

wurden in DMSO-$D_6$ aufgenommen, soweit nicht anders angegeben. Die chemischen Verschiebungen $\delta$ sind relativ zum Tetramethylsilan-Signal (innerer Standard) in ppm angegeben. Kopplungskonstanten J sind in Hertz (Hz) angegeben. Folgende Abkürzungen werden zur Charakterisierung der Multiplizitäten verwendet: s = singulett, d = dublett, t = triplett, q = quartett, m = multiplett, br. = breit oder verbreitert. Ferner wurden folgende Abkürzungen verwendet: Kp = Siedepunkt, Schmp. = Schmelzpunkt.

Bei der Trennung von Diastereomeren werden die L-Aminosäureester der optisch rechtsdrehenden threo-5-Arylhydroxymethyl-2(5H)-furanone sowie die D-Aminosäureester der entsprechenden linksdrehenden -2(5H)-furanone als Diastereomere A bezeichnet, während die L-Aminosäureester linksdrehender -2(5H)-furanone sowie die D-Aminosäureester der entsprechenden rechtsdrehenden -2(5H)-furanone als Diastereomere B bezeichnet sind.

Die dünnschichtchromatographische Ermittlung von Rf-Werten erfolgte an Kieselgel (DC-Fertigplatten), bei 10 cm Laufhöhe des Elutionsmittels. Die Sichtbarmachung der Substanzflecken erfolgte durch Fluoreszenz-Löschung bei 254 nm, Anfärbung mit Joddampf oder mit Ninhydrin-Sprühreagenz.

Die als Ausgangsmaterial benötigten racemischen threo-4-Alkoxy-5-arylhydroxymethyl-2(5H)-furanone der allgemeinen Formel II können beispielsweise nach dem aus der deutschen Patentanmeldung P 36 15 157.2 bekannten Verfahren oder auf analoge Weise hergestellt werden. Der gesamte Inhalt der genannten deutschen Patentanmeldung wird durch die hiermit erfolgte Bezugnahme in die vorliegende Beschreibung aufgenommen.

Die folgenden Beispiele Nr. 1 bis 101 sind Referenzbeispiele für die Herstellung der Ausgangsprodukte der allgemeinen Formel II. Die Verbindungen gemäß den Beispielen Nr. 30 bis 32, 36, 62, 63, 90 und 91 sind neu. Die Beispiele Nr. 102 bis 141 und 150 bis 157 betreffen erfindungsgemäße Aminosäureester. Die Beispiele Nr. 142 bis 149 betreffen optisch aktive threo-5-Arylhydroxymethyl-4-methoxy-2(5H)-furanone der allgemeinen Formeln IIa und IIb, die als reaktive Zwischenprodukte zur Herstellung der erfindungsgemäßen Aminosäureester verwendet werden können.

Beispiel Nr. 1

Ethyl-3-methoxy-2(E)-butenoat :

Das Gemisch aus 520 g (4 Mol) Ethylacetoacetat, 400 ml Methanol und 2 ml 36 %iger Salzsäure (oder 1 ml konz. Schwefelsäure) wird auf 50°C erwärmt. Unter Rühren tropft man 425 g (4 Mol) Trimethylorthoformiat so zu, daß die Mischung bei ca. 50°C gehalten wird. Anschließend destilliert man gebildetes Methylformiat und überschüssiges Methanol ab und fraktioniert den Rückstand über eine Vigreux-Kolonne. Zwischen 184°C und 186°C destillieren 548 g (3.8 Mol) reines Ethyl-3-methoxy-2(E)-butenoat über.Ausbeute : 95 %.

Beispiel Nr. 2

Ethyl-3-ethoxy-2(E)-butenoat :

Herstellung analog Beispiel Nr. 1 durch Umsetzung von Ethylacetoacetat mit Triethylorthoformiat in Ethanol mit Salzsäure als Katalysator. Ausbeute : 84.5 %.
Kp = 191° - 195°C. Schmp. = 31° - 33°C.

Beispiel Nr. 3

Methyl-3-methoxy-2(E)-pentenoat :

Herstellung analog Beispiel Nr. 1 durch Umsetzung von Methyl-3-oxopentanoat mit Trimethylorthoformiat in Methanol und Schwefelsäure als Katalysator. Ausbeute : 87.7 %. Kp = 76 - 78°C (20 mbar).

Beispiel Nr. 4

3-Methoxy-5-phenyl-2(E),4(E)-pentadiensäure :

Methode a :

Unter Rühren und Stickstoffatmosphäre werden zu der Mischung aus 53 g (0.5 Mol) Benzaldehyd, 100 ml Dimethylsulfoxid und 72 g (0.5 Mol) Ethyl-3-methoxy-2(E)-butenoat 11.6 g (0.05 Mol) Benzyltriethylammoniumchlorid zugefügt und danach die Lösung von 33.6 g (0.6 Mol) Kaliumhydroxid in 35 ml Wasser zugetropft.

Man erwärmt ca. 16 Stdn. lang auf 110°C, evaporiert die Lösung, löst den Rückstand in 400 ml Wasser und extrahiert Ausgangs - und Nebenprodukte mit 100 ml Dichlormethan. Aus der wäßrigen Phase fällt nach Ansäuern mit 70 ml 10M-Salzsäure das Rohprodukt aus, das nach Absaugen, säurefrei Waschen mit Wasser, Umkristallisation aus Ethanol und Trocknen bei 100°C im Vakuum 34.9 g (0.171 Mol) Reinprodukt mit Schmp. = 154 - 155°C ergibt. Ausbeute : 34.2 %.

Literatur-Schmp. = 157,5 - 158°C [E. E. Smissman und A. N. Voldeng, J. Org. Chem. 29, 3161 (1964)].

| | |
|---|---|
| Analyse : | $C_{12}H_{12}O_3$ (204.23) |
| Ber. : | C (70.57), H (5.92) |
| Gef. : | C (70.49), H (6.16) |

Methode b :

Unter Rühren und Stickstoffatmosphäre werden zu der Lösung von 3785 g (26.25 Mol) Ethyl-3-methoxy-2(E)-butenoat in 6 Litern Dimethylsulfoxid nacheinander 2653 g (25 Mol) Benzaldehyd und 926 ml (2.5 Mol) 40 %iges wäßriges Tetraethylammoniumhydroxid zugetropft. Nach Erhitzen auf 100°C tropft man die Lösung von 1470 g (26.25 Mol) Kaliumhydroxid in 1500 ml Wasser zu und rührt 4 Stdn. bei 100°C. Nach Abkühlen auf ca. 20°C gießt man die Mischung in 50 Liter Wasser und extrahiert Verunreinigungen mit 10 Litern Dichlormethan. Die Wasserphase wird unter kräftigem Rühren mit ca. 3 Litern 33 %iger Salzsäure auf pH = 2 angesäuert und das ausgefällte Rohprodukt über einen Druckfilter abfiltriert, mit Wasser chloridfrei gewaschen und mit Stickstoff trocken geblasen. Der Filterkuchen wird in 6 Litern Ethanol suspendiert, erneut filtriert und nach Trockenblasen im Vakuum bis zur Endtemperatur von 85°C getrocknet. Man erhält 3071 g (15.04 Mol) Reinprodukt mit Schmp. 159° - 160°C. Ausbeute : 60.1 %.

Beispiel Nr. 5

5-(2-Chlorphenyl)-3-methoxy-2(E),4(E)-pentadiensäure :

3785 g (26.25 Mol) Ethyl-3-methoxy-2(E)-butenoat werden analog Beispiel Nr. 4 b in 6 Litern Dimethylsulfoxid mit 3515 g (25 Mol) 2-Chlorbenzaldehyd, 926 ml (2.5 Mol) 40 %igem Tetraethylammoniumhydroxid und 2940 g (26.25 Mol) 50 %iger Kalilauge umgesetzt und 4 Stdn. bei 100°C nachgerührt. Nach dem Abkühlen verdünnt man mit 10 Litern Wasser und extrahiert Nebenprodukte mit 10 Litern Dichlormethan. Die wäßrige Phase wird in die Mischung aus 3 Litern 33 %iger Salzsäure und 50 Litern Wasser eingerührt, wonach der pH-Wert 3,5 bis 4 beträgt. Das ausgefällte Rohprodukt wird nach Filtrieren, Waschen mit Wasser bis zur Chloridfreiheit und Trockenblasen in 20 Litern Ethanol suspendiert, erneut abgesaugt, trockengeblasen und bis zur Endtemperatur von 85°C bei 20 mbar getrocknet. Man erhält 5045 g (21.14 Mol) Reinprodukt mit Schmp. 202°C. Ausbeute : 84.55 %.

| | |
|---|---|
| Analyse : | $C_{12}H_{11}ClO_3$ (238.67) |
| Ber. : | C (60.39), H (4.65), Cl (14.85) |
| Gef. : | C (60.33), H (4.85), Cl (14.72) |
| 300 MHz-$^1$H-NMR : | 11.8 - 12.2 (1 H, br.m, COOH), |
| | 8.08 (1 H, d, $J_{5/4}$ = 16 Hz, H-5), |
| | 7.54 (1 H, d, $J_{4/5}$ = 16 Hz, H-4), |
| | 5.29 (1 H, s, H-2), |
| | 3.785 (3 H, s, $OCH_3$), |
| | 7.35 - 7.75 (4 H, m, aromat. Protonen). |

Analog zu den in Beispielen Nr. 4 a, b und 5 verwendeten Synthese-Methoden werden durch Kondensation von Benzaldehyd bzw. substituierten Benzaldehyden mit den in Beispielen Nr. 1 bis 3 beschriebenen 3-Alkoxy-2(E)-alkenoaten folgende 3-Alkoxy-5(subst.)phenyl-2(E),4(E)-pentadiensäuren hergestellt: siehe Tabelle 1.

Tabelle 1 : 3-Alkoxy-5-phenylpentadiensäuren

| Beispiel Nr. | Bezeichnung | Ausbeute [%] *) | Schmp.[°C] (umkrist.aus) |
|---|---|---|---|
| 6 | 5-(2-Chlorphenyl)-3-ethoxy-2(E), 4(E)-pentadiensäure | 41 | 179-181 (Ethanol) |
| 7 | 5-(2-Chlorphenyl)-3-methoxy-4-methyl-2(E),4(E)-pentadiensäure | 24 | 126-128 (Methanol) |
| 8 | 5-(4-Chlorphenyl)-3-methoxy-2(E),4(E)-pentadiensäure | 36 | 200 (Ethanol) |
| 9 | 5-(2-Bromphenyl)-3-methoxy-2(E),4(E)-pentadiensäure | 71 | 206-207 (Ethanol) |
| 10 | 5-(3-Bromphenyl)-3-methoxy-2(E),4(E)-pentadiensäure | 14 | 161-163 (Ethanol) |
| 11 | 5-(2-Fluorphenyl)-3-methoxy 2(E),4(E)-pentadiensäure | 66 | 188-190 (Ethanol) |
| 12 | 5-(2-Trifluormethylphenyl)-3-methoxy-2(E),4(E)-pentadiensäure | 15 | 173-174 (Ether) |
| 13 | 5-(3-Trifluormethylphenyl)-3-methoxy-2(E),4(E)-pentadiensäure | 41 | 152-153 (Ethanol) |
| 14 | 5-(4-Trifluormethylphenyl)-3-methoxy-2(E),4(E)-pentadiensäure | 32 | 181-183 (Methanol) |
| 15 | 3-Methoxy-5-(2-nitrophenyl)-2(E),4(E)-pentadiensäure | | 209-211 (Ethanol) |
| 16 | 3-Methoxy-5-(3-nitrophenyl)-2(E),4(E)-pentadiensäure | 9 | 198-199 |
| 17 | 3-Methoxy-5(2,5-dimethylphenyl)-2(E),4(E)-pentadiensäure | 49 | 161-162 (Ether) |
| 18 | 5-(2-Chlor-5-methylphenyl)-3-methoxy-2(E),4(E)-pentadiensäure | 48 | 179-181 (Methanol) |
| 19 | 5-(2,3-Dichlorphenyl)-3-methoxy-2(E),4(E)-pentadiensäure | 10 | 215-216 (Ethanol) |
| 20 | 5-(2,4-Dichlorphenyl)-3-methoxy-2(E),4(E)-pentadiensäure | 73 | 196-197 (Ethanol) |

Fortsetzung Tabelle 1

| Beispiel Nr | Bezeichnung | Ausbeute [%] *) | Schmp.[°C] (umkrist. aus) |
|---|---|---|---|
| 21 | 5-(2,4-Dichlorphenyl)-3-ethoxy-2(E),4(E)-pentadiensäure | 50 | 198-200 (Ethanol) |
| 22 | 5-(2,5-Dichlorphenyl)-3-methoxy-2(E),4(E)-pentadiensäure | 55 | 198-199 (Ethanol) |
| 23 | 5-(2,5-Dichlorphenyl)-3-ethoxy-2(E),4(E)-pentadiensäure | 49 | 213-215 (Ethanol) |
| 24 | 5-(3,4-Dichlorphenyl)-3-methoxy-2(E),4(E)-pentadiensäure | 46 | 188-190 (Ethanol) |
| 25 | 5-(3,5-Dichlorphenyl)-3-methoxy-2(E),4(E)-pentadiensäure | 38 | 211-213 (2-Propanol) |
| 26 | 5-[3,5-Bis-(trifluormethyl)-phenyl]-3-methoxy-2(E),4(E)-pentadiensäure | 6 | 217-219 (Chloroform) |
| 27 | 5-(2-Chlor-5-trifluormethylphenyl)-3-methoxy-2(E),4(E)-pentadiensäure | 40 | 188-192 (Ethanol) |
| 28 | 5-(4-Chlor-2-trifluormethylphenyl)-3-methoxy-2(E),4(E)-pentadiensäure | 22 | 190-193 (Ethanol) |
| 29 | 5-(4-Brom-2-chlorphenyl)-3-methoxy-2(E),4(E)-pentadiensäure | 66 | 192-194 (Aceton) |
| 30 | 3-Methoxy-5-(2,6-dimethylphenyl)-2(E),4(E)-pentadiensäure | 20 | 168-170 (Ethanol/ Wasser) |
| 31 | 3-Methoxy-5-(2-methylphenyl)-2(E),4(E)-pentadiensäure | 70 | 174-176 (Ethanol) |
| 32 | 5-(2,4-Difluorphenyl)-3-methoxy-2(E),4(E)-pentadiensäure | 27 | 180-182 (Ethanol/ Wasser) |

*) Bei Ausbeuten unter 50 % sind Reaktions-, Aufarbeitungs- und Umkristallisationsbedingungen noch nicht optimiert.

Beispiel Nr. 33

Benzyl-5-(2-chlorphenyl)-3-methoxy-2(E),4(E)-pentadienoat :

Zu der Mischung aus 48 g (0.2 Mol) 5-(2-Chlorphenyl)-3-methoxy-2(E),4(E)-pentadiensäure, 600 ml Aceton und 60 g (0.43 Mol) Kaliumcarbonat tropft man unter Rühren 34.2 g (0.2 Mol) Benzylbromid und erhitzt 16 Stdn. zum Rückfluß. Nach Abfiltrieren anorganischer Rückstände, Evaporieren des Filtrats und Umkristallisation aus tert. Butylmethylether erhält man 56.1 g (0.17 Mol) reinen Benzylester mit Schmp. 83-86°C. Ausbeute : 85 %

| | |
|---|---|
| Analyse : | $C_{19}H_{17}ClO_3$ (328.80) |
| Ber. : | C (69.41), H (5.21), Cl (10.78) |
| Gef. : | C (69.42), H (5.08), Cl (10.09). |

Beispiel Nr. 34

Ethyl-5-(2-chlorphenyl)-3-methoxy-2(E),4(E)-pentadienoat :

a) Die Mischung aus 31 g (0.13 Mol) 5-(2-Chlorphenyl)-3-methoxy-2(E),4(E)-pentadiensäure, 400 ml Aceton, 36 g (0.26 Mol) Kaliumcarbonat und 20.8 g (0.13 Mol) Jodethan wird 18 Stdn. unter Rückfluß zum Sieden erhitzt. Nach dem Abkühlen, Filtrieren und Evaporieren des Filtrats nimmt man das ölige Rohprodukt in 100 ml n-Pentan auf, filtriert unlösliches Kaliumjodid ab, evaporiert das Filtrat und trocknet bei 45°C und 20 mbar nach. Man erhält 33.3 g (0.125 Mol) reinen Ethylester mit Schmp. 49 - 51°C. Ausbeute : 96 %.

| | |
|---|---|
| Analyse : | $C_{14}H_{15}ClO_3$ (266.73) |
| Ber. : | C (63.05), H (5.67), Cl (13.29) |
| Gef. : | C (63.37), H 5.69), Cl (13.3) |

b) Unter Rühren tropft man zu dem Gemisch aus 35 Litern Butanon, 2386 g (10 Mol) 5-(2-Chlorphenyl)-3-methoxy-2(E),4(E)-pentadiensäure, 2073 g (15 Mol) Kaliumcarbonat und 16.6 g (0.1 Mol) Kaliumjodid bei 56°C 1635 g (15 Mol) Bromethan und rührt 24 Stdn. bei 56°C nach. Nach dem Abkühlen und Abfiltrieren anorganischer Bestandteile wäscht man das Filtrat mit 2 mal 10 Litern Wasser und evaporiert die Butanonphase. Man erhält 2348 g (8.80 Mol) Ethylester, der beim Erkalten kristallisiert. Dünnschichtchromatographie und Infrarotspektrum zeigen die Identität mit dem zuvor beschriebenen Produkt (34 a) an. Schmp. 45°C. Ausbeute : 88 %.

Beispiel Nr. 35

Methyl-5-(2-chlorphenyl)-3-methoxy-2(E),4(E)-pentadienoat :

Das Gemisch aus 23.9 g (0.1 Mol) 5-(2-Chlorphenyl)-3-methoxy-2(E),4(E)-pentadiensäure, 500 ml Aceton und 30.4 g (0.22 Mol) Kaliumcarbonat wird unter Rühren mit 9.5 ml (0.1 Mol) Dimethylsulfat versetzt und 4 Stdn. lang zum Rückfluß erhitzt. Nach dem Abkühlen und Filtrieren evaporiert man das Filtrat, löst das Rohprodukt in 100 ml Chloroform, wäscht mit 2 mal 30 ml Wasser, evaporiert die Chloroformphase und kristallisiert aus Isopropanol / Wasser um. Nach Trocknen bei 50°C / 20 mbar erhält man 22.18 g (87.8 mmol) reinen Methylester mit Schmp. 50 - 52°C. Ausbeute : 87.8 %.

| | |
|---|---|
| Analyse : | $C_{13}H_{13}ClO_3$ (252.69) |
| Ber. : | C (61.79), H (5.18), Cl (14.03) |
| Gef.: | C (61.30), H (5.07), Cl (13.7) |

Analog zu den in Beispielen Nr. 33 bis Nr. 35 beschriebenen Methoden wurden die in Tabelle 2 aufgeführten 3-Alkoxy-5-phenyl-2(E),4(E)-pentadiensäureester hergestellt.

Tabelle 2 : 3-Alkoxy-5-phenylpentadienoate

| Beispiel Nr. | Bezeichnung | Ausbeute [%] | Schmp. [°C] (umkrist.aus) |
|---|---|---|---|
| 36 | Ethyl-3-methoxy-(2-methylphenyl)-2(E),4(E)-pentadienoat | 90 | Oel (evaporiert) |
| 37 | Isopropyl-5-(2-chlorphenyl)-3-methoxy-2(E),4(E)-pentadienoat | 95 | 55-58 (Pentan) |
| 38 | sec. Butyl-5-(2-chlorphenyl)-3-methoxy-2(E),4(E)-pentadienoat | 85 | 41-42 (Isopropanol/Wasser) |
| 39 | tert.Butyl-5-(2-chlorphenyl)-3-methoxy-2(E),4(E)-pentadienoat | 30 | Oel (evaporiert) |
| 40 | Ethyl-3-methoxy-5-phenyl-2(E),4(E)-pentadienoat | 96 | 34 (evaporiert) |
| 41 | Ethyl-5-(2-chlorphenyl)-3-ethoxy-2(E),4(E)-pentadienoat | 85 | 79-81 (Methanol) |
| 42 | Ethyl-5-(4-chlorphenyl)-3-methoxy-2(E),4(E)-pentadienoat | 94 | 52-53 (Isopropanol/Pentan) |
| 43 | Ethyl-5-(2-Bromphenyl)-3-methoxy-2(E),4(E)-pentadienoat | 98 | 45-46 (Pentan) |
| 44 | Ethyl-5-(2-fluorphenyl)-3-methoxy-2(E),4(E)-pentadienoat | 99 | 46-48 (Isopropanol) |
| 45 | Ethyl-5-(2-trifluormethylphenyl)-3-methoxy-2(E),4(E)-pentadienoat | 99 | 75 (Ethanol) |
| 46 | Ethyl-5-(3-trifluormethylphenyl)-3-methoxy-2(E),4(E)-pentadienoat | 92 | 46-47 (Ethanol) |
| 47 | Ethyl-5-(4-trifluormethylphenyl)-3-methoxy-2(E),4(E)-pentadienoat | 52 | 42-44 (Ethanol) |
| 48 | Ethyl-3-methoxy-5-(2.5-dimethylphenyl)-2(E),4(E)-pentadienoat | 94 | 53-54 (Ethanol) |
| 49 | Ethyl-5-(2-chlor-5-methylphenyl)-3-methoxy-2(E),4(E)-pentadienoat | 95 | 76-78 (Ethanol) |

Fortsetzung Tabelle 2

| Beispiel Nr. | Bezeichnung | Ausbeute [%] | Schmp.[°C] (umkrist. aus) |
|---|---|---|---|
| 50 | Ethyl-5-(2.3-dichlorphenyl)-3-methoxy-2(E),4(E)-pentadienoat | 78 | 110-111 (Ethanol) |
| 51 | Ethyl-5-(2.4-dichlorphenyl)-3-methoxy-2(E),4(E)-pentadienoat | 92 | 81-83 (Isopropanol) |
| 52 | Ethyl-5-(2.4-dichlorphenyl) -3-ethoxy-2(E),4(E)-pentadienoat | 92 | 64-67 (Isopropanol) |
| 53 | Ethyl-5-(2.5-dichlorphenyl)-3-methoxy-2(E),4(E)-pentadienoat | 92 | 97-98 (Ethanol) |
| 54 | Ethyl-5-(2.5-dichlorphenyl)-3-ethoxy-2(E),4(E)-pentadienoat | 64 | 87-90 (Isopropanol) |
| 55 | Ethyl-5-(3.4-dichlorphenyl)-3-methoxy-2(E),4(E)-pentadienoat | 89 | 76-78 (Ethanol) |
| 56 | Ethyl-5-(3.5-dichlorphenyl)-3-methoxy-2(E),4(E)-pentadienoat | 93 | 89-91 (Ethanol) |
| 57 | Ethyl-5-[3.5-bis-(trifluormethyl)-phenyl]-3-methoxy-2(E),4(E)-pentadienoat | 88 | 86-88 (evaporiert) |
| 58 | Ethyl-5-(2-chlor-5-trifluormethyl-phenyl)-3-methoxy-2(E),4(E)-pentadienoat | 76 | 47-50 (Ethanol |
| 59 | Ethyl-5-(4-chlor-2-trifluormethyl-phenyl)-3-methoxy-2(E),4(E)-pentadienoat | 92 | 79-80 (Ethanol / Wasser) |
| 60 | Ethyl-5-(4-brom-2-chlorphenyl)-3-methoxy-2(E),4(E)-pentadienoat | 91 | 91-93 (Ethanol) |
| 61 | Ethyl-5-(2-chlorphenyl)-3-methoxy-4-methyl-2(E),4(E)-pentadienoat | 98 | Oel (evaporiert) |
| 62 | Ethyl-5-(2,6-dimethylphenyl)-3-methoxy-2(E),4(E)-pentadienoat | 91 | 101-103 (Ethanol) |
| 63 | Ethyl-5-(2,4-difluorphenyl)-3-methoxy-2(E),4(E)-pentadienoat | 98 | 67-68 |

Beispiel Nr. 64

threo-5-(2-Chlorphenylhydroxymethyl)-4-methoxy-2(5H)-furanon :

Anhand der Synthese dieser Ausgangsverbindung werden die verschiedenen Ausführungsformen der durch Osmiumtetroxid katalysierten Oxidation der 3-Alkoxy-5-phenyl-2(E),4(E)-pentadiensäure-Derivate zu den threo-4-Alkoxy-5-phenylhydroxymethyl-2(5H)-furanonen dargestellt.

Verfahrensvariante a :

Zu der Mischung aus 48.0 g (201 mmol) 5-(2-Chlorphenyl)-3-methoxy-2(E),4(E)-pentadiensäure, 400 ml Wasser und 400 ml (566 mmol) 20 %igem wäßrigen Tetraethylammoniumhydroxid gibt man unter Rühren und Kühlen auf 0°C 50 ml einer 0.02M-Lösung von Osmiumtetroxid in Aceton oder tert. Butanol und 56 ml (403 mmol) 70 %iges wäßriges tert. Butylhydroperoxid. Nach 6 Stdn. Rühren bei 0°C läßt man 8 Tage bei 0-5°C stehen u. reduziert danach überschüssiges Hydroperoxid durch Verrühren mit 10 %iger wäßriger Natriumsulfit-Lösung. Durch Zugabe von 1M-Schwefelsäure stellt man auf pH=2, wobei nicht umgesetztes Ausgangsprodukt auskristallisiert. Dieses wird abgesaugt (10.1 g ≙ 42.3 mmol). Das Filtrat wird mit 3 mal 100 ml Chloroform extrahiert und der Extrakt mit 50 ml 1M-Salzsäure frei von quartären Ammoniumsalzen gewaschen. Der Extrakt enthält ein Gemisch aus threo-5-(2-Chlorphenyl)-4,5-dihydroxy-3-methoxy-2(E)-pentensäure und threo-5-(2-Chlorphenylhydroxymethyl)-4-methoxy-2(5H)-furanon. Durch Evaporieren vervollständigt man den Ringschluß und erhält nach Umkristallisation aus Ethylacetat 18.12 g (71.2 mmol) reines threo - 5-(2-Chlorphenylhydroxymethyl)-4-methoxy-2(5H)-furanon mit Schmp. 149-151°C. Ausbeute bezogen auf umgesetztes Ausgangsprodukt : 45.1 %.

Analyse : $C_{12}H_{11}ClO_4$ (254.67)
Ber.: C (56.59), H (4.35), Cl (13.92)
Gef.: C (56.27), H (4.35), Cl (14.0)

300 MHz-$^1$NMR : 7.3-7.7 (4 H, m, aromatische Protonen)
5.96 (1H, d, $J_{OH/H\alpha}$ = 5.5 Hz, α-OH),
5.440 (1 H, d, $J_{H-3/H-5}$ = 1.3 Hz, H-3),
5.225 (1 H, dd, $J_{H\alpha/H-5}$ = 2 Hz, Hα),
5.008 (1 H, s, H-5)
3.923 (3 H, s, $OCH_3$)

75.46 MHz-$^{13}$C-NMR (breitbandentkoppelt) :
C-2 (172.530), C-3 (90.268), C-4 (180.229)
C-5 (79.872), C-α (66.721), $C_{Ar}$-1' (138.409),
$C_{Ar}$-2' bis 6' (130.650, 129.571, 129.421, 129.032 und 127.265), $OCH_3$ (60.071).

Die Zuordnung der $^{13}$C-Signale wurde durch Offresonanz- und Gated-Spektrum sowie durch SFORD-Experimente gesichert.

Verfahrensvariante b:

Zur Lösung von 26.7 g (100 mmol) Ethyl-5-(2-Chlorphenyl)-3-methoxy-2(E),4(E)-pentadienoat in 450 ml Aceton gibt man 6.5 g (25 mmol) Tetraethylammoniumacetat-tetrahydrat und tropft unter Rühren und Kühlen auf 0°C nacheinander 25 ml 0.02M-Osmiumtetroxid-Lösung in tert. Butanol und 23.6 ml (170 mmol) 70 %iges wäßriges tert. Butylhydroperoxid zu. Man läßt 12 Tage bei 4°C stehen, gibt 200 ml Dichlormethan und 140 ml 10 %ige wäßrige Natriumsulfitlösung zu, rührt, bis kein Hydroperoxid mehr nachweisbar ist, trennt die organische Phase ab, extrahiert die wäßrige Phase mit 2 x 100 ml Dichlormethan und wäscht die vereinigten organ. Phasen mit wäßriger Kochsalzlösung. Die organische Phase enthält ein Gemisch von threo-Ethyl-5-(2-chlorphenyl)-4,5-dihydroxy-3-methoxy-2(E)-pentenoat und threo-5-(2-Chlorphenylhydroxymethyl)-4-methoxy-2(5H)-furanon. Durch Evaporieren vervollständigt man den Ringschluß und erhält nach Umkristallisieren aus tert.

Butylmethyläther 19.4 g (76.2 mmol) Reinprodukt mit Schmp. 149-151°C, das mit dem nach Beispiel Nr.64 a gewonnenen Produkt dünnschichtchromatographisch und IR-spektroskopisch identisch ist. Ausbeute : 72.6 %.

Verfahrensvariante c :

Wie bei Variante b, jedoch wird statt des Ethylesters das Benzyl-5-(2-chlorphenyl)-3-methoxy-2(E),4(E)-pentadienoat eingesetzt. Ausbeute an threo-5-(2-Chlorphenylhydroxymethyl)-4-methoxy-2(5H)-furanon nach Umkristallisation aus Ethanol : 67.5 %. Schmp. 149 - 151°C.

Verfahrensvariante d :

Zur Lösung von 26.7 g (100 mmol) Ethyl-5-(2-Chlorphenyl)-3-methoxy-2(E),4(E)-pentadienoat in 360 ml Aceton gibt man unter Rühren 10 ml 0.02molare Osmiumtetroxid-Lösung in tert. Butanol und die Lösung von 14.9 g (110 mmol) N-Methylmorpholin-N-oxid-Hydrat in 30 ml Wasser und rührt 4 Tage bei Raumtemperatur. Durch Verrühren mit 5.2 g (50 mmol) Natriumbisulfit, gelöst in 50 ml Wasser, reduziert man überschüssiges N-Oxid, stellt mit 1M-Schwefelsäure auf pH = 4 ein, zieht im Vakuum das Aceton ab und extrahiert die verbleibende wäßrige Mischung mit 2 x 200 ml Dichlormethan. Der Extrakt wird mit verdünnter Schwefelsäure und Wasser frei von N-Methylmorpholin gewaschen und nach Trocknen über wasserfreiem Natriumsulfat im Vakuum konzentriert. Es kristallisieren 18.13 g threo-5-(2-Chlorphenylhydroxymethyl)-4-methoxy-2(5H)-furanon aus. Evaporieren des Filtrats und Umkristallisation aus Ethylacetat ergeben weitere 1.20 g Reinprodukt. Ausbeute : 19.33 g (75.9 mmol) ≙ 75.9 %.

Verfahrensvariante e :

Wie bei Variante d, jedoch wird die Reaktion statt in Aceton im 2-Phasensystem Butanon / Wasser durchgeführt/und statt 110 mmol 160 mmol N-Methylmorpholin-N-oxid eingesetzt. Ausbeute : 76.2 %.

Verfahrensvariante f :

Wie bei Variante d, jedoch im 2-Phasensystem Dichlormethan / Wasser. Ausbeute : 66.7 %.

Verfahrensvariante g :

Das Gemisch aus 23.6 g (80 mmol) sec. Butyl-5-(2-chlorphenyl)-3-methoxy-2(E),4(E)-pentadienoat, 200 ml Butanon, 15 ml 0.02M-Osmiumtetroxid-Lösung in tert. Butanol, 17.6 g (160 mmol) N-Methylmorpholin-N-oxid-Hydrat und 50 ml Wasser wird 7 Tage bei Raumtemperatur gerührt. Nach Zugabe von 100 ml Dichlormethan, verrührt man mit 100 ml 2 %iger wäßriger Natriumbi sulfit-Lösung, trennt die organische Phase ab und wäscht sie mit 2 mal 100 ml 0.5M-Salzsäure und 5mal mit 100 ml Wasser. Die organische Phase enthält ein Gemisch von threo-sec. Butyl-5-(2-chlorphenyl)-4.5-dihydroxy-3-methoxy-2(E)-pentenoat und threo-5-(2-Chlorphenylhydroxymethyl)-4-methoxy-2(5H)-furanon. Durch Zugabe von 0.1 ml 32 % Salzsäure und Evaporieren der organischen Phase vervollständigt man den Ringschluß und erhält nach Umkristallisation aus Ether 10.66 g (41.9 mmol) threo-5-(2-Chlorphenylhydroxymethyl)-4-methoxy-2(5H)-furanon. Ausbeute : 52.3 %.

In gleicher Weise kann man statt des sec. Butylesters die entsprechenden Methyl-, Isopropyl- oder tert. Butylester einsetzen.

Analog zu den in Beispiel Nr. 64 beschriebenen Verfahrensvarianten a bis g werden durch Oxidation der in den Beispielen Nr. 4 bis Nr. 63 beschriebenen 3-Alkoxy-5-phenyl-2(E),4(E)-pentadiensäure-Derivate folgende threo-4-Alkoxy-5-phenylhydroxymethyl-2(5H)-furanon-Derivate hergestellt : siehe Tabelle 3.

Tabelle 3 : threo-4-Alkoxy-5-phenylhydroxymethyl-2(5H)-furanone

| Beispiel Nr. | Bezeichnung | Variante | Ausb. [%] | Schmp.[°C] (umkrist aus) |
|---|---|---|---|---|
| 65 | threo-4-Methoxy-5-phenylhydroxy-methyl-2(5H)-furanon | a<br>b<br>d | 46<br>49<br>59 | 157 (Dichlormethan) |
| 66 | threo-5-(2-Chlorphenylhydroxymethyl)-4-ethoxy-2(5H)-furanon | a | 24 | 114-116 (Isopropanol) |
| 67 | threo-5-(4-Chlorphenylhydroxy-methyl)-4-methoxy-2(5H)furanon | b | 47 | 148-150 (Dichlormethan) |
| 68 | threo-5-(2-Bromphenylhydroxy-methyl)-4-methoxy-2(5H)-furanon | d | 85 | 162-164 (Dichlormethan) |
| 69 | threo-5-(3-Bromphenylhydroxy-methyl)-4-methoxy-2(5H)-furanon | | 50 | 167-168 (Aceton / Ethanol) |
| 70 | threo-5-(2-Fluorphenylhydroxy-methyl)-4-methoxy-2(5H)-furanon | d | 87 | 130-133 (Dichlormethan) |
| 71 | threo-4-Methoxy-5-(2-trifluormethyl-phenylhydroxymethyl)-2(5H)furanon | b | 62 | 166-167 (Ethanol / Wasser) |
| 72 | threo-4-Methoxy-5-(3-trifluormethyl-phenylhydroxymethyl)-2(5H)-furanon | b | 27 | 128-129 (Ethanol / Wasser) |
| 73 | threo-4-Methoxy-5-(4-trifluormethyl-phenylhydroxymethyl)-2(5H)-furanon | b | 28 | 171 (Methanol) |
| 74 | threo-4-Methoxy-5-(2-nitrophenylhy-droxymethyl)-2(5H)-furanon | a | 10 | 170-172 (Ethylacetat) |
| 75 | threo-4-Methoxy-5-(3-nitrophenyl-hydroxymethyl)-2(5H)-furanon | a | 21 | 149-151 (Ethylacetat) |
| 76 | threo-5-(2.5-Dimethylphenylhydroxy-methyl)-4-methoxy-2(5H)-furanon | b | 47 | 171-173 (Ethanol) |
| 77 | threo-5-[(2-Chlor-5-methylphenyl)-hydroxymethyl]-4-methoxy-2(5H)-furanon | b | 71 | 174-175 (Methanol) |

1. Fortsetzung Tabelle 3

| Beispiel Nr. | Bezeichnung | Variante | Ausb. [%] | Schmp.[°C] (umkrist. aus) |
|---|---|---|---|---|
| 78 | threo-5-(2.3-Dichlorphenylhydroxymethyl)-4-methoxy-2(5H)-furanon | e | 75 | 161-162 (Methanol) |
| 79 | threo-5-(2.4-Dichlorphenylhydroxymethyl)-4-methoxy-2(5H)furanon | a<br>b<br>e | 38<br>87<br>55 | 174 (Dichlormethan) |
| 80 | threo-5-(2.4-Dichlorphenylhydroxymethyl)-4-ethoxy-2(5H)-furanon | b | 72 | 133 (Isopropanol) |
| 81 | threo-5-(2.5-Dichlorphenylhydroxymethyl)-4-methoxy-2(5H)-furanon | a<br>b | 15<br>70 | 187 (Dichlormethan) |
| 82 | threo-5-(2.5-Dichlorphenylhydroxymethyl)-4-ethoxy-2(5H)-furanon | b | 70 | 175-178 (Isopropanol) |
| 83 | threo-5-(3.4-Dichlorphenylhydroxymethyl)-4-methoxy-2(5H)-furanon | b | 53 | 158-159 (Ethylacetat) |
| 84 | threo-5-(3.5-Dichlorphenylhydroxymethyl)-4-methoxy-2(5H)-furanon | b | 62 | 177-179 (Ethanol) |
| 85 | threo-5-[3.5-Bis-(trifluormethyl)-phenylhydroxymethyl]-4-methoxy--2(5H)-furanon | e | 52 | 191-194 (Ethylacetat) |
| 86 | threo-5-[(2-Chlor-5-trifluormethyl-phenyl)-hydroxymethyl]-4-methoxy-2-(5H)-furanon | e | 69 | 196-199 (Ethylacetat) |
| 87 | threo-5-[(4-Chlor-2-trifluormethyl-phenyl)-hydroxymethyl]-4-methoxy-2(5H)-furanon | e | 76 | 166-172 (Ethylacetat) |
| 88 | threo-5-[(4-Brom-2-chlorphenyl)-hydroxymethyl]-4-methoxy-2(5H)-furanon | e | 84 | 173-176 (Methanol) |
| 89 | threo-5-(2-Chlorphenylhydroxymethyl)-4-methoxy-5-methyl-2(5H)-furanon | * | 10 | 181-184 (Ethylacetat) |

* Oxidation mit Natriumchlorat in tert. Butanol / Wasser unter Rückfluß

2. Fortsetzung Tabelle 3

| Beispiel Nr. | Bezeichnung | Variante | Ausb. [%] | Schmp.[°C] (umkrist. aus) |
|---|---|---|---|---|
| 90 | threo-5-(2,6-Dimethylphenylhydroxymethyl)-4-methoxy-2(5H)-furanon | d | 36 | 152-153 (Dichlormethan/ Pentan) |
| 91 | threo-5-(2,4-Difluorphenylhydroxymethyl)-4-methoxy-2(5H)-furanon | d | 26 | 164 (Dichlormethan) |
| 92 | threo-5-(4-Bromphenylhydroxymethyl)-4-methoxy-2-(5H)-furanon | d | 82 | 157-158 ($CCl_4$ / Ethylacetat) |
| 93 | threo-4-Methoxy-5-(2-methylphenylhydroxymethyl)-2(5H)-furanon | d | 61 | 174-176 (Dichlormethan) |
| 94 | threo-4-Methoxy-5-(3-methylphenylhydroxymethyl)-2(5H)-furanon | d | 65 | 127-130 (Dichlormethan/ Pentan) |
| 95 | threo-4-Methoxy-5-(4-methylphenylhydroxymethyl)-2(5H)-furanon | e | 71 | 148-151.5 (Isopropanol/ Pentan) |
| 96 | threo-5-(3-Fluorphenylhydroxymethyl)-4-methoxy-2(5H)-furanon | d | 82 | 123-126 (Dichlormethan/ Pentan) |
| 97 | threo-5-(4-Fluorphenylhydroxymethyl)-4-methoxy-2(5H)-furanon | d | 70 | 141 (Toluol/tert. Butylmethylether) |
| 98 | threo-5-(3-Chlorphenylhydroxymethyl)-4-methoxy-2(5H)-furanon | d | 62 | 150-152 (Chloroform / Pentan) |
| 99 | threo-4-Methoxy-5-(4-nitrophenylhydroxymethyl)-2(5H)-furanon | d | 47 | 226-228 (Isopropanol) |

3. Fortsetzung Tabelle 3

| Beispiel Nr. | Bezeichnung | Variante | Ausb. [%] | Schmp.[°C] (umkrist. aus) |
|---|---|---|---|---|
| 100 | threo-5-(2-Chlor-6-fluorphenyl-hydroxymethyl)-4-methoxy-2(5H)-furanon | d | 60 | 129-133 ($CCl_4$ / Pentan) |
| 101 | threo-5-(2.6-Dichlorphenylhydroxy-methyl)-4-methoxy-2(5H)-furanon | d | 72 | 176-180 (Toluol / Aceton) |

Beispiel Nr. 102

threo-5-[N-benzyloxycarbonyl-L-prolyloxy-(2-chlorphenyl)-methyl]-4-methoxy-2(5H)-furanon ; Diastereomere A und B :

Unter Rühren und Kühlen auf ca. -5°C gibt man zu der Mischung aus 102 g (0.4 Mol) threo-5-(2-Chlorphenylhydroxymethyl)-4-methoxy-2(5H)-furanon, 100 g (0.4 Mol)N-Benzyloxycarbonyl-L-prolin und 1200 ml wasserfreiem Dichlormethan nacheinander 6 g (0.04 Mol) 4-Pyrrolidinopyridin und portionenweise insgesamt 91 g (0.44 Mol) N,N′-Dicyclohexylcarbodiimid zu. Man rührt 4 Stdn. bei 0°C nach, filtriert ausgefallenen N,N′-Dicyclohexylharnstoff ab, wäscht das Filtrat 2 mal mit je 100 ml 5 %iger Essigsäure und 2 mal mit je 100 ml Wasser, trocknet über wasserfreiem Natriumsulfat und evaporiert. Das ölige Rohprodukt wird in 1500 ml Aceton aufgenommen, ca. 15 Stdn. bei -8°C stehen gelassen und restlicher N,N′-Dicyclohexylharnstoff abfiltriert. Das Filtrat wird evaporiert, in 250 ml Ethanol aufgenommen und 18 Stdn. bei 5°C stehen gelassen. Das ausfallende Kristallisat wird abgesauft und 2 mal aus Ethanol umkristallisiert. Man erhält 84.2 g (173 mmol) des Diastereomeren A der Titelverbindung mit Schmp. 164 - 167°C und dc-Rf = 0.53 (n-Butanol). Ausbeute : 43.3 %. $[\alpha]_D^{20}$ -16.6 (c = 1; Methanol).

| | |
|---|---|
| Analyse : | $C_{25}H_{24}ClNO_7$ (485.93) |
| Ber. : | C (61.79), H (4,98), N (2.88), Cl (7.30) |
| Gef. : | C (61.62), H (4.85), N (2.81), Cl (7.6 ) |

Zur Gewinnung des Diastereomeren B der Titelverbindung werden die vereinigten ethanolischen Mutterlaugen evaporiert, aus Isopropanol oder n-Butanol kristallisiert und 2 mal aus Isopropanol umkristallisiert. Man erhält 72.0 g (148.2 mmol) des Diastereomeren B mit Schmp. 112 - 115°C und dc-Rf = 0.6 (n-Butanol). Ausbeute : 37 %. $[\alpha]_D^{20}$ -61.3 (c = 1; Methanol)

| | |
|---|---|
| Analyse : | $C_{25}H_{24}ClNO_7$ (485.93) |
| Ber. : | C (61.79), H (4.98), N (2.88), Cl (7.30) |
| Gef. : | C (61.78), H (4.93), N (2.85), Cl (7.6 ) |

Beispiel Nr. 103

threo-5-[2-Chlorphenyl -(L-prolyloxy)methyl]-4-methoxy-2(5H)-furanon Hydrobromid ; Diastereomere A und B :

A :

48.6 g (100 mmol) des Diastereomen A aus Beispiel Nr. 102 werden in 800 ml Ethylacetat suspendiert. Unter Rühren und Eisbad-Kühlung tropft man 50 ml 33 %ige Bromwasserstofflösung in Eisessig zu und läßt 3 Tage bei ca. 20°C stehen. Das Kristallisat wird abgesaugt, mit Ethylacetat säurefrei gewaschen und im Vakuum getrocknet. Man erhält 36.8 g (85 mmol) des Diastereomeren A der Titelverbindung in Form des Hydrobromids mit Schmp. 223 - 225°C (Zersetz.) und dc-Rf = 0.63 (Chloroform / Methanol 6 / 4). Ausbeute : 85 % . $[\alpha]_D^{20}$ -3.0 (c = 1 ; Methanol).

| | |
|---|---|
| Analyse : | $C_{17}H_{18}ClNO_5$ x HBr (432.71) |
| Ber. : | C (47.19), H (4.43), N 3.24), Cl (8.19) Br. (18.47) |
| Gef. : | C (47.43), H (4.38), N (3.10), Cl (8.1) Br. (18.7 ) |

300 MHz - $^1$H-NMR (DMSO-$d_6$) :
9.24 (sehr breit, NH-pro), 7.43-7.65 (4 H, m, arom. Protonen),
6.40 (1 H, d, $J_{\alpha/5}$ = 2.65 Hz, H-α), 5.58 (1 H, d, $J_{3/5}$ = 0.88 Hz, H-3),
5.42 (1 H, dd, H-5), 461 (1 H, dd, $J_1$ = 7.08 Hz, $J_2$ = 6.63 Hz, H-2-pro),
3.91 (3 H, s, 4-$OCH_3$), 3.1-3.5 (2 H, m, 2 H-5-pro), 2.25-2.35 (1 H, m, H-3a-pro), 1.85-2.03 (3 H, m, H-3b-pro und 2 H-4-pro).
$^1$H-NMR-spektroskopisch sind keine Signale des Diastereomeren B nachzuweisen. Die absolute Konfiguration wird durch Röntgenbeugung am Einkristall ermittelt und ist R an C-4 und S an C-α.

B :

47.9 (98.6 mmol) des Diastereomeren B aus Beispiel Nr. 102 werden wie unter Nr. 103 A beschrieben in 450 ml Ethylacetat mit 48 ml 33 %iger Bromwasserstoffsäure in Eisessig versetzt und nach Stehenlassen über Nacht aufgearbeitet. Man erhält 40.6 (93.8 mmol) des Diastereomeren B der Titelverbindung in Form des Hydrobromids mit Schmp. 190 - 193°C (Zersetz.) und dc-Rf = 0.54 (Chloroform / Methanol 6 / 4). Ausbeute : 95.2 %. $[\alpha]_D^{20}$-33.5 (c = 1; Methanol)

Analyse : $C_{17}H_{18}ClNO_5$ x HBr (432.71)
Ber. : C (47.19), H (4.43), N (3.24), Cl (8.19), Br (18.47)
Gef. : C (47.38), H (4.36), N (3.27), Cl (7.8 ), Br. (18.1 ) 300 MHz - NMR (DMSO-$d_6$) :
9.32 (sehr breit, NH-pro), 7.4-7.56 (4 H, m, arom. Protonen),
6.36 (1 H, d, J $_{\alpha/5}$ = 3.54 Hz, H-α), 5.52 (1 H, s, H-3),
5.38 ( 1 H, d, H-5), 4.53 (1 H, t, $J_1$ = 7.96 Hz, $J_2$ = 6.52 Hz, H-2-pro),
3.89 (3 H, s, 4-$OCH_3$), 3.22 (2 H, t, $J_{5/4}$ = 7.08 Hz, 2 H-5-pro),
2.27-2.40 (1 H, m, H-3a-pro), 1.75-2.05 (3 H, m, H-3b-pro und 2 H-4-pro).
Das $^1$H-NMR-Spektrum zeigt außerdem die Anwesenheit von ca. 5 % des Diastereomeren 103 A an (Verhältnis der Integrale der jeweiligen 4-$OCH_3$-Protonen).

Beispiel Nr. 104

threo-5-[2-Chlorphenyl-(L-prolyloxy)-methyl]-4-methoxy-2(5H)-furanon Hydrochlorid ; Diastereomere A und B :

Analog Beispiel Nr. 102 werden 28 g (110 mmol) threo-5-(2-Chlorphenylhydroxymethyl)-4-methoxy-2(5H)-furanon bei -5°C in 500 ml Dichlormethan mit 23.7 g (110 mmol) N-tert. Butoxycarbonyl-L-prolin unter Zugabe von 1.63 g(11 mmol) 4-Pyrrolidinopyridin und 25.2 g (122 mmol) N,N'-Dicyclohexylcarbodiimid verestert und aufgearbeitet. Man erhält 50.1 g eines Gemisches der beiden diastereomeren N-Benzyloxycarbonyl-L-prolylester, die in 300 ml Ethylacetat gelöst und unter Eisbadkühlung und Rühren mit 18 ml 36 %iger Chlorwasserstoffsäure versetzt werden. Man läßt 18 Stdn. bei ca. 20°C stehen. Das auskristallisierte Produkt wird abgesaugt und ergibt nach Umkristallisation aus Methanol und Trocknen im Vakuum 11.57 g (29.8 mmol) des Diastereomeren A der Titelverbindung in Form des Hydrochlorids mit Schmp. 213 -214°C. Ausbeute : 27.1 % . $[\alpha]_D^{20}$ -4.2 (c = 1; Methanol).

Analyse : $C_{17}H_{18}ClNO_5$ x HCl (388.26)
Ber. : C (52.59), H (4.93), N (3.61), Cl (18.26)
Gef. : C (52.44), H (4.94), N (3.61), Cl (18.3 )

Das Filtrat der Hydrolysemischung wird evaporiert und ergibt nach Umkristallisation aus Isopropanol und Trocknen im Vakuum 11.7 g (30.1 mmol) des Diastereomeren B der Titelverbindung in Form des Hydrochlorids mit Schmp. 190 - 191°C. Ausbeute : 27.4 %. $[\alpha]_D^{20}$ -39.4 (c = 1; Methanol).

Analyse : $C_{17}H_{18}ClNO_5$ x HCl (388.26)
Ber. : C (52.59), H (4.93), N (3.61), Cl (18.26)
Gef. : C (52.34), H (4.94), N (3.52), Cl (18.1)

Beispiel Nr. 105

threo-5-[Glycyloxy-(phenyl)-methyl]-4-methoxy-2(5H)-furanon Hydrobromid :

Unter Rühren und Kühlen auf -5°C gibt man zu der Mischung aus 44.0 g (200 mmol) threo-4-Methoxy-5-phenylhydroxymethyl-2(5H)-furanon, 42.0 g (210 mmol) N-Benzyloxycarbonylglycin, 1 Liter wasserfreiem Dichlormethan und 3.0 g (20 mmol) 4-Pyrrolidinopyridin portionenweise insgesamt 45.8 g (222 mmol) N,N'-Dicyclohexylcarbodiimid und rührt bis zur völligen Veresterung weiter. Nach der Aufarbeitung analog Beispiel Nr. 102 erhält man in quantitativer Ausbeute threo-5-[N-Benzyloxycarbonylglycyloxy-(phenyl)-methyl]-4-methoxy-2(5H)-furanon als nicht kristallisierbares Öl. Dieses wird in 800 ml Ethylacetat unter Eiskühlung mit 100 ml 33 %iger Bromwasserstoffsäure in Eisessig versetzt. Nach Stehenlassen über Nacht saugt man das Kristallisat ab, wäscht mit Ethylacetat säurefrei und trocknet im Vakuum. Man erhält 63.6 g (177.5 mmol) der Titelverbindung in Form des Hydrobromids mit Schmp. 204 - 205°C und dc-Rf (Chloroform / Methanol 6 / 4) = 0.48. Ausbeute : 88.9 %.

Analyse : $C_{14}H_{15}NO_5$ x HBr (358.21)
Ber. : C (46.94), H (4.50), N (3.91), Br (22.31)
Gef. : C (46.93), H (4.50), N (3.88), Br (21.0 ).

Beispiel Nr. 106

threo-5-[N-Acetylglycyloxy-(2-chlorphenyl)-methyl]-4-methoxy-2(5H)-furanon :

Analog Beispiel Nr. 102 tropft man zu der Mischung aus 25.5 g (100 mmol) threo-5-(2-Chlorphenylhydroxymethyl)-4-methoxy-2(5H)-furanon, 11,8 g (100 mmol) N-Acetylglycin, 400 ml wasserfreiem Dichlormethan und 1.5 g (10 mmol) 4-Pyrrolidinopyridin bei -5°C die Lösung von 21.7 g (105 mmol) N,N'-Dicyclohexylcarbodiimid in 100 ml Dichlormethan und rührt 4 Stdn. bei -5°C nach. Das Rohprodukt ergibt nach Umkristallisation aus Ethanol und anschließend aus Isopropanol 19.5 g (55.1 mmol) der Titelverbindung mit Schmp. 160 - 162°C und dc-Rf (Chloroform / Methanol 95 / 5) = 0.45. Ausbeute : 55.1 %.

| | |
|---|---|
| Analyse : | $C_{16}H_{16}ClNO_6$ (353.77) |
| Ber. : | C (54.32), H (4.56), N (3.96), Cl (10.02) |
| Gef. : | C (53.46), H (4.39), N (3.80), Cl (10.0) |

Beispiel Nr. 107

threo-5-[N-Acetyl-L-prolyloxy-(2-chlorphenyl)-methyl]-4-methoxy-2(5H)-furanon ; Diastereomer A :

Zu der Mischung aus 100 ml wasserfreiem Dichlormethan, 4.0 ml (50 mmol) Pyridin und 0.3 g (2 mmol) 4-Pyrrolidinopyridin gibt man unter Rühren und Eisbadkühlung nacheinander 8.65 g (20 mmol) threo-5-[2-Chlorphenyl-(L-prolyloxy)-methyl]-4-methoxy-2(5H)-furanon Hydrobromid (Diastereomer A aus Beispiel Nr. 103) und 2.8 ml (30 mmol) Acetanhydrid zu und rührt 2,5 Stdn. bei 0°C nach. Man wäscht mit Eiswasser frei von Pyridin, trocknet die Dichlormethanphase über wasserfreiem Natriumsulfat, evaporiert und kristallisiert das Produkt durch Behandeln mit wasserfreiem Ethanol. Umkristallisation aus Ethanol ergibt 6.79 g (17.2 mmol) der Titelverbindung mit Schmp. 168 - 170°C und dc-Rf (Chloroform / Methanol 6 / 4) = 0.77. Ausbeute : 86.2 %. $[\alpha]_D^{20}$ -33 (c = 1; Methanol).

| | |
|---|---|
| Analyse : | $C_{19}H_{20}ClNO_6$ (393.83) |
| Ber. : | C (57.95), H (5.12), N (3.56), Cl (9.00) |
| Gef. : | C (57.72), H (5.20), N (3.45), Cl (9.2) |

In analoger Weise, wie in den Beispielen Nr. 102 bis Nr. 107 beschrieben, werden die folgenden Aminosäureester-Derivate erhalten. Die jeweils angegebenen Summenformeln entsprechen den Ergebnissen der Elementaranalysen.

Beispiel Nr. 108

threo-4-Methoxy-5-[phenyl-(L-phenylglycyloxy)-methyl]-2(5H)-furanon Hydrobromid ; Diastereomere A und B :

Herstellung durch Veresterung von threo-4-Methoxy-5-phenylhydroxymethyl-2(5H)-furanon mit N-Benzyloxycarbonyl-L-phenylglycin und anschließende Abspaltung der Schutzgruppe mit HBr / Eisessig.

Diastereomer A :

$C_{20}H_{19}NO_5$ x HBr (434.30), Schmp. 208-210°C (Zersetz., aus Isopropanol / Pentan) ; dc-Rf (Chloroform / Methanol 6 / 4) = 0.78. $[\alpha]_D^{20}$ +56.4 (c = 1; Methanol).

Das Produkt enthält laut $^1$H-NMR-Spektrum ca. 27 % des Diastereomeren B. Ausbeute: 13.4 %

Diastereomer B :

$C_{20}H_{19}NO_5$ x HBr (434.30), Schmp. 218-219°C (Zersetz., aus Isopropanol / Ethanol 2 / 1). dc-Rf (Chloroform / Methanol 6 / 4) = 0.70. $[\alpha]_D^{20}$ +29.8 (c = 1; Methanol). Ausbeute : 31.3 %

$^1$H-NMR-spektrometrisch ist kein Diastereomer A nachzuweisen.

Beispiel Nr. 109

threo-5-[N-Benzyloxycarbonyl-L-prolyloxy-(phenyl)-methyl]-4-methoxy-2(5H)-furanon; Diastereomer A und B:

Herstellung durch Veresterung von threo-4-Methoxy-5-phenylhydroxymethyl-2(5H)-furanon mit N-Benzyloxycarbonyl-L-prolin.

Diastereomer A:

$C_{25}H_{25}NO_7$ (451.49), Schmp. 138,5 - 139,5°C (aus Ethanol); dc-Rf (Toluol / Aceton 8 / 2) = 0.31. $[\alpha]_D^{20}$ -3.3 (c = 1; Methanol). Ausbeute : 36.9 %

Diastereomer B:

$C_{25}H_{25}NO_7$ (451.49), Schmp. 88 - 92°C (2 x aus Isopropanol); dc-Rf (Toluol / Aceton 8 / 2) = 0.33. $[\alpha]_D^{20}$ -89.8 (c = 1; Methanol). Ausbeute : 20.9 %

Beispiel Nr. 110

threo-4-Methoxy-5-[phenyl-(L-prolyloxy)-methyl]-2(5H)-furanon Hydrobromid; Diastereomere A und B :

Herstellung durch Abspaltung der Schutzgruppe aus den Diastereomeren A und B des Beispiels Nr. 109 mittels HNr / Eisessig.

Diastereomer A :

$C_{17}H_{19}NO_5$ x HBr (398.27). Schmp. 185°C (aus Ethylacetat). dc-Rf (Chloroform / Methanol 6 / 4) = 0.58. $[\alpha]_D^{20}$ +18.2 (c = 1; Methanol). Ausbeute: 85.1 %.

Diastereomer B :

$C_{17}H_{19}NO_5$ x HBr (398.27). Schmp. 191 - 194°C (aus Ethanol). dc-Rf (Chloroform / Methanol 6 / 4) = 0.54. $[\alpha]_D^{20}$ -71.3 (c = 1; Methanol). Ausbeute: 85.4 %.

Beispiel Nr. 111

threo-5-[2-Chlorphenyl-(glycyloxy)-methyl]-4-methoxy-2(5H)-furanon Hydrobromid :

Herstellung durch Veresterung von threo-5-(2-Chlorphenylhydroxymethyl)-4-methoxy-2(5H)-furanon mit N-Benzyloxycarbonylglycin und anschließende Abspaltung der Schutzgruppe.
$C_{14}H_{14}ClNO_5$ x HBr (392.65). Schmp. 202 - 205°C (aus Isopropanol / Ethanol 2 / 1). dc-Rf (Chloroform / Methanol 6 / 4) = 0.58. Ausbeute : 62.6 %.

Beispiel Nr. 112

threo-5-[2-Chlorphenyl-(glycyloxy)-methyl]-4-methoxy-2(5H)-furanon Hydrochlorid :

Aus dem Hydrobromid des Beispiels Nr. 111 durch Überführung in das Hydrochlorid erhalten.
$C_{14}H_{14}ClNO_5$ x HCl (348.20). Schmp. 188 - 189°C (aus Isopropanol / Methanol). Ausbeute : 89.3 %

Beispiel Nr. 113

threo-5-[4-Aminobutanoyloxy-(2-chlorphenyl)-methyl]-4-methoxy-2(5H)-furanon Hydrobromid :

Herstellung durch Veresterung von threo-5-(2-Chlorphenylhydroxymethyl)-4-methoxy-2(5H)-furanon mit 4-(N-benzyloxycarbonylamino)-buttersäure mit nachfolgender Abspaltung der Schutzgruppe.
$C_{16}H_{18}ClNO_5$ x HBr (420.70). Schmp. 181 - 182°C (aus Ethanol). dc-Rf (Chloroform / Methanol 6 / 4) = 0.5.

Ausbeute : 78.5 %

Beispiel Nr. 114

threo-5-[4-Aminobutanoyloxy-(2-chlorphenyl)-methyl]-4-methoxy-2(5H)-furanon Hydrochlorid :

Hergestellt durch Überführung des Hydrobromids aus Beispiel Nr. 113 in das Hydrochlorid. $C_{16}H_{18}ClNO_5$ x HCl (376.25). Schmp. 165 - 169 °C (aus Isopropanol).

Beispiel Nr. 115

threo-5-[4-(N-Acetylamino)-butanoyloxy-(2-chlorphenyl)-methyl]-4-methoxy-2( 5H)-furanon :

Hergestellt durch Acetylierung der Substanz aus Beispiel Nr. 113. $C_{18}H_{20}ClNO_6$ (381.82). Glasiges Produkt (aus Isopropanol). dc-Rf (Chloroform / Methanol 95 / 5)=0.35. Ausbeute : 91 %.

Beispiel Nr. 116

threo-5-[N-Benzyloxycarbonyl-L-phenylglycyloxy-(2-chlorphenyl)-methyl]-4-methoxy-2(5H)-furanon :

Herstellung durch Veresterung von threo-5-(2-Chlorphenylhydroxymethyl)-4-methoxy-2(5H)-furanon mit N-Benzyloxycarbonyl-L-phenylglycin.
Man erhält nach Umkristallisation aus Ethanol ein Gemisch der beiden Diastereomeren A und B.
$C_{28}H_{24}ClNO_7$ (521.96). Schmp. 137 - 140°C. $[\alpha]_D^{20}$ +32.4 (c = 1; Methanol) Ausbeute : 74.6 %.

Beispiel Nr. 117

threo-5-[2-Chlorphenyl-(L-phenylglycyloxy)-methyl]-4-methoxy-2(5H)-furanon Hydrobromid ; Diastereomere A und B :

Herstellung durch Abspaltung der Schutzgruppe aus der Substanz des Beispiels Nr. 116 und nachfolgende fraktionierte Kristallisation.

Diastereomer A :

$C_{20}H_{18}ClNO_5$ x HBr (468.74). Schmp. 215°C (Zersetz.; aus Ethanol / Pentan), dc-Rf (Chloroform / Methanol 9 / 1) = 0.64 $[\alpha]_D^{20}$ +47.3 (c = 1; Methanol) . Ausbeute : 22.8 %

Diastereomer B :

$C_{20}H_{18}ClNO_5$ x HBr (468.74). Zersetzungsbereich 138 - 180°C (aus Isopropanol). dc-Rf (Chloroform / Methanol 9 / 1) = 0.54. $[\alpha]_D^{20}$ +0.9 (c = 1; Methanol). Ausbeute : 9.9 %.

Beispiel Nr. 118

threo-5-[N-Benzyloxycarbonyl-D-phenylglycyloxy-(2-chlorphenyl)-methyl]-4-methoxy-2(5H)-furanon:

Herstellung analog Beispiel Nr. 116 durch Veresterung mit N-Benzyloxycarbonyl-D-phenylglycin. Man erhält nach Umkristallisation aus Ethanol ein Gemisch der beiden Diastereomeren A und B.
$C_{28}H_{24}ClNO_7$ (521.96). Schmp. 137 - 140°C. $[\alpha]_D^{20}$ -32.0 (c = 1 ; Methanol). Ausbeute : 75.3 %.

Beispiel Nr. 119

threo-5-[2-Chlorphenyl-(D-phenylglycyloxy)-methyl]-4-methoxy-2(5H)-furanon Hydrobromid ; Diastereomere A und B :

Herstellung durch Abspaltung der Schutzgruppe aus der Substanz des Beispiels Nr. 118 und nachfolgende fraktionierte Kristallisation.

Diastereomer A :

$C_{20}H_{18}ClNO_5$ x HBr x $H_2O$ (486.76). Schmp. 215°C (Zersetz.; aus Ethanol / Pentan). dc-Rf (Chloroform / Methanol 9 / 1) = 0.64 ; $[\alpha]_D^{20}$ -48.3 (c = 1; Methanol. Ausbeute : 25.8 %.

Diastereomer B :

$C_{20}H_{18}ClNO_5$ x HBr (468.74). Zersetzungsbereich : 138 - 180°C (aus Isopropanol). dc-Rf (Chloroform / Methanol 9 / 1) = 0.54. $[\alpha]_D^{20}$ -0.9 (c = 1 ; Methanol). Ausbeute : 16.4 %.

Beispiel Nr. 120

threo-5-[2-Chlorphenyl-(L-phenylalanyloxy)-methyl]-4-methoxy-2(5H)-furanon Hydrobromid ; Diastereomer B :

Herstellung durch Veresterung von threo-5-(2-Chlorphenylhydroxymethyl)-4-methoxy-2(5H)-furanon mit N-Benzyloxycarbonyl-L-phenylanin und nachfolgende Abspaltung der Schutzgruppe mittels HBr / Eisessig in Ethylacetat. Als Erstkristallisat erhält man das Diastereomer B in Form des Hydrobromids.
$C_{21}H_{20}ClNO_5$ x HBr (482.77). Schmp. 139 - 143°C (3 mal aus Ethylacetat). dc-Rf (Chloroform / Methanol 95 / 5) = 0.30. $[\alpha]_D^{20}$ -3.2 (c = 1 ; Methanol). Ausbeute : 11.3 %. Diastereomer A : siehe folgendes Beispiel.

Beispiel Nr. 121

threo-5-[2-Chlorphenyl-(N-isopropyliden-L-phenylalanyloxy)-methyl]-4-methoxy-2(5H)-furanon Hydrobromid ; Diastereomere A und B :

Das Filtrat des Erstkristallisats aus dem Beispiel Nr. 120 wird evaporiert, in Aceton gelöst und 25 Stdn. bei -6°C stehen gelassen. Das Diastereomer A fällt in Form des N-Isopropyliden - Derivates als Hydrobromid kristallin aus.
$C_{24}H_{24}ClNO_5$ x HBr (522.84). Schmp. 192 - 193°C (Zersetz.; 2 mal aus Methanol). dc-Rf (Chloroform / Methanol 95 / 5) = 0.43. $[\alpha]_D^{20}$ -16 (c = 1; Methanol). Ausbeute : 28.4 %.

Erwärmt man das Diastereomer B aus Beispiel Nr. 120 in Aceton, erhält man ebenfalls das entsprechende N-Isopropyliden-Derivat B.
$C_{24}H_{24}ClNO_5$ x HBr (522.84). Schmp. 180 - 183°C (Zersetz.;aus Aceton). dc-Rf (Chloroform / Methanol 95 / 5) = 0.32. $[\alpha]_D^{20}$ -31.9 (c = 1 ; Methanol).

Beispiel Nr. 122

threo-5-[2-Chlorphenyl-(L-glutaminyloxy)-methyl]-4-methoxy-2(5H)-furanon Hydrobromid ; Diastereomer A :

Herstellung durch Veresterung von threo-5-(2-Chlorphenylhydroxymethyl)-4-methoxy-2(5H)-furanon mit N-Benzyloxycarbonyl-L-glutamin und nachfolgende Abspaltung der Schutzgruppe mit HBr / Eisessig.

Diastereomer A :

$C_{17}H_{19}ClN_2O_6$ x HBr x $H_2O$ (481.74). Schmp. 186 - 188°C (Zersetz.; 2 mal aus Isopropanol / Ethanol). dc-Rf (Chloroform / Methanol 6 / 4) = 0.35. $[\alpha]_D^{20}$ +21.4 (c = 0.5 ; Methanol). Ausbeute: 16.2 %.
Das Diastereomer B wurde nicht isoliert.

Beispiel Nr. 123

threo-5-[2-Chlorphenyl-(α-L-glutamyloxy)-methyl]-4-methoxy-2(5H)-furanon Hydrobromid ; Diastereomer A :

Herstellung durch Veresterung von threo-5-(2-Chlorphenylhydroxymethyl)-4-methoxy-2(5H)-furanon mit N-Benzyloxycarbonyl-L-glutaminsäure-5-tert. butylester und nachfolgende Abspaltung der N-Benzyloxycarbonyl- und der tert. Butoxy-Schutzgruppe.

Diastereomer A:

$C_{17}H_{18}ClNO_7$ x HBr (464.71). Schmp. 190 - 194°C ( 2 mal Isopropanol). dc-Rf (Chloroform / Methanol 6 / 4) = 0.16 $[\alpha]_D^{20}$ +11.1 (c = 1; Methanol. Ausbeute : 25.2 %.
Das Diastereomer B wurde nicht isoliert.

Beispiel Nr. 124

threo-5-[N-Acetyl-L-methionyloxy-(2-chlorphenyl)-methyl]-4-methoxy-2(5H)-furanon ; Diastereomer A:

Herstellung durch Veresterung von threo-5-(2-Chlorphenylhydroxymethyl)-4-methoxy-2(5H)-furanon mit N-Acetyl-L-methionin.

Diastereomer A :

$C_{19}H_{22}ClNO_6S$ (427.91). Schmp. 107 - 110°C (2 mal aus Ethylacetat / Pentan). dc-Rf (Toluol / Aceton 6 / 4) = 0.53. $[\alpha]_D^{20}$ -0.7 (c = 1 ; Methanol). Ausbeute : 12 %.
Das Diastereomer B wurde nicht isoliert.

Beispiel Nr. 125

threo-5-[2-Chlorphenyl-(5-oxo-L-prolyloxy)-methyl]-4-methoxy-2(5H)-furanon ; Diastereomere A und B:

Herstellung durch Veresterung von threo-5-(2-Chlorphenylhydroxymethyl)-4-methoxy-2(5H)-furanon mit 5-Oxo-L-prolin und Trennung der Diastereomeren mittels Säulenchromatographie an Kieselgel mit Chloroform / Methanol 98 / 2 als Eluens.

Diastereomer A :

$C_{17}H_{16}ClNO_6$ (365.78). Zersetzungsbereich 75 - 130°C (aus tert. Butylmethylether). dc-Rf (Chloroform / Methanol 95 / 5) = 0.37. $[\alpha]_D^{20}$ +4.0 (c = 1; Methanol).

Diastereomer B:

$C_{17}H_{16}ClNO_6$ (365.78). Schmp. 135 -145°C (aus Dichlormethan / Pentan). dc-Rf (Chloroform / Methanol 95 / 5) = 0.29. $[\alpha]_D^{20}$ -2.7 (c = 1; Methanol).

Beispiel Nr. 126

threo-5-[N-Benzyloxycarbonyl-L-prolyloxy-(2-bromphenyl)-methyl]-4-methoxy-2(5H)-furanon ; Diastereomere A und B :

Herstellung durch Veresterung von threo-5-(2-Bromphenylhydroxymethyl)-4-methoxy-2(5H)-furanon mit N-Benzyloxycarbonyl-L-prolin.

Diastereomer A :

$C_{25}H_{24}BrNO_7$ (530.39) . Schmp. 136 - 137°C (aus Isopropanol). dc-Rf (Toluol / Aceton 8 / 2) = 0.28. $[\alpha]_D^{20}$ -26.2 (c = 1; Methanol). Ausbeute : 37.2 %

Diastereomer B :

$C_{25}H_{24}BrNO_7$ (530.39). Schmp. 106 - 109°C (aus Isopropanol / tert. Butylmethylether). dc-Rf (Toluol / Aceton 8 / 2) = 0.32. $[\alpha]_D^{20}$ -46.0 (c = 1; Methanol). Ausbeute : 7.3 %.
Aus den Mutterlaugen wurden weitere 47 % eines Gemisches der beiden Diastereomeren erhalten.

Beispiel Nr. 127

threo-5-[2-Bromphenyl-(L-prolyloxy)-methyl]-4-methoxy-2(5H)-furanon Hydrobrobromid ; Diastereomere A und B :

Herstellung durch Abspaltung der Schutzgruppe aus den Substanzen des Beispiels Nr. 126 mittels HBr / Eisessig.

Diastereomer A :

$C_{17}H_{18}BrNO_5$ x HBr (477.17). Schmp. 222 - 224°C (aus Ethylacetat). dc-Rf (Butanol; Polygram Sil G) = 0.12. $[\alpha]_D^{20}$ -12.3 (c =1; Methanol). Ausbeute : 94.4 %.

Diastereomer B:

$C_{17}H_{18}Br\ NO_5$ x HBr (477.17). Schmp. 191 - 193°C (aus Ethylacetat). dc-Rf (Butanol; Polygram Sil G) = 0.09. $[\alpha]_D^{20}$ -22.4 (c = 1; Methanol). Ausbeute : 60 %.

Beispiel Nr. 128

threo-5-[N-Benzyloxycarbonyl-L-prolyloxy-(2-fluorphenyl)-methyl]-4-methoxy-2(5H)-furanon; Diastereomere A und B :

Herstellung durch Veresterung von threo-5-(2-Fluorphenylhydroxymethyl)-4-methoxy-2(5)-furanon mit N-Benzyloxycarbonyl-L-prolin.

Diastereomer A:

$C_{25}H_{24}FNO_7$ (469.48). Schmp. 169 - 171°C (aus Methanol). dc-Rf (Toluol / Aceton 8 / 2) = 0.25. $[\alpha]_D^{20}$ +2.0 (c = 1; Methanol). Ausbeute : 27.7 %.

Diastereomer B :

$C_{25}H_{24}FNO_7$ (469.48). Schmp. 138 - 140°C (aus Ethanol). dc-Rf (Toluol / Aceton 8 / 2) = 0.29. $[\alpha]_D^{20}$-84.3 (c = 1; Methanol). Ausbeute : 30.2 %.
Aus den Mutterlaugen wurden weitere 17.3 % eines Gemisches der beiden Diastereomeren gewonnen.

Beispiel Nr. 129

threo-5-[2-Fluorphenyl-(L-prolyloxy)-methyl]-4-methoxy-2(5H)-furanon Hydrobromid ; Diastereomere A und B:

Herstellung durch Abspaltung der Schutzgruppe aus den Substanzen des Beispiels Nr. 128.

Diastereomer A :

$C_{17}H_{18}FNO_5$ x HBr (416.26). Schmp. 208 - 210°C (Zersetz.; aus Ethanol). dc-Rf (Chloroform / Methanol 9 / 1) = 0.34 . $[\alpha]_D^{20}$ +15.4 (c = 0.57; Methanol). Ausbeute : 87.5 % .

Diastereomer B :

$C_{17}H_{18}FNO_5$ x HBr (416.26). Schmp. 175 - 180°C (aus Ethanol). dc-Rf (Chloroform / Methanol 9 / 1) 0.31. $[\alpha]_D^{20}$ -58.8 (c = 0.57; Methanol). Ausbeute : 77.6 %.

Beispiel Nr. 130

threo-5-[2.4-Dichlorphenyl-(glycyloxy)-methyl]-4-methoxy-2(5H)-furanon Hydrobromid :

Herstellung durch Veresterung von threo-5-(2.4-Dichlorphenylhydroxymethyl)-4-methoxy-2(5H)-furanon mit N-Benzyloxycarbonylglycin und nachfolgende Abspaltung der Schutzgruppe mittels HBr / Eisessig.
$C_{14}H_{13}Cl_2NO_5$ x HBr (427.10). Schmp. 222 - 223°C (aus Ethanol) dc-Rf (Chloroform / Methanol 6 / 4) = 0.57. Ausbeute : 79.1 %.

Beispiel Nr. 131

threo-5-[2.4-Dichlorphenyl-(glycyloxy)-methyl]-4-methoxy-2(5H)-furanon Hydrochlorid :

Herstellung durch Überführung des Hydrobromids aus Beispiel Nr. 130 in das Hydrochlorid.
$C_{14}H_{13}Cl_2NO_5$ x HCl (382.64). Schmp. 206 - 208°C (2 x aus Methanol / Isopropanol).

Beispiel Nr. 132

threo-5-[N-Acetylglycyloxy-(2.4-dichlorphenyl)-methyl]-4-methoxy-2(5H)-furanon.

Herstellung durch Veresterung von threo-5-(2.4-Dichlorphenylhydroxymethyl)-4-methoxy-2(5H)-furanon mit N-Acetylglycin.
$C_{16}H_{15}Cl_2NO_6$ (388.22). Schmp. 197 - 198°C (2 mal aus Ethanol). dc-Rf (Chloroform / Methanol 95 / 5) = 0.27. Ausbeute : 49.7 %.

Beispiel Nr. 133

threo-5-[L-Alanyloxy-(2.4-dichlorphenyl)-methyl]-4-methoxy-2(5H)-furanon-Hydrobromid :

Herstellung durch Veresterung von threo-5-(2.4-Dichlorphenylhydroxymethyl)-4-methoxy-2(5H)-furanon mit N-Benzyloxycarbonyl-L-alanin und nachfolgende Abspaltung der Schutzgruppe. Man erhält nach Kristallisation aus Ethylacetat ein Gemisch der Diastereomeren A und B, das nicht weiter aufgetrennt wurde.
$C_{15}H_{15}Cl_2NO_5$ x HBr (441.19). Schmp. 173 - 175°C. Dc-Rf (Chloroform / Methanol 6 / 4) = 0.6. $[\alpha]_D^{20}$ +0.9 (c = 1; Methanol). Ausbeute : 79.8 %.

Beispiel Nr. 134

threo-5-[N-Benzyloxycarbonyl-L-prolyloxy-(2.4-dichlorphenyl)-methyl]-4-methoxy-2(5H)-furanon. Diastereomere A und B :

Herstellung durch Veresterung von threo-5-(2.4-Dichlorphenylhydroxymethyl)-4-methoxy-2(5H)-furanon mit N-Benzyloxycarbonyl-L-prolin.

Diastereomer A :

$C_{25}H_{23}Cl_2NO_7$ (520.38). Schmp. 111-113°C (aus Ethanol). dc-Rf (Chloroform / Methanol 6 / 4) = 0.74. $[\alpha]_D^{20}$ +19.9 (c = 1; Methanol). Ausbeute: 33 %.

Diastereomer B :

$C_{25}H_{23}Cl_2NO_7$ (520.38). Schmp. 117 - 119°C (aus Ethanol). dc-Rf (Toluol / tert.Butylmethylether 7 / 3) = 0.16. $[\alpha]_D^{20}$: -83.9 (c = 1; Methanol). Ausbeute: 28.4 %.

Beispiel Nr. 135

threo-5-[2.4-Dichlorphenyl-(L-prolyloxy)-methyl]-4-methoxy-2(5H)-furanon Hydrobromid ; Diastereomere A und B :

Herstellung durch Abspaltung der Schutzgruppe aus den Substanzen des Beispiels Nr. 134 mittels HBr Eisessig.

Diastereomer A :

$C_{17}H_{17}Cl_2NO_5$ x HBr (467.16). Schmp. 198 - 200°C (aus Ethylacetat). dc-Rf (Chloroform / Methanol 95 / 5) = 0.26. $[\alpha]_D^{20}$ +20.2 (c = 1; Methanol). Ausbeute : 87.7 %.

Diastereomer B:

$C_{17}H_{17}Cl_2NO_5$ x HBr (467.16). Schmp. 179 - 180°C (aus Ethylacetat / n-Hexan). dc-Rf (Chloroform / Methanol 95 / 5) = 0.26. $[\alpha]_D^{20}$ -55.8 (c = 1; Methanol). Ausbeute : 90.8 %.

Beispiel Nr. 136

threo-5-[N-Benzyloxycarbonyl-L-prolyloxy-(2.5-dichlorphenyl)-methyl]-4-methoxy-2(5H)-furanon; Diastereomere A und B :

Hergestellt durch Veresterung von threo-5-(2.5-Dichlorphenylhydroxymethyl)-4-methoxy-2(5H)-furanon mit N-Benzyloxycarbonyl-L-prolin.

Diastereomer A :

$C_{25}H_{23}Cl_2NO_7$ (520.38). Schmp. 90 - 94°C (2 x aus Isopropanol / n-Hexan). dc-Rf (n-Butanol ) = 0.55. $[\alpha]_D^{20}$ +7.9 (c = 0.5; Methanol).

Diastereomer B :

$C_{25}H_{23}Cl_2NO_7$ (520.38). Schmp. 90- 95°C (aus Butanol / n-Pentan). dc-Rf (n-Butanol) = 0.60. $[\alpha]_D^{20}$ -55.8 (c = 0.5; Methanol).

Beispiel Nr. 137

threo-5-[2.5-Dichlorphenyl-(L-prolyloxy)-methyl)-4-methoxy-2(5H)-furanon Hydrobromid ; Diastereomere A und B.

Herstellung durch Abspaltung der Schutzgruppen bei den Substanzen des Beispiels Nr. 136 mittels HBr / Eisessig.

Diastereomer A :

$C_{17}H_{17}Cl_2NO_5$ x HBr (467.16). Schmp. 200 - 202°C (aus Ethanol). dc-Rf (Chloroform / Methanol 6 / 4) = 0.68. $[\alpha]_D^{20}$ +16.3 (c = 1 ; Methanol ). Ausbeute : 91.4 %.

Diastereomer B :

$C_{17}H_{17}Cl_2NO_5$ x HBr (467.16). Schmp. 171 - 173°C (aus Ethylacetat). dc-Rf (Chloroform / Methanol 95 / 5) = 0.3. $[\alpha]_D^{20}$ -56.7 (c = 1; Methanol).

Beispiel Nr. 138

threo-5-[N-Benzyloxycarbonyl-L-prolyloxy-(2-trifluormethylphenyl)-methyl]-4-methoxy-2(5H)-furanon ; Diastereomere A und B :

Herstellung durch Veresterung von threo-4-Methoxy-5-(2-trifluormethylphenylhydroxymethyl)-2(5H)-furanon mit N-Benzyloxycarbonyl-L-prolin.

Diastereomer A :

$C_{26}H_{24}F_3NO_7$ (519.49). Schmp. 136 - 138°C (aus Isopropanol). dc-Rf (Toluol / Aceton 8 / 2) = 0.33. $[\alpha]_D^{20}$ +10.2 (c = 0.5 ; Methanol). Ausbeute : 27.7 %.

Diastereomer B :

$C_{26}H_{24}F_3NO_7$ (519.49)

Fraktion 1 :

Schmp. 95 - 100°C (aus Isopropanol / Pentan). dc-Rf (Toluol / Aceton 8 / 2) = 0.35. $[\alpha]_D^{20}$ -53.0 (c = 0.5 ; Methanol). Ausbeute 20.8 % (enthält noch Anteile an Diastereomer A).

Fraktion 2 :

Schmp. 108 - 113°C $[\alpha]_D^{20}$ -79.4 (c = 0.5; Methanol) Ausbeute : 0.4 % (dc : diastereomeren-rein).

Fraktion 3 :

Schmp. 114 - 121°C. $[\alpha]_D^{20}$ -60.4 (c = 0.5 ; Methanol). Ausbeute : 9.6 % (enthält noch Anteile des Diastereomeren A).

Fraktion 4 :

Öl. Ausbeute : 33.9 %. Gemisch der Diastereomeren A und B (hauptsächlich B).

Beispiel Nr. 139

threo-4-Methoxy-5-[L-prolyloxy-(2-trifluormethylphenyl)-methyl]-2(5H)-furanon Hydrobromid ; Diastereomere A und B :

Herstellung durch Abspaltung der Schutzgruppe bei den Substanzen des Beispiels Nr. 138 mittels HBr / Eisessig.

Diastereomer A :

$C_{18}H_{18}F_3NO_5$ x HBr (466.27). Schmp. 210 - 211°C (aus Ethylacetat). dc-Rf (Chloroform / Methanol 6 / 4) = 0.58. $[\alpha]_D^{20}$ +24.2 (c = 0.5 ; Methanol) . Ausbeute : 90.2 %. Diastereomeren-Reinheit > 97 % ($^1$H-NMR-spektroskopisch ermittelt).

Diastereomer B :

$C_{18}H_{18}F_3NO_5$ x Hbr (466.27)

Fraktion 1 (Acidolyse von Fraktion 4 aus Beispiel Nr. 138 B) : Schmp. 192 - 194°C (aus Ethylacetat). dc-Rf (Chloroform / Methanol 6 / 4) = 0.60. $[\alpha]_D^{20}$ -72.8 (c = 0.5 ; Methanol). Ausbeute : 19.4 %. Diastereomerenreinheit > 99 % ($^1$H-NMR-spektroskopisch).

Fraktion 2 (Acidolyse von Fraktion 1 aus Beispiel Nr. 138 B) : Schmp. 185 - 189°C ; $[\alpha]_D^{20}$ -56.4 (c = 0.5 ;

Methanol). Ausbeute :10.6 %. Diastereomerenreinheit = 77.5 % ($^1$H-NMR-spektroskopisch werden 22.5 % Gehalt an Diastereomer A ermittelt).

Fraktion 3 (Acidolyse von Fraktion 3 aus Beispiel Nr. 138 B) : Schmp. 189 - 190°C ; $[\alpha]_D^{20}$ -48.6 (c = 0.5 ; Methanol). Ausbeute : 87.9 % (enthält noch Anteile an Diastereomer A).

Beispiel Nr. 140

threo-5-[N-Benzyloxycarbonyl-L-prolyloxy-(2-methylphenyl)-methyl]-4-methoxy-2(5H)-furanon. Diastereomere A und B :

Herstellung durch Veresterung von threo-4-Methoxy-5-(2-methylphenylhydroxymethyl)-2(5H)-furanon mit N-Benzyloxycarbonyl-L-prolin.

Diastereomer A :

$C_{26}H_{27}NO_7$ (465.52) . Schmp. 132 - 134°C (aus Isopropanol). dc-Rf (Toluol / Aceton 8 / 2) = 0.31. $[\alpha]_D^{20}$ +13.8 (c = 0.5 ; Methanol). Ausbeute : 32.2 %.

Diastereomer B :

$C_{26}H_{27}NO_7$ (465.52). Schmp. 114 - 116°C (aus Isopropanol / Ethanol 1 / 1). dc-Rf (Toluol / Aceton 8 / 2) = 0.41. $[\alpha]_D^{20}$ -85.8 (c = 0.5 ; Methanol) . Ausbeute : 22.0 % .
Aus den Mutterlaugen erhält man ein Gemisch der beiden Diastereomeren A und B in 38.1 % Ausbeute.

Beispiel Nr. 141 :

threo-4-Methoxy-5-[2-methylphenyl-(L-prolyloxy)-methyl]-2(5H)-furanon Hydrobromid ; Diastereomere A und B :

Herstellung durch Abspaltung der Schutzgruppe bei den Substanzen des Beispiels Nr. 140 mittels HBr / Eisessig.

Diastereomer A :

$C_{18}H_{21}NO_5$ x HBr (412.30). Schmp. 222 - 225°C (Zersetz. ; aus Ethylacetat). dc-Rf (Chloroform / Methanol 6 / 4) = 0.58. $[\alpha]_D^{20}$ +5.8 (c = 0.5; Methanol). Diastereomeren-Reinheit > 98 %. ($^1$H-NMR-spektroskopisch). Ausbeute : 70.6 %.
Aus der Mutterlauge wird weiteres Diastereomer A mit Schmp. 221 - 223°C (Zersetz.) und $[\alpha]_D^{20}$ +5.0 (c = 0.5 ; Methanol) in 25.1 % Ausbeute erhalten.

Diastereomer B :

$C_{18}H_{21}NO_5$ x HBr (412.30). Schmp. 195 - 196°C (Zersetzung ; aus Ethylacetat). dc-Rf (Chloroform / Methanol 6 / 4) = 0.55. $[\alpha]_D^{20}$ -58.0 (c = 0.5 ; Methanol). Diastereomeren-Reinheit > 99 % ($^1$H-NMR-spektroskopisch). Ausbeute : 92.7 %.

Acidolyse des Diastereomeren-Gemisches aus der Mutterlauge des Beispiels Nr. 140 ergibt in 14.2 % Ausbeute Diastereomer A mit Schmp. 219 - 22°C (Zersetzung ; aus Ethanol) und $[\alpha]_D^{20}$ +5.0 (c = 0.5 ; Methanol), sowie in 36.2 % Ausbeute Diastereomer B mit Schmp. 205 - 207 °C (Zersetzung ; aus Ethanol) und $[\alpha]_D^{20}$ -50.2 (c = 0.5 ; Methanol).

Beispiel Nr. 142

(+)-threo-5-(2-Chlorphenylhydroxymethyl)-4-methoxy-2(5H)-furanon :

Verfahren a :

Das Gemisch aus 17.3 g (40 mmol) threo-5-[2-Chlorphenyl-(L-prolyloxy)-methyl]-4-methoxy-2(5H)-furanon Hydrobromid Diastereomer A (Herstellung siehe Beispiel Nr. 103), 1 Liter wasserfreiem Methanol und 5.9 g (80 mmol) wasserfreiem Lithiumcarbonat wird 4 Tage bei 0°C gerührt, danach mit 80 ml 1M-Salzsäure versetzt und im Vakuum auf ca. 150 ml konzentriert. Man gibt 300 ml Chloroform zu, extrahiert die Chloroformphase mit 50 ml 1M-Salzsäure und mit 2 x 50 ml Wasser, trocknet sie über wasserfreiem Natriumsulfat und evaporiert. Umkristallisation aus Ethylacetat / n-Pentan ergibt nach Trocknen bei 100°C im Vakuum 4.98 g (19.6 mmol) der Titelverbindung mit Schmp. 127°C - 128°C und $[\alpha]_D^{20}$ +98.0 (c = 0.2 ; Aceton). $C_{12}H_{11}ClO_4$ (254.67). Elementaranalyse und $^1$H-NMR-Spektrum stimmen mit denen der racemischen Verbindung des Beispiels Nr. 64 überein.
Ausbeute : 49 %.

Verfahren b :

750 mg (1.73 mmol) threo-5-[2-Chlorphenyl-(L-prolyloxy)-methyl]-4-methoxy-2(5H)-furanon Hydrobromid Diastereomer A werden in 50 ml Wasser 16 Stdn. unter Rückflußkochen erhitzt. Nach dem Abkühlen saugt man auskristallisiertes Produkt ab. Umkristallisation aus Tetrachlormethan ergibt 251 mg (0.99 mmol) der Titelverbindung mit Schmp. 123°C und $[\alpha]_D^{20}$ +99.0 (c = 0.25 ; Aceton). Ausbeute 57 %.

Beispiel Nr. 143

(-)-threo-5-(2-Fluorphenylhydroxymethyl)-4-methoxy-2(5H)-furanon :

Das Gemisch aus 12.5 g (30 mmol) threo-[2-Fluorphenyl-(L-prolyloxy)-methyl]-4-methoxy-2(5H)-furanon Hydrobromid Diastereomer B (aus Beispiel Nr. 129) und 400 ml Wasser wird 20 Stdn. bei 85°C und 2 Stdn. bei 100°C gerührt. Nach dem Abkühlen extrahiert man das Produkt mit 200 ml Dichlormethan, wäscht den Extrakt mit 3 x 70 ml Wasser und extrahiert die vereinigten Waschwässer mit 100 ml Dichlormethan.

Die vereinigten Dichlormethanextrakte werden evaporiert und aus tert. Butylmethylether umkristallisiert. Man erhält nach Trocknen bei 70°C im Vakuum 6.513 g (27.3 mmol) der Titelverbindung mit Schmp. 152°C - 154°C und $[\alpha]_D^{20}$ -111.8 (c = 0.51 ; Aceton). Ausbeute : 91 %.

| | |
|---|---|
| Analyse : | $C_{12}H_{11}FO_4$ (238.22) |
| Ber. : | C (60.50), H (4.66), F (7.98) |
| Gef. : | C (60.32). H (4.60), F (8.0) |

In analoger Weise, wie in den Beispielen Nr. 142 und 143 beschrieben, werden durch saure oder durch basische katalysierte Spaltung der entsprechenden Aminosäureester die folgenden optisch aktiven threo-4-Methoxy-5-phenylhydroxymethyl-2(5H)-furanon-Derivate erhalten.

Beispiel Nr. 144

(-)-threo-5-(2-Chlorphenylhydroxymethyl)-4-methoxy-2(5H)-furanon :

Herstellung aus dem Diastereomeren B des Beispiels Nr. 103.
$C_{12}H_{11}ClO_4$ (254.67). Schmp. 125 - 126°C (aus Wasser). $[\alpha]_D^{20}$ -96.6 (c = 0.5 ; Aceton). Ausbeute : 85.6 % .

Beispiel Nr. 145

(+)-threo-4-Methoxy-5-phenylhydroxymethyl-2(5H)-furanon :

Herstellung aus dem Diastereomeren A des Beispiels Nr. 110.
$C_{12}H_{12}O_4$ (220.28). Schmp. 160 - 163°C (2 x aus Ethanol / Wasser). $[\alpha]_D^{20}$ +101.2 (c = 0.5; Aceton). Ausbeute : 75.9 %.

Beispiel Nr. 146

(-)-threo-4-Methoxy-5-phenylhydroxymethyl-2(5H)-furanon :

Herstellung aus dem Diastereomeren B des Beispiels Nr. 110.
$C_{12}H_{12}O_4$ (220.28). $[\alpha]_D^{20}$ -103.6 (c = 0.5 ; Aceton ). Ausbeute 64.4 % .

Beispiel Nr. 147

(+)-threo-5-(2-Bromphenylhydroxymethyl)-4-methoxy-2(5H)-furanon :

Herstellung aus dem Diastereomeren A des Beispiels Nr. 127.
$C_{12}H_{11}BrO_4$ (299.13). Schmp. 134 - 138°C (aus 80 %igem Ethanol). $[\alpha]_D^{20}$ +73.6 (c = 0.5 ; Aceton). Ausbeute : 74.3 %.

Beispiel Nr. 148

(+)-threo-5-(2.4-Dichlorphenylhydroxymethyl)-4-methoxy-2(5H)-furanon :

Herstellung aus dem Diastereomeren A des Beispiels Nr. 135.
$C_{12}H_{10}Cl_2O_4$ (289.12). Schmp. 140 - 143°C (aus Isopropanol). $[\alpha]_D^{20}$ +118.5 (c = 0.2 ; Aceton). Ausbeute 69.9 %.

Beispiel Nr. 149

(-)-threo-5-(2.4-Dichlorphenylhydroxymethyl)-4-methoxy-2(5H)-furanon :

Herstellung aus dem Diastereomeren B des Beispiels Nr. 135.
$C_{12}H_{10}Cl_2O_4$ (289.12). Schmp. 138 - 141°C (aus Isopropanol). $[\alpha]_D^{20}$ -119.5 (c = 0.2 ; Aceton). Ausbeute : 77.5 %.

Beispiel Nr. 150

(+)-threo-5-[N-Acetylglycyloxy-(2-chlorphenyl)-methyl]-4-methoxy-2(5H)-furanon :

Herstellung analog Beispiel Nr. 106 durch Veresterung von (+)-threo-5-(2-Chlorphenylhydroxymethyl)-4-methoxy-2(5H)-furanon (Herstellung siehe Beispiel Nr. 142) mit N-Acetylglycin.
$C_{16}H_{16}ClNO_6$ (353.77). Schmp. 170 - 171°C (aus Isopropanol / Methanol 1 / 1). dc-Rf (Chloroform / Methanol 95 / 5) = 0.45. $[\alpha]_D^{20}$ +4.8 (c = 0.5; Methanol p.a.). Ausbeute : 63.5 %.

Beispiel Nr. 151

(+)-threo-5-[2-Chlorphenyl-(glycyloxy)-methyl]-4-methoxy-2(5H)-furanon Hydrobromid :

Unter Rühren und Kühlen auf -5°C gibt man zu der Mischung aus 7.64 g (30 mmol) (+)-threo-5-(2-Chlorphenylhydroxymethyl)-4-methoxy-2(5H)-furanon (Herstellung siehe Beispiel Nr. 142), 6.28 g (30 mmol) N-Benzyloxycarbonylglycin und 150 ml wasserfreiem Dichlormethan nacheinander 0.3 g (2 mmol) 4-Pyrrolidinopyridin und die Lösung von 6.8 g (33 mmol) Dicyclohexylcarbodiimid in 30 ml wfr. Dichlormethan. Nach 4 Stdn. Rühren bei -5°C filtriert man ausgefallenen N,N'-Dicyclohexylharnstoff ab, evaporiert das Filtrat, löst in 100 ml Aceton, läßt über Nacht bei -8°C stehen und filtriert restlichen N,N'-Dicyclohexylharnstoff ab. Evaporieren des Filtrats ergibt 14.1 g öliges (+)-threo-5-[N-Benzyloxycarbonylglycyloxy-(2-chlorphenyl)-methyl]-4-methoxy-2(5H)-furanon.
Dieses wird in 300 ml Ethylacetat gelöst, unter Eiskühlung mit 30 ml 33 %iger Bromwasserstofflösung in Eisessig versetzt und 24 Stdn. stehen gelassen. Nach Absaugen des Kristallisats, Waschen mit Ethylacetat und Trocknen bei 105°C im Vakuum erhält man 10.29 g (26.2 mmol) der Titelverbindung in Form des Hydrobromids mit Schmp. 215 - 216°C (Zersetz.) und dc-Rf (Chloroform / Methanol 95 / 5) = 0.18. Ausbeute : 87.4 % . $[\alpha]_D^{20}$ +17.4 (c = 0.5 ; Methanol).
Analyse : $C_{14}H_{14}ClNO_5$ x HBr (392.65)

| | |
|---|---|
| Ber. : | C (42.83), H (3.85), N (3.57) |
| Gef. : | C (43.20), H (4.01, N (3.41) |

Beispiel Nr. 152

(+)-threo-5-[2-Chlorphenyl-(N-pivaloylglycyloxy)-methyl]-4-methoxy-2(5H)-furanon :

Unter Rühren und Kühlen auf 0°C gibt man zu der Lösung von 2.22 g (15 mmol) 4-Pyrrolidinopyridin und 4.0 ml (50 mmol) Pyridin in 100 ml wasserfreiem Dichlormethan nacheinander 5.89 g (15 mmol) (+)-threo-5-[2-Chlorphenyl-(glycyloxy)-methyl]-4-methoxy-2(5H)-furanon Hydrobromid (Herstellung siehe Beispiel Nr. 151) und 2.0 ml (16 mmol) Pivalinsäurechlorid und rührt 4 Stdn. bei 0°C. Man wäscht mit Eiswasser und 0.1M Salzsäure frei von Pyridin und 4-Pyrrolidinopyridin , trocknet die Dichlormethanphase über wasserfreiem Natriumsulfat, evaporiert und kristallisiert aus tert. Butylmethylether / n-Pentan. Umkristallisation aus tert. Butylmethylether ergibt nach Trocknen bei 70°C im Vakuum 4.3 g (10.9 mmol) der Titelverbindung mit Schmp. 100 - 102°C und dc-Rf (Chloroform / Methanol 95 / 5) = 0.42. Ausbeute : 72 %. $[\alpha]_D^{20}$ +22.6 (c = 0.5 ; Methanol).

| | |
|---|---|
| Analyse : | $C_{19}H_{22}ClNO_6$ (395.85) |
| Ber. : | C (57.65), H (5.60), N (3.54), Cl (8.96) |
| Gef. : | C (57.85), H (5.81), N (3.58), Cl (9.3). |

Beispiel Nr. 153

(-)-threo-5-[2-Chlorphenyl-(glycyloxy)-methyl]-4-methoxy-2(5H)-furanon-Hydrobromid:

Herstellung analog Beispiel Nr. 151 durch Veresterung von (-)-threo-5-(2-Chlorphenylhydroxymethyl)-4-methoxy-2(5H)-furanon (Herstellung siehe Beispiel Nr. 144) mit N-Benzyloxycarbonylglycin und nachfolgende Abspaltung der Schutzgruppe mittels HBr/Eisessig in Ethylacetat. Umkristallisation aus Ethanol ergibt die Titelverbindung mit Schmp. 225 - 226°C (Zersetz.) und dc-Rf (Chloroform/Methanol 95/5) = 0.18. Ausbeute: 69 %. $[\alpha]_D^{20}$ -17.8 (c = 1; Methanol).

| | |
|---|---|
| Analyse : | $C_{14}H_{14}ClNO_5$ x HBr (392.65) |
| Ber. : | C(42.83), H(3.85), N(3.57), Br(20.35), Cl(9.03) |
| Gef. : | C(42.68), H(3.78), N(3.48), Br(19.8), Cl(9.1) |

Beispiel Nr. 154

threo-5-[N-(tert.Butoxycarbonyl)-2-methylalanyloxy-(2-chlorphenyl)-methyl]-4-methoxy-2(5H)-furanon:

Herstellung durch Veresterung von threo-5-(2-Chlorphenylhydroxymethyl)-4-methoxy-2(5H)-furanon mit N-(tert.-Butoxycarbonyl)-2-methylalanin. Schmp. 192 - 195°C (aus Methanol). dc-Rf (Chloroform/Methanol 95/5) = 0.58.

| | |
|---|---|
| Analyse : | $C_{21}H_{26}ClNO_7$ (439.90) |
| Ber. : | C(57.34), H(5.96), N(3.18), Cl(8.06) |
| Gef. : | C(57.24), H(5.72), N(3.15), Cl(8.0) |

Beispiel Nr. 155

threo-5-[2-Chlorphenyl-(2-methylalanyloxy)-methyl]-4-methoxy-2(5H)-furanon-Hydrobromid:

Herstellung durch Abspaltung der Schutzgruppe aus der Substanz des Beispiels Nr. 154 mittels HBr/Eisessig. Schmp. 177 - 181°C (aus Aceton). dc-Rf (Chloroform/Methanol 6/4) = 0.6. Ausbeute: 65 %.

| | |
|---|---|
| Analyse : | $C_{16}H_{18}ClNO_5$ x HBr (420.70) |
| Ber. : | C(45.68), H(4.55), N(3.33), Br(18.99), Cl(8.43) |
| Gef. : | C(44.65), H(4.42), N(3.18), Br(18.9), Cl(9.0) |

Beispiel Nr. 156

threo-5-[1-(N-Benzyloxycarbonylamino)-1-cyclohexylcarbonyloxy-(2-chlorphenyl)-methyl]-4-methoxy-2(5H)-furanon:

Herstellung durch Veresterung von threo-5-(2-Chlorphenylhydroxymethyl)-4-methoxy-2(5H)-furanon mit 1-(N-Benzyloxycarbonylamino)-1-cyclohexancarbonsäure. Schmp. 151 - 153°C (aus Isopropanol). dc-Rf (Chloroform/Methanol 9/1) = 0.6. Ausbeute: 54.6 %.

| | |
|---|---|
| Analyse : | $C_{27}H_{28}ClNO_7$ (513.98) |
| Ber. : | C (63.10), H (5.49), N (2.73), Cl (6.90) |
| Gef. : | C (63.11), H (5.49), N (2.72), Cl (7.1) |

Die zur Veresterung verwendete 1-(N-Benzyloxycarbonylamino)-1-cyclohexancarbonsäure wurde durch Umsetzung von 1-Amino-1-cyclohexancarbonsäure mit Chlorameisensäurebenzylester in Gegenwart von Natronlauge erhalten. Schmp. 151 - 154°C (aus Trichlorethylen).

Beispiel Nr. 157

threo-5-[1-Amino-1-cyclohexylcarbonyloxy-(2-chlorphenyl)-methyl]-4-methoxy-2(5H)-furanon-Hydrobromid:

Herstellung durch Abspaltung der Schutzgruppe aus der Substanz des Beispiels Nr. 156 mittels HBr/Eisessig in Ethylacetat und Umkristallisation aus Isopropanol. Schmp. 196 - 197°C. dc-Rf (Chloroform/Methanol 98/2) = 0.45. Ausbeute: 71.9 %.

| | |
|---|---|
| Analyse : | $C_{19}H_{22}ClNO_5$ x HBr (460.76) |
| Ber. : | C(49.53), H(5.03), N(3.04), Br(17.34), Cl(7.69) |
| Gef. : | C(49.45), H(5.05), N(3.03), Br(16.9), Cl(7.9). |

Beispiele für die Herstellung pharmazeutischer Zubereitungen der erfindungsgemäßen Substanzen.

A. Tabletten:

Zur Herstellung von Tabletten a 250 mg Einzelgewicht, die je nach gewünschter Wirkungsstärke 5 bis 100 mg Wirkstoff enthalten, benötigt man

| | |
|---|---|
| erfindungsgemäße Substanz | 200 bis 4 000 g |
| Zellulosepulver | 2 000 g |
| Maisstärke | 1 200 g |
| kolloide Kieselsäure | 80 g |
| Magnesiumstearat | 20 g |
| Milchzucker | ad 10 000 g |

Wirkstoff und Hilfsstoffe werden homogen vermischt und in der üblichen Weise zu Tabletten von je 250 mg Gewicht und 9 mm Durchmesser verpreßt. Falls gewünscht, werden die Tabletten mit einem Filmüberzug versehen.

B. Kapseln:

Zur Herstellung von Kapseln, die je nach gewünschter Wirkungsstärke 5 bis 100 mg Wirkstoff enthalten, benötigt man

| | | |
|---|---|---|
| erfindungsgemäße Substanz | | 500 bis 10 000 g |
| Maisstärke | | 2 000 g |
| kolloide Kieselsäure | | 300 g |
| Magnesiumstearat | | 50 g |
| Zellulosepulver | ad | 20 000 g |

Die feingepulverten Stoffe werden homogen gemischt und in Hartgelatinekapseln der Größe 2 in der Menge von 200 mg pro Kapsel abgefüllt.

C. Saft:

Zur Herstellung eines Saftes mit einem Gehalt von 0.035 bis 0.7 Gew.% Wirkstoff, je nach gewünschter Wirkungsstärke, benötigt man

| | | |
|---|---|---|
| erfindungsgemäße Substanz | | 35 bis 700 g |
| Propylenglykol | | 20 000 g |
| Glycerin | | 20 000 g |
| Methylzellulose | | 1 000 g |
| Natriumcyclamat | | 500 g |
| Saccharin-Natrium | | 50 g |
| demineralisiertes Wasser | ad | 100 000 g |

Der Wirkstoff wird fein gemahlen und homogen in die Lösung der Hilfsstoffe eingerührt.

D. Zäpfchen:

Zur Herstellung von Zäpfchen a 3 g Einzelgewicht, enthaltend 5 bis 100 mg Wirkstoff pro Zäpfchen, je nach gewünschter Wirkungsstärke, benötigt man

| | | |
|---|---|---|
| erfindungsgemäße Substanz | | 50 bis 1 000 g |
| kolloide Kieselsäure | | 60 g |
| Lecithin | | 150 g |
| Hartfett | ad | 30 000 g |

Der feingemahlene Wirkstoff wird in die Schmelze der Hilfsstoffe homogen eingerührt und zu Zäpfchen von 3 g Eigengewicht vergossen.

Zur Ermittlung der anticonvulsiven / antiepileptischen Aktivität der erfindungsgemäßen Substanzen wurde die von E. A. Swinyard et al., J. Pharmacol. exp. Therapeut. 106, 319-330 (1952) und von L. A. Woodbury et al., Arch. int.Pharmacodyn.92, 97 - 107 (1952) beschriebene Methodik verwendet. Männlichen Mäusen (NMRI) mit einem Körpergewicht von 20 - 25 g wird über Corneal-Elektroden ein Wechselstrom von 50 Hz und 50 mA während 0.2 sec. appliziert (HSE-Schock-Reizgerät ; Typ 207). Der Maximal-Elektroschock-Krampf (MES) besteht aus einer tonischen Streckung der Hinterextremitäten, klonischen Zuckungen und einem Bewußtseinsverlust. Als Wirksamkeitskriterium wird die Hemmung des Extensorenkrampfes durch die erfindungsgemäßen Substanzen betrachtet. Die Mäuse hatten vor den Experimenten freien Zugang zu Futter und Wasser. Die Testsubstanzen wurden als Suspension in 0.2 % Agar p.o. durch Schlundsonde appliziert ; die Kontrolltiere erhielten adäquate Volumina Agar. 1 Stunde und 3 Stunden nach Applikation wurde der Test auf Schutzwirkung

gegen MES durchgeführt.

In Tabelle Nr. 4 sind die Ergebnisse des MES-Tests bei Dosierungen von 50 bis 100 mg der erfindungsgemäßen Verbindungen pro kg Körpergewicht im Vergleich zu gebräuchlichen Antiepileptika aufgeführt. Als % Wirkung ist der prozentuale Anteil derjenigen Tiere angegeben, die bei dem MES-Test zu 100 % gegen den Extensorenkrampf geschützt waren. Pro Versuch wurden 10 Tiere verwendet.

In Tabelle Nr. 4 sind als ED 50-Werte diejenigen Dosierungen der erfindungsgemäßen Substanzen und von gebräuchlichen Antiepileptika aufgeführt, die im MES-Test 1 Stunde nach Applikation 50 % der Tiere vollständig vor dem Extensorenkrampf zu schützen vermochten. Die Bestimmung der ED 50-Werte erfolgte nach Lichtfield und Wilcoxon [J. Pharmacol. exp. Therapeut. 96, 99 (1949)] jeweils mit 4 bis 5 Tiergruppen zu 8 - 10 Tieren je Dosisstufe.

Die mit * gekennzeichneten ED 50-Werte sind geschätzte Werte, die aus mehreren Versuchen mit unterschiedlichen Dosierungen resultieren.

Während der oben beschriebenen Experimente wurden die Tiere innerhalb der gesamten Versuchsdauer (bis zu 4 Stunden) auf Anzeichen substanzbedingter Verhaltensänderungen und Neurotoxizität (Motilität, Muskeltonus, Atemfrequenz, Körpertemperatur und Allgemeinverhalten) beobachtet. Bis zu einer Dosierung von 100 mg / kg konnten bei allen geprüften erfindungsgemäßen Substanzen keinerlei Neurotoxizitäts-Symptome festgestellt werden. Im Vergleich hierzu sind in Tabelle Nr. 5 die unteren Grenzdosierungen gebräuchlicher Anticonvulsiva angeführt, die nach p. o. Applikation Neurotoxizitätssymptome bei Mäusen hervorrufen.

Bei sämtlichen erfindungsgemäßen Verbindungen wurde in Dosierungen bis zu 300 mg / kg p. o. keinerlei Mortalität der Mäuse festgestellt.

Die vorliegenden Untersuchungen zeigen im Ergebnis eine gute antikonvulsive Wirkung und hervorragende therapeutische Breite der erfindungsgemäßen Verbindungen.

Tabelle Nr. 4 : Ergebnisse des MES-Tests (n = 10)

| Substanz aus Beispiel Nr. | Dosis [mg / kg ] | Schutzwirkung [%] 1 h | 3 h | ED 50-Werte [mg / kg ] |
|---|---|---|---|---|
| 103 A | 100 | 100 | 100 | 10.7 |
| 103 B | 100 | 50 | 0 | 95 |
| 104 A | 50 | 100 | 100 | |
| 104 B | 100 | 60 | 0 | 105 |
| 105 | 100 | 70 | 0 | 87.5 |
| 106 | 50 | 100 | 70 | 23.1 |
| 107 A | 100 | 100 | 60 | 10-15* |
| 108 A | 100 | 90 | 0 | 70-80* |
| 110 A | 100 | 100 | 0 | 40-50* |
| 111 | 50 | 100 | 80 | 24.2 |
| 112 | 50 | 100 | 70 | 22.2 |
| 113 | 50 | 100 | 50 | 25-35* |
| 114 | 50 | 100 | 30 | 40.0 |
| 115 | 100 | 100 | 100 | 30-40* |
| 117 A | 50 | 100 | 100 | 16 |
| 117 B | 50 | 90 | 0 | 25.3 |
| 119 B | 50 | 100 | 20 | 37.6 |
| 121 A | 50 | 100 | 90 | |
| 122 A | 50 | 100 | 100 | 15-20* |
| 123 A | 50 | 70 | 20 | |
| 124 A | 100 | 100 | 20 | 25-35* |
| 125 A | 50 | 100 | 80 | |
| 127 A | 50 | 100 | 100 | |
| 129 A | 100 | 100 | 100 | 40-50* |
| 129 B | 100 | 50 | 0 | 95-105* |
| 130 | 100 | 100 | 100 | 40-50* |
| 131 | 100 | 100 | 100 | 54.5 |
| 132 | 100 | 20 | 50 | |
| 133 | 100 | 100 | 100 | 60-70* |
| 135 A | 100 | 100 | 100 | 65-75* |
| 136 A | 50 | 60 | 50 | 40-50* |

Fortsetzung von Tabelle Nr. 4

| Substanz aus Beispiel Nr. | Dosis [mg / kg ] | Schutzwirkung [%] 1 h | 3 h | ED 50-Werte [mg / kg ] |
|---|---|---|---|---|
| 137 A | 50 | 100 | 100 | |
| 138 A | 100 | 30 | 20 | |
| 139 A | 50 | 100 | 50 | 15-20* |
| 139 B | 100 | 40 | 10 | |
| 141 A | 100 | 40 | 10 | 110-120* |
| 150 | 50 | 100 | 100 | 14.4 |
| 151 | 50 | 100 | 100 | 12.2 |
| 152 | 50 | 100 | 90 | 16.3 |
| 153 | 100 | 40 | 0 | |
| 155 | 25 | 50 | 10 | 20-30* |
| 157 | 100 | 100 | 40 | 27.5 |
| Carbamazepin | 50 | 100 | 100 | 14.6 |
| Diazepam | 50 | 100 | 100 | 13.8 |
| Phenytoin | 50 | 100 | 100 | 9.1 |
| Ethosuximid | 100 | 0 | 0 | >250** |
| Phenobarbital | 100 | 100 | 100 | 19.2 |
| Valproinsäure (ip) | 100 | 0 | 0 | 205 |

* geschätzte Werte

** 4 Stunden nach Verabreichung

Tabelle Nr. 5 : Neurotoxische Grenzdosis an der Maus

| Substanz | Dosis p. o. [mg / kg] |
|---|---|
| Carbamazepin | 100 |
| Diazepam | 40 |
| Diphenylhydantoin | 100 |
| Ethosuximid | 500 |
| Pentobarbital | 60 |
| Phenobarbital | 60 |
| Valproinsäure | 350 |
| erfindungsgemäße Verbindungen | alle > 100 |

**Patentansprüche**

1. Aminosäureester der allgemeinen Formel I,

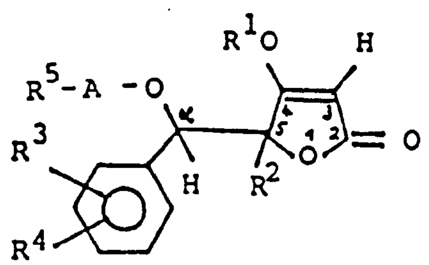

(I)

worin die Sauerstoffatome an C-5 und C-α relativ zueinander die threo-Position einnehmen und worin
$R^1$ eine Methyl- oder Ethylgruppe,
$R^2$ ein Wasserstoffatom oder eine Methylgruppe,
$R^3$ und $R^4$ unabhängig voneinander ein Wasserstoff-, Fluor-, Chlor- oder Bromatom, eine Niederalkylgruppe mit 1 bis 3 C-Atomen, eine Perfluorniederalkylgruppe mit 1 bis 3 C-Atomen, die Difluormethoxy- oder Nitrogruppe,
A den Acylrest einer der folgenden Aminosäuren:

Alanin, Leucin, Isoleucin, Norleucin, Valin, Norvalin, Phenylglycin, Phenylalanin, Prolin, 5-Oxoprolin, Glutaminsäure, Glutamin, Asparaginsäure, Asparagin, Methionin, Glycin, β-Alanin, 4-Aminobuttersäure, 2-Methylalanin oder einer 1-Amino-1-cycloalkancarbonsäure mit einem 3-7-gliedrigen Cycloalkylrest,

wobei jeweils ein Wasserstoffatom der Aminogruppe dieser Aminosäuren durch den Rest $R^5$ ersetzt ist, und

$R^5$ ein Wasserstoffatom, einen Niederalkanoyl-Rest mit 2 bis 5 C-Atomen, den Benzyloxycarbonyl-Rest (Z) oder den tert.-Butoxycarbonyl-Rest (BOC) bedeuten,
sowie deren pharmakologisch verträgliche Säureadditionssalze.

2. Aminosäureester nach Anspruch 1 in Form ihrer Racemate.

3. Aminosäureester nach Anspruch 1 in Form ihrer Enantiomere oder in Form von Enantiomerengemischen.

4. Aminosäureester nach Anspruch 1 in Form ihrer Diastereomere oder in Form von Diastereomerengemischen.

5. Aminosäureester nach einem der Ansprüche 1 bis 4, dadurch gekennzeichnet, daß einer der Reste $R^3$ oder $R^4$ ein Wasserstoffatom und der andere ein in 2'-Stellung befindliches Fluor-, Chlor- oder Bromatom oder eine in 2'-Stellung befindliche Methyl- oder Trifluormethylgruppe ist.

6. Aminosäureester nach einem der Ansprüche 1 bis 4, dadurch gekennzeichnet, daß einer der beiden Reste $R^3$ oder $R^4$ ein in 2'-Stellung befindliches Fluor-, Chloroder Bromatom oder eine in 2'-Stellung befindliche Trifluormethylgruppe und der andere ein Chlor- oder Bromatom oder eine Trifluormethylgruppe, jeweils in 4'-, 5'- oder 6'-Stellung, ist.

7. Aminosäureester nach einem der Ansprüche 1 bis 6, dadurch gekennzeichnet, daß ihre durch den threo-5-Arylhydroxymethyl-2(5H)-furanon-Rest gebildete alkoholische Komponente, für sich allein gesehen, optisch rechtsdrehend ist.

8. Verfahren zur Herstellung von Aminosäureestern der allgemeinen Formel I,

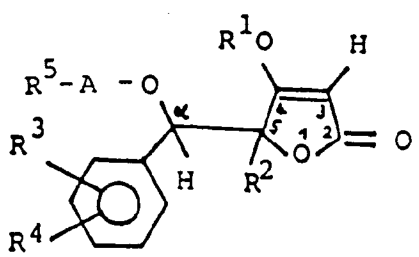

(I)

worin die Sauerstoffatome an C-5 und C-$\alpha$ relativ zueinander die threo-Position einnehmen und worin $R^1$, $R^2$, $R^3$, $R^4$, A und $R^5$ die in Anspruch 1 genannten Bedeutungen besitzen, aber $R^5 \neq$ H ist, dadurch gekennzeichnet, daß

(A) ein threo-4-Alkoxy-5-arylhydroxymethyl-2(5H)-furanon der allgemeinen Formel II,

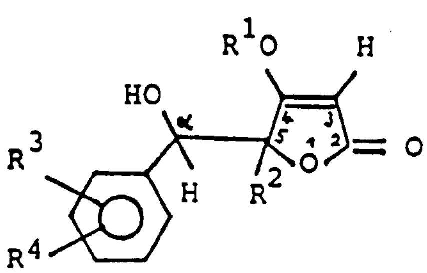

(II)

worin $R^1$, $R^2$, $R^3$ und $R^4$ die in Anspruch 1 genannten Bedeutungen besitzten, mit einem reaktiven Aminosäurederivat der allgemeinen Formel III

$R^5$-A-X (III)

umgesetzt wird, worin

A die in Anspruch 1 genannte Bedeutung besitzt,

$R^5$ einen Niederalkanoyl-Rest mit 2 bis 5 C-Atomen, den Benzyloxycarbonyl-Rest (Z) oder den tert.-Butoxycarbonyl-Rest (BOC) bedeutet und

X OH, Cl, Br oder die Gruppe O-A-$R^5$ ist,

unter Bildung eines Diastereomerengemischs des entsprechenden Aminosäureesters der allgemeinen Formel I.

9. Verfahren nach Anspruch 8, dadurch gekennzeichnet, daß in Stufe (A) ein reaktives Aminosäurederivat der allgemeinen Formel III

$R^5$-A-X (III)

verwendet wird, worin $R^5$ und X die in Anspruch 8 genannte Bedeutung besitzen und A ein Acylrest einer der in Anspruch 1 genannten chiralen Aminosäuren ist.

10. Verfahren nach Anspruch 9 zur Herstellung der Aminosäureester der allgemeinen Formel I, worin $R^5 \neq$ H, dadurch gekennzeichnet, daß

(B) das in Stufe (A) erhaltene Diastereomerengemisch in an sich bekannter Weise in die Diastereomeren der allgemeinen Formeln VIIa und VIIb,

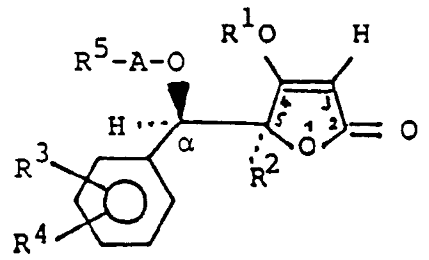

(VIIa)

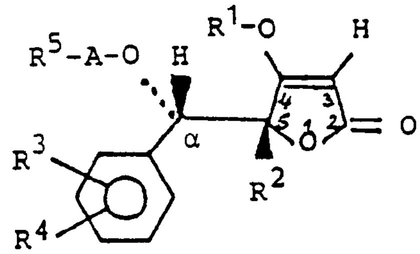

(VIIb)

worin $R^1$, $R^2$, $R^3$, $R^4$, A und $R^5$ die in Anspruch 1 genannten Bedeutungen besitzen, aber $R^5 \neq H$ ist, aufgetrennt wird.

11. Verfahren nach Anspruch 9 oder 10 zur Herstellung derjenigen Verbindungen der allgemeinen Formel I, worin $R^5 = H$, dadurch gekennzeichnet, daß

(C) der an das Stickstoffatom der Aminogruppe gebundene Rest $R^5$ der in Stufe (A) und/oder (B) erhaltenen Aminosäureester acidolytisch oder hydrogenolytisch abgespalten und durch ein Wasserstoffatom ersetzt wird, unter Bildung der eine freie Aminogruppe aufweisenden Aminosäureester der allgemeinen Formeln VIII und/oder VIIIa und VIIIb,

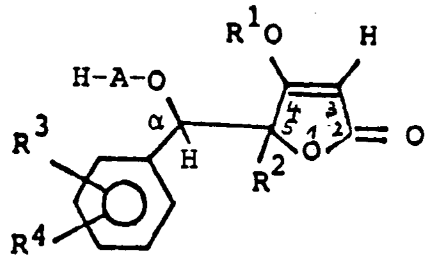

(VIII)

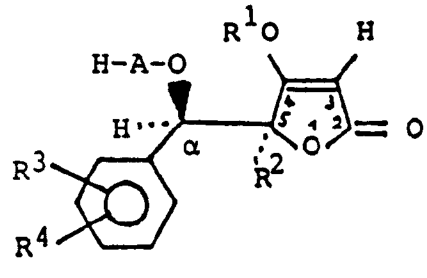

(VIIIa)

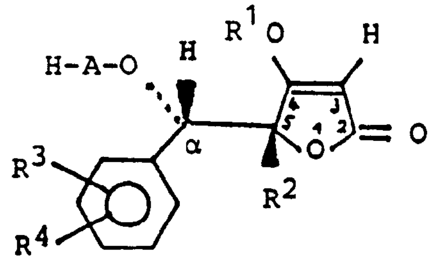

(VIIIb)

worin $R^1$, $R^2$, $R^3$ und $R^4$ die in Anspruch 1 und A die in Anspruch 9 genannten Bedeutungen besitzen.

12. Verfahren nach Anspruch 11, dadurch gekennzeichnet, daß

(D) die in Stufe (C) erhaltenen Diastereomerengemische in an sich bekannter Weise in die Diastereomeren der allgemeinen Formeln VIIIa und VIIIb aufgetrennt werden.

13. Verfahren nach Anspruch 12 zur Herstellung enantiomerer Verbindungen der allgemeinen Formel I, dadurch gekennzeichnet, daß

(E) die aus Stufe (C) und/oder (D) abgetrennten diastereomeren Ester der allgemeinen Formeln VIIIa und VIIIb getrennt voneinander unter Bildung der enantiomeren Furanone der allgemeinen Formeln IIa und IIb

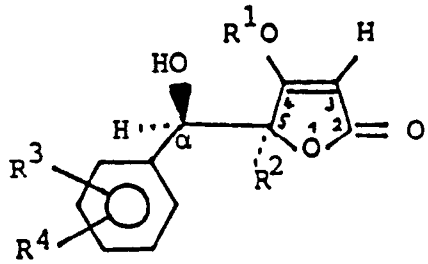

(IIa)

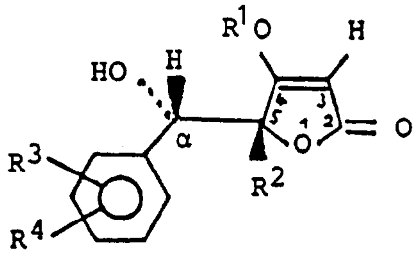

(IIb)

verseift werden und daß

(F) die so erhaltenen einzelnen enantiomeren Furanone der allgemeinen Formeln IIa und IIb analog zur Stufe (A) mit einem reaktiven Aminosäurederivat der allgemeinen Formel III

$$R^5\text{-A-X} \quad \text{(III)}$$

umgesetzt werden, worin $R^5$ und X die in Anspruch 8 angegebenen Bedeutungen besitzen und A den Acylrest der achiralen Aminosäuren Glycin, β-Alanin, 2-Methylalanin, 4-Aminobuttersäure oder einer 1-Amino-1-cycloalkancarbonsäure mit 3-7-gliedrigem Cycloalkylrest bedeutet, unter Bildung der entsprechenden enantiomeren Aminosäureester der allgemeinen Formeln VIIa und VIIb, worin 5 ≠ H ist.

14. Verfahren nach Anspruch 13 zur Herstellung derjenigen enantiomeren Verbindungen der allgemeinen Formel I, worin $R^5$ = H, dadurch gekennzeichnet, daß

(G) der Rest $R^5$ der enantiomeren Aminosäureester der allgemeinen Formeln VIIa und VIIb analog zur Stufe (C) acidolytisch oder hydrogenolytisch abgespalten und durch ein Wasserstoffatom ersetzt wird, unter Bildung der entsprechenden Aminosäureester der allgemeinen Formeln VIIIa und VIIIb, die eine freie Aminogruppe aufweisen.

15. Verfahren nach einem der Ansprüche 11, 12 oder 14 zur Herstellung derjenigen diastereomeren oder enantiomeren Verbindungen der allgemeinen Formel I, worin $R^5$ einen Niederalkanoylrest mit 2 bis 5 C-Atomen bedeutet, dadurch gekennzeichnet, daß man

(H) die eine freie Aminogruppe aufweisenden Aminosäureester der allgemeinen Formeln VIII, VIIIa oder VIIIb mit einem reaktiven Acylderivat der allgemeinen Formeln

$R^5$-Y oder $R^5$-O-$R^5$

umsetzt, worin Y ein Chlor- oder Bromatom und $R^5$ einen Niederalkanoyl-Rest mit 2 bis 5 C-Atomen bedeuten, oder mit einem Keten der Formel $R^7$=C=O umsetzt, worin $R^7$ einen Alkylidenrest mit 1 bis 4 C-Atomen bedeutet.

16. Verfahren nach einem der Ansprüche 8 bis 15, dadurch gekennzeichnet, daß in Stufe (A) oder (F) ein reaktives Aminosäurederivat der allgemeinen Formel III

$R^5$-A-X (III)

verwendet wird, worin A und X die in Anspruch 8 genannten Bedeutungen besitzen und $R^5$ eine von (Z) und (BOC) verschiedene, sonstige, in der Peptidchemie gebräuchliche, sauer oder hydrogenolytisch abspaltbare Schutzgruppe für Aminogruppen von Aminosäuren bedeutet, und daß diese sonstige Schutzgruppe in Stufe (C) oder (G) wieder abgespalten und durch ein Wasserstoffatom ersetzt wird.

17. Verfahren nach Anspruch 16, dadurch gekennzeichnet, daß anschließend an die in Stufe (C) oder (G) erfolgende Abspaltung der Schutzgruppe ein anderer Rest $R^5$ analog Stufe (H) eingeführt wird.

18. Verfahren zur Herstellung von Aminosäureestern der allgemeinen Formel I

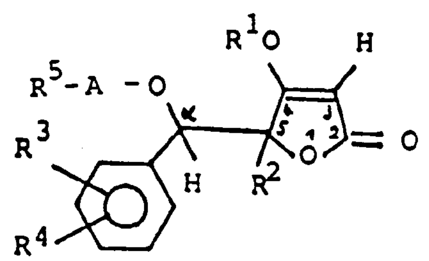

(I)

worin die Sauerstoffatome an C-5 und C-α relativ zueinander die threo-Position einnehmen und worin $R^1$, $R^2$, $R^3$, $R^4$, A und $R^5$ die in Anspruch 1 genannten Bedeutungen besitzen, dadurch gekennzeichnet, daß

(A) ein threo-4-Alkoxy-5-arylhydroxymethyl-2(5H)-furanon der allgemeinen Formel II,

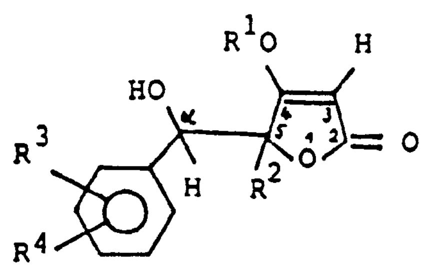

(II)

worin $R^1$, $R^2$, $R^3$ und $R^4$ die in Anspruch 1 genannten Bedeutungen besitzen, mit einem reaktiven Aminosäurederivat der allgemeinen Formel III

$R^5$-A-X (III)

umgesetzt wird, worin A und $R^5$ die in Anspruch 1 genannten Bedeutungen besitzen und worin X OH, Cl, Br oder die Gruppe O-A-$R^5$ ist,

unter Bildung eines Diastereomerengemischs des entsprechenden Aminosäureesters der allgemeinen Formel I.

19. Verfahren nach Anspruch 18, dadurch gekennzeichnet, daß, falls $R^5 \neq H$, der an das Stickstoffatom der Aminogruppe gebundene Rest $R^5$ der in Stufe (A) erhaltenen Aminosäureester analog zur Stufe (C) acidolytisch oder hydrogenolytisch abgespalten und durch ein Wasserstoffatom ersetzt wird, unter Bildung der entsprechenden, eine freie Aminogruppe aufweisenden Aminosäureester der allgemeinen Formel VIII.

20. Verfahren nach Anspruch 18 oder 19, dadurch gekennzeichnet, daß das in Stufe (A) entstandene Diastereomerengemisch der Aminosäureester der allgemeinen Formel I mit $R^5$ = H oder die in Stufe (C) entstandenen Aminosäureester der allgemeinen Formel VIII durch Umsetzung mit einem reaktiven Acylderivat der allgemeinen Formeln

$R^5$-Y oder $R^5$-O-$R^5$

worin Y ein Chlor- oder Bromatom und $R^5$ einen Niederalkanoyl-Rest mit 2 bis 5 C-Atomen bedeuten, oder mit einem Keten der Formel $R^7$=C=O, worin $R^7$ einen Alkylidenrest mit 1 bis 4 C-Atomen bedeutet, N-acyliert werden.

21. Verfahren nach einem der Ansprüche 8 bis 20, dadurch gekennzeichnet, daß man in Stufe (A) des Verfahrens die Veresterung in Gegenwart eines Carbodiimids als Kondensationsmittel, und in Gegenwart von Pyridin, 4-Dimethylaminopyridin und/oder 4-Pyrrolidinopyridin in einem wasserfreien aprotischen Lösungsmittel, ausgewählt aus der Gruppe Chloroform, Dichlormethan, niederaliphatischer oder cycloaliphatischer Ether, Dimethylformamid und Acetonitril, bei Temperaturen zwischen -30° und +30°C durchführt.

22. Arzneimittel, enthaltend eine oder mehrere der Verbindungen gemäß einem der Ansprüche 1 bis 7.

23. Verbindungen gemäß einem der Ansprüche 1 bis 7 zur Verwendung als therapeutische Wirkstoffe und bei der Behandlung von Konvulsionen und Erkrankungen des epileptischen Formenkreises bei warmblütigen Tieren und beim Menschen.

24. Verwendung der Verbindungen gemäß einem der Ansprüche 1 bis 7 bei der Herstellung von Antikonvulsiva und Antiepileptika.

## Claims

1. Amino acid esters of the general formula I

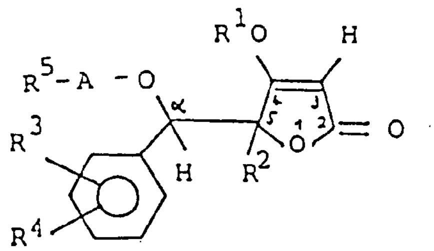

(I)

wherein the oxygen atoms at C-5 and C-α assume relatively to each other the threo position and wherein
$R^1$ is a methyl or ethyl group,
$R^2$ is a hydrogen atom or a methyl group,
$R^3$ and $R^4$ are independently from each other a hydrogen, fluorine, chlorine or bromine atom, a lower alkyl group having 1 to 3 C atoms, a perfluoro lower alkyl having 1 to 3 C atoms, the difluoromethoxy or nitro group,
A is the acyl radical of one of the following amino acids:

alanine, leucine, isoleucine, norleucine, valine, norvaline, phenylglycine, phenylalanine, proline, 5-oxoproline, glutamic acid, glutamine, asparaginic acid, asparagine, methionine, glycine, β-alanine, 4-aminobutyric acid,-2-methylalanine or a 1-amino-1-cycloalkane carboxylic acid having a 3-7-member cycloalkyl radical,

in each case a hydrogen atom of the amino group of said amino acids being replaced by the radical $R^5$, and

$R^5$ is a hydrogen atom, a lower alkanoyl radical having 2 to 5 C atoms, the benzyloxycarbonyl radical (Z) or the tert. butoxycarbonyl radical (BOC),
and pharmacologically acceptable acid addition salts thereof.

2. Amino acid esters according to claim 1 in the form of the racemates thereof.

3. Amino acid esters according to claim 1 in the form of the enantiomers thereof or in the form of enantiomer mixtures.

4. Amino acid esters according to claim 1 in the form of the diastereomers thereof or in the form of diastereomer mixtures.

5. Amino acid esters according to any one of claims 1 to 4, characterized in that one of the radicals $R^3$ or $R^4$ is a hydrogen atom and the other a fluorine, chlorine or bromine atom located in 2′-position or a methyl or trifluoromethyl group located in 2′-position.

6. Amino acid esters according to any one of claims 1 to 4, characterized in that one of the two radicals $R^3$

or $R^4$ is a fluorine, chlorine or bromine atom located in 2′-position or a trifluoromethyl group located in 2′-position and the other is a chlorine or bromine atom or a trifluoromethyl group, respectively in 4′, 5′ or 6′-position.

7. Amino acid esters according to any one of claims 1 to 6, characterized in that the alcoholic component thereof formed by the threo-5-arylhydroxymethyl-2(5H)-furanone radical is optically dextrorotary, considered on its own.

8. Process for the preparation of amino acid esters of the general formula I

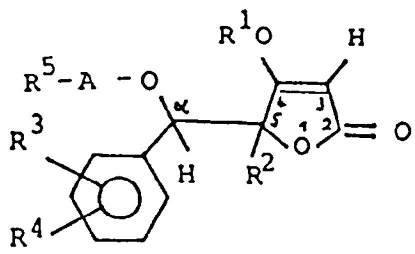

(I)

wherein the oxygen atoms at C-5 and C-α assume the threo position relatively to each other and wherein $R^1$, $R^2$, $R^3$, $R^4$, A and $R^5$ have the meanings given in claim 1 but $R^5 \neq H$, characterized in that

(A) a threo-4-alkoxy-5-arylhydroxymethyl-2(5H)-furanone of the general formula II

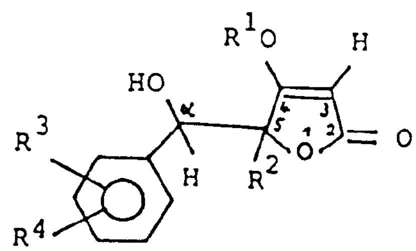

(II)

wherein $R^1$, $R^2$, $R^3$ and $R^4$ have the meanings defined in claim 1 is reacted with a reactive amino acid derivative of the general formula III

$R^5$-A-X (III)

wherein

A has the meaning defined in claim 1,

$R^5$ is a low alkanoyl radical having 2 to 5 C atoms, the benzyloxycarbonyl radical (Z) or the tert. butoxycarbonyl radical (BOC) and

X is OH, Cl, Br or the group O-A-$R^5$,

to form a diastereomer mixture of the corresponding amino acid ester of the general formula I.

9. Process according to claim 8, characterized in that in step (A) a reactive amino acid derivative of the general formula III

$R^5$-A-X (III)

is used, wherein $R^5$ and X have the meaning defined in claim 8 and A is an acyl radical of one of the chiral amino acids named in claim 1.

10. Process according to claim 9 for the preparation of amino acid esters of the general formula I, wherein $R^5 \neq H$, characterized in that,

(B) the diastereomer mixture obtained in step (A) is separated in a manner known per se into the diastereomers of the general formulae VIIa and VIIb,

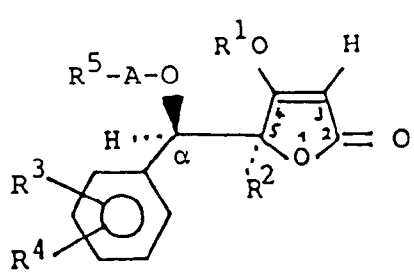

(VIIa)

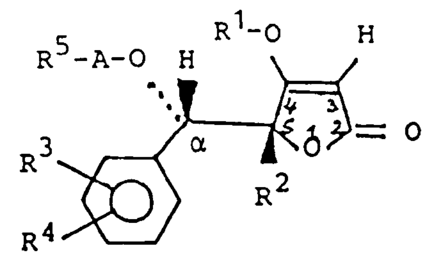

(VIIb)

wherein $R^1$, $R^2$, $R^3$,$R^4$, A and $R^5$ have the meanings defined in claim 1 but $R^5 \neq H$.

11. Process according to claim 9 or 10 for the preparation of the compounds of the general formula I in which $R^5$ = H, characterized in that

(C) the radical $R^5$ bonded to the nitrogen atom of the amino group, of the amino acid esters obtained in step (A) and/or (B) is split off acidolytically or hydrogenolytically and replaced by a hydrogen atom, forming the amino acid esters having a free amino group and being of the general formulae VIII and/or VIIIa and VIIIb,

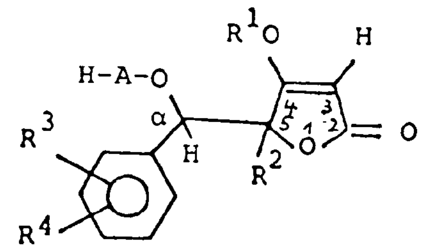

(VIII)

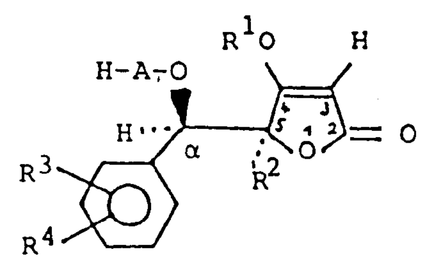

(VIIIa)

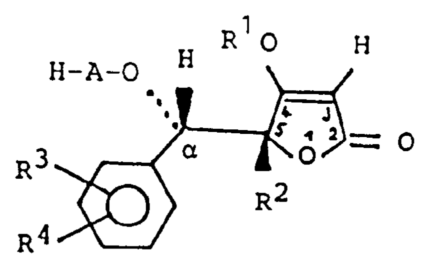

(VIIIb)

wherein $R^1$, $R^2$, $R^3$ and $R^4$ have the meanings defined in claim 1 and A the meaning defined in claim 9.

12. Process according to claim 11, characterized in that

(D) The diastereomer mixtures obtained in step (C) are separated in a manner known per se into the diastereomers of the general formulae VIIIa and VIIIb.

13. Process according to claim 12 for the preparation of enantiomeric compounds of the general formula I, characterized in that

(E) the diastereomeric esters of the general formulae VIIIa and VIIIb separated from step (C) and/or (D)

are saponified separately from each other to form the enantiomeric furanones of the general formulae IIa and IIb

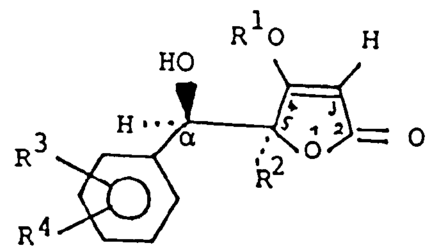

(IIa)

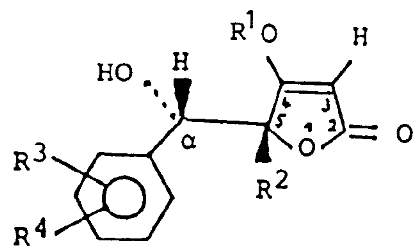

(IIb)

and that

(F) the individual enantiomeric furanones of the general formulae IIa and IIb thus obtained are reacted analogously to step (A) with a reactive amino acid derivative of the general formula III

$R^5$-A-X (III)

wherein $R^5$ and X have the meanings defined in claim 8 and A denotes the acyl radical of the achiral amino acids glycine, β-alanine, 2-methylalanine, 4-aminobutyric acid or a 1-amino-1-cycloalkane caboxylic acid having a 3-7-member cycloalkyl radical, to form the corresponding enantiomeric amino acid esters of the general formulae VIIa and VIIb, wherein $R^5 \neq H$.

14. Process according to claim 13 for the preparation of the enantiomeric compounds of the general formula I wherein $R^5$ = H, characterized in that

(G) the radical $R^5$ of the enantiomeric amino acid esters of the general formulae VIIa and VIIb are split off acidolytically or hydrogenolytically analogously to step (C) and replaced by a hydrogen atom to form the corresponding amino acid esters of the general formulae VIIIa and VIIIb which have a free amino group.

15. Process according to any one of claims 11, 12 or 14 for the preparation of the diastereomeric or enantiomeric compounds of the general formula I in which $R^5$ is a low alkanoyl radical having 2 to 5 C atoms, characterized in that

(H) the amino acid esters having a free amino group and being of the general formulae VIII, VIIIa or VIIIb are reacted with a reactive acyl derivative of the general formulae

$R^5$-Y or $R^5$-O-$R^5$ (III)

wherein Y is a chlorine or bromine atom and $R^5$ is a low alkanoyl radical having 2 to 5 C atoms, or reacted with a ketene of the formula $R^7$=C=O, wherein $R^7$ is an alkylidene radical having 1 to 4 C atoms.

16. Process according to any one of claims 8 to 15, characterized in that in step (A) or (F) a reactive amino acid derivative of the general formula III

$R^5$-A-X (III)

is used, wherein A and X have the meanings defined in claim 8 and $R^5$ denotes a protective group different from (Z) and (BOC) and which is otherwise usual in peptide chemistry and can be split off acidically or hydrogenolytically, for amino groups of amino acids, and that said other protective group is again split off in step (C) or (G) and replaced by a hydrogen atom.

17. Process according to claim 16, characterized in that following the splitting off of the protective group in step (C) or (G) another radical $R^5$ is introduced analogously to step (H).

18. Process for the preparation of amino acid esters of the general formula I

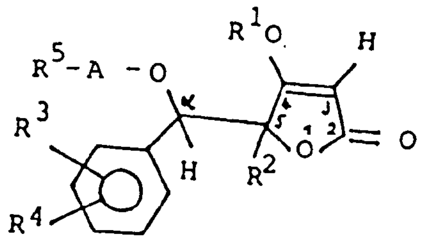

(I)

wherein the oxygen atoms at C-5 and C-α assume the threo position relatively to each other and wherein $R^1$, $R^2$, $R^3$, $R^4$, A and $R^5$ have the meanings defined in claim 1, characterized in that

(A) a threo-4-alkoxy-5-arylhydroxymethyl-2(5H)-furanone of the general formula II,

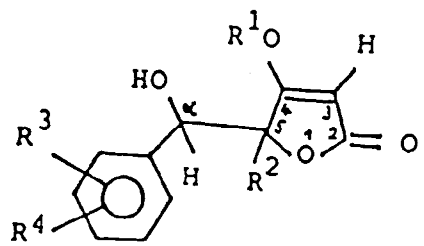

(II)

wherein $R^1$, $R^2$, $R^3$ and $R^4$ have the meanings defined in claim 1 is reacted with a reactive amino acid derivative of the general formula III

$R^5$-A-X (III)

wherein A and $R^5$ have the meanings defined in claim 1 and X is OH, Cl, Br or the group O-A-$R^5$, to form a diastereomer mixture of the corresponding amino acid ester of the general formula I.

19. Process according to claim 18, characterized in that if $R^5 \neq$ H the radical $R^5$, bonded to the nitrogen atom of the amino group, of the amino acid esters obtained in step (A) is split off acidolytically or hydrogenolytically analogously to step (C) and replaced by a hydrogen atom to form the corresponding amino acid esters having a free amino group and the general formula VIII.

20. Process according to claim 18 or 19, characterized in that the diastereomer mixture of the amino acid esters obtained in step (A) and having the general formula I with $R^5$ = H or the amino acid esters obtained in step (C) and having the general formula VIII are N-acylated by reaction with a reactive acyl derivative of the general formulae

$R^5$-Y or $R^5$-O-$R^5$

wherein Y is a chlorine or bromine atom and $R^5$ a low alkanoyl radical having 2 to 5 C atoms, or with a ketene of the formula $R^7$=C=O, wherein $R^7$ denotes an alkylidene radical having 1 to 4 C atoms.

21. Process according to any one of claims 8 to 20, characterized in that in step (A) of the process the esterification is carried out in the presence of a carbodiimide as condensing agent and in the presence of pyridine, 4-dimethylaminopyridine and/or 4-pyrrolidinopyridine in an anhydrous aprotic solvent, selected from the group chloroform, dichloromethane, low aliphatic or cycloaliphatic ethers, dimethylformamide and acetonitrile, at temperatures between -30° and +30°C.

22. Pharmaceutical preparation containing one or more of the compounds according to any one of claims 1 to 7.

23. Compounds according to any one of claims 1 to 7 for use as therapeutic agents and in the treatment of convulsions and diseases of the epileptic group in warm-blooded animals and in humans.

24. Use of the compounds according to any one of claims 1 to 7 in the preparation of anticonvulsives and antiepileptics.

## Revendications

1. Esters d'acides aminés de formule générale I,

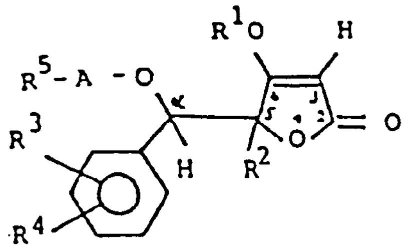

(I)

où les atomes d'oxygène en C-5 et C-α prennent l'un par rapport à l'autre la position thréo et où
$R^1$ représente un groupe méthyle ou éthyle,
$R^2$ représente un atome d'hydrogène ou un groupe méthyle,
$R^3$ et $R^4$ représentent indépendamment l'un de l'autre un atome d'hydrogène, de fluor, de chlore ou de brome, un groupe alcoyle inférieur en $C_1$ à $C_3$, un groupe perfluoroalcoyle inférieur en $C_1$ à $C_3$, le groupe difluorométhoxy ou le groupe nitro,
A représente le radical acyle d'un des acides aminés suivants:
alanine, leucine, isoleucine, norleucine, valine, norvaline, phénylglycine, phénylalanine, proline, 5-oxoproline, acide glutamique, glutamine, acide aspartique, asparagine, méthionine, glycine, β-alanine, acide 4-aminobutyrique, 2-méthylalanine ou un acide 1-amino-1-cycloalcanecarboxylique ayant un radical cycloalcoyle à 3 à 7 chaînons,
où à chaque fois un atome d'hydrogène de ces acides aminés est remplacé par le radical $R^5$ , et
$R^5$ représente un atome d'hydrogène, un radical alcanoyle inférieur en $C_2$ à $C_5$ , le radical benzyloxycarbonyle (Z) ou le radical tert.-butoxycarbonyle (BOC),
ainsi que leurs sels d'addition d'acides pharmacologiquement acceptables.

2. Esters d'acides aminés selon la revendication 1 sous la forme de leurs racémates.

3. Esters d'acides aminés selon la revendication 1 sous la forme de leurs énantiomères ou sous la forme de mélanges d'énantiomères.

4. Esters d'acides aminés selon la revendication 1 sous la forme de leurs diastéréomères ou sous forme de mélanges de diastéréomères.

5. Esters d'acides aminés selon l'une des revendications 1 à 4, caractérisés en ce que l'un des radicaux $R^3$ ou $R^4$ est un atome d'hydrogène et l'autre un atome de fluor, de chlore ou de brome se trouvant en position 2′ ou un groupe méthyle ou trifluorométhyle se trouvant en position 2′.

6. Esters d'acides aminés selon l'une des revendications 1 à 4, caractérisés en ce que l'un des deux radicaux $R^3$ ou $R^4$ est un atome de fluor, de chlore ou de brome se trouvant en position 2′ et l'autre est un atome de chlore ou de brome ou un groupe trifluorométhyle, à chaque fois en position 4′, 5′ ou 6′.

7. Esters d'acides aminés selon l'une des revendications 1 à 6, caractérisés en ce que leur composant alcoolique constitué par le radical thréo-5-aryl-hydroxyméthyl-2(5H)-furanone, considéré seul, est dextrogyre.

8. Procédé de préparation d'esters d'acides aminés de formule générale I,

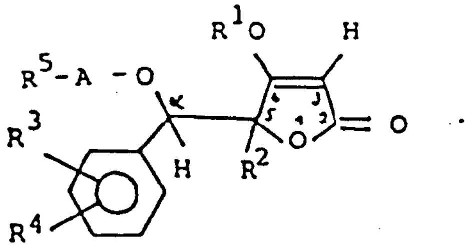

(I)

où les atomes d'oxygène en C-5 et C-α prennent l'un par rapport à l'autre la position thréo et où $R^1$, $R^2$, $R^3$, $R^4$, A et $R^5$ ont les significations mentionnées dans la revendication 1, mais $R^5 \neq H$, caractérisé en ce que
(A) on fait réagir une thréo-4-alcoxy-5-arylhydroxyméthyl-2(5H)-furanone de formule générale II,

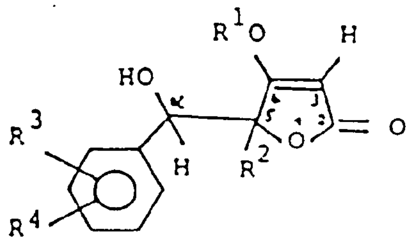

(II)

où $R^1$, $R^2$, $R^3$ et $R^4$ ont les significations mentionnées dans la revendication 1, avec un dérivé d'acide aminé réactif de formule générale III

$R^5$-A-X (III)

où

A a la signification donnée dans la revendication 1,

$R^5$ représente un radical alcanoyle inférieur en $C_2$ à $C_5$, le radical benzyloxycarbonyle (Z) ou le radical tert.-butoxycarbonyle (BOC) et

X représente OH, Cl, Br ou le groupe O-A-$R^5$,

avec formation d'un mélange de diastéréomères de l'ester d'acide aminé correspondant de formule générale I.

9. Procédé selon la revendication 8, caractérisé en ce que dans l'étape (A) on utilise un dérivé d'acide aminé réactif de formule générale III

$R^5$-A-X (III)

où $R^5$ et X ont la signification donnée dans la revendication 8 et A représente un radical acyle d'un des acides aminés chiraux mentionnés dans la revendication 1.

10. Procédé selon la revendication 9 de préparation des esters d'acides aminés de formule générale I, où $R^5 \neq H$, caractérisé en ce que

(B) on sépare de façon connue le mélange de diastéréomères obtenu dans l'étape (A) pour donner les diastéréomères de formules générales VIIa et VIIb,

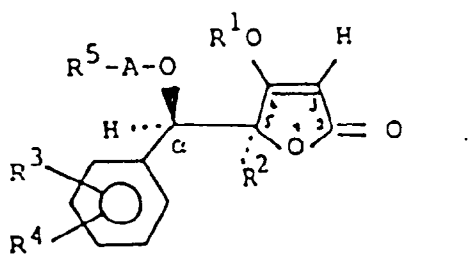

(VIIa)

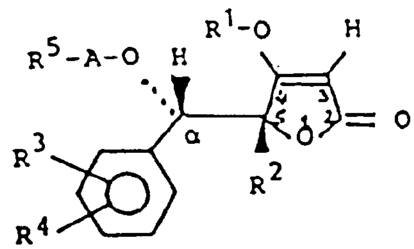

(VIIb)

où $R^1$, $R^2$, $R^3$, $R^4$, A et $R^5$ ont les significations données dans la revendication 1, mais $R^5 \neq H$.

11. Procédé selon la revendication 9 ou 10 de préparation des composés de formule générale I, où $R^5$ = H, caractérisé en ce que

(C) on sépare par acidolyse ou hydrogénolyse le radical $R^5$ lié à l'atome d'azote du groupe amino des esters d'acides aminés obtenus dans l'étape (A) et/ou (B) et on le remplace par un atome d'hydrogène, avec formation des esters d'acides aminés de formules générales VIII et/ou VIIIa et VIIIb présentant un groupe amino libre,

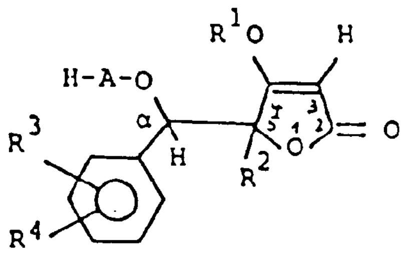

(VIII)

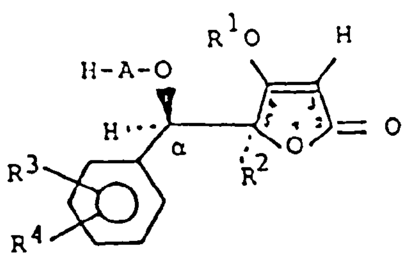

(VIIIa)

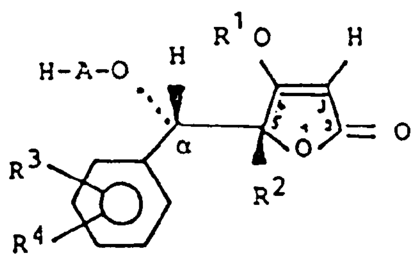

(VIIIb)

où $R^1$, $R^2$, $R^3$ et $R^4$ ont la signification donnée dans la revendication 1 et A a la signification donnée dans la revendication 9.

12. Procédé selon la revendication 11, caractérisé en ce que

(D) on sépare de façon connue les mélanges de diastéréomères obtenus dans l'étape (C) pour donner les diastéréomères de formules générales VIIIa et VIIIb.

13. Procédé selon la revendication 12 de préparation de composés énantiomères de formule générale I, caractérisé en ce que

(E) on saponifie séparément l'un de l'autre les esters diastéréomères de formules générales VIIIa et VIIIb séparés dans l'étape (C) et/ou (D) avec formation des furanones énantiomères de formules générales IIa et IIb

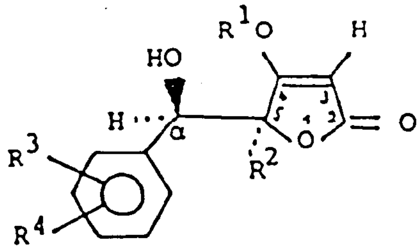

(IIa)

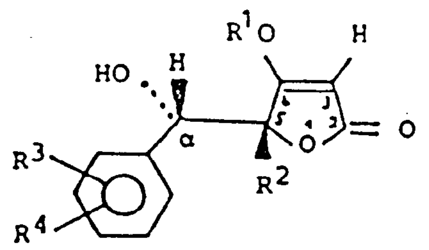

(IIb)

et en ce que

(F) on fait réagir les furanones énantiomères de formules générales IIa et IIb isolées ainsi obtenues, de manière analogue à l'étape (A) avec un dérivé d'acide aminé réactif de formule générale III

$R^5$-A-X (III)

où $R^5$ et X ont les significations données dans la revendication 8 et A représente le radical acyle des acides aminés achiraux glycine, β-alanine, 2-méthyl-alanine, acide 4-aminobutyrique, ou d'un acide 1-amino-1-cycloalcanecarboxylique ayant un radical cycloalcoyle à 3 à 7 chaînons, avec formation des esters d'acides aminés énantiomères correspondants de formules générales VIIa et VIIb, où $R^5 \neq$ H.

14. Procédé selon la revendication 13 de préparation des composés énantiomères de formule générale I où $R^5$ = H, caractérisé en ce que

(G) on sépare par acidolyse ou hydrogénolyse le radical $R^5$ des esters d'acides aminés énantiomères de formules générales VIIa et VIIb, de manière analogue à l'étape (C), et on le remplace par un atome d'hydrogène, avec formation des esters d'acides aminés correspondants de formules générales VIIIa et VIIIb qui présentent un groupe amino libre.

15. Procédé selon l'une des revendications 11, 12 ou 14 de préparation des composés diastéréomères ou énantiomères de formule générale I où $R^5$ représente un radical alcanoyle inférieur en $C_2$ à $C_5$ , caractérisé en ce que

(H) on fait réagir les esters d'acides aminés de formules générales VIII, VIIIa ou VIIIb présentant un groupe amino libre avec un dérivé acyle réactif de formules générales

$R^5$-Y ou $R^5$-O-$R^5$

où Y représente un atome de chlore ou de brome et $R^5$ représente un radical alcanoyle inférieur en $C_2$ à $C_5$, ou avec un cétène de formule $R^7$=C=0 où $R^7$ représente un radical alcoylidène en $C_1$ à $C_4$.

16. Procédé selon l'une des revendications 8 à 15, caractérisé en ce que dans l'étape (A) ou (F) on utilise un dérivé d'acide aminé réactif de formule générale III

$R^5$-A-X (III)

où A et X ont les significations données dans la revendication 8 et $R^5$ représente un groupe protecteur pour groupes amino d'acides aminés particulier, habituel en chimie des peptides, différent de (Z) et (BOC), séparable par acidolyse ou hydrogénolyse, et en ce que ces groupes particuliers sont à nouveau séparés dans l'étape -(C) ou (G) et remplacés par un atome d'hydrogène.

17. Procédé selon la revendication 16, caractérisé en ce qu'après la séparation du groupe protecteur réalisée dans l'étape (C) ou (G) on introduit un autre radical $R^5$ de manière analogue à l'étape (H).

18. Procédé de préparation d'esters d'acides aminés de formule générale I

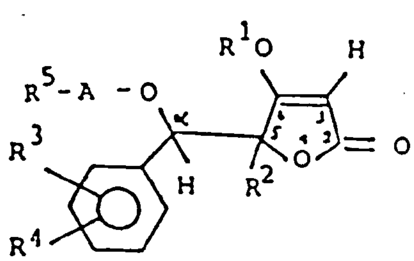

(I)

où les atomes d'oxygène en C-5 et C-α prennent la position thréo l'un par rapport à l'autre et où $R^1$, $R^2$, $R^3$, $R^4$ , A et $R^5$ ont les significations données dans la revendication 1, caractérisé en ce que

(A) on fait réagir une thréo-4-alcoxy-5-arylhydroxyméthy1-2(5H)-furanone de formule générale II,

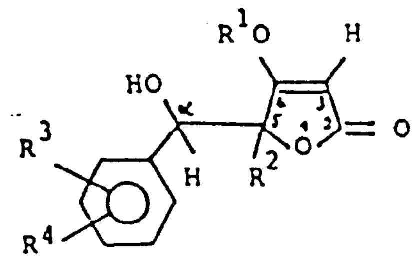

(II)

où $R^1$, $R^2$, $R^3$ et $R^4$ ont les significations données dans la revendication 1, avec un dérivé d'acide aminé réactif de formule générale III

$$R^5\text{-A-X} \quad \text{(III)}$$

où A et $R^5$ ont les significations données dans la revendication 1 et où X représente OH, Cl, Br ou le groupe O-A-$R^5$,
avec formation d'un mélange de diastéréomères de l'ester d'acide aminé correspondant de formule générale I.

19. Procédé selon la revendication 18, caractérisé en ce que, si $R^5 \neq H$, le radical $R^5$ de l'ester d'acide aminé obtenu dans l'étape (A), lié à l'atome d'azote du groupe amino, est séparé par acidolyse ou par hydrolyse de manière analoggue à l'étape (C) et remplacé par un atome d'hydrogène, avec formation des esters d'acides aminés de formule générale VIII correspondants présentant un groupe amino libre.

20. Procédé selon la revendication 18 ou 19, caractérisé en ce qu'on N-acyle le mélange de diastéréomères des esters d'acides aminés de formule générale I obtenu dans l'étape (A), où $R^5$ = H, ou les esters d'acides aminés de formule générale VIII obtenus dans l'étape (C), par réaction avec un dérivé d'acyle réactif de formules générales

$$R^5\text{-Y} \quad \text{ou} \quad R^5\text{-O-}R^5$$

où Y représente un atome de chlore ou de brome et $R^5$ représente un radical alcanoyle inférieur en $C_2$ à $C_5$, ou avec un cétène de formule $R^7$=C=O , où $R^7$ représente un radical alcoylidène en $C_1$ à $C_4$ .

21. Procédé selon l'une des revendications 8 à 20, caractérisé en ce qu'on effectue dans l'étape (A) du procédé l'estérification en présence d'un carbodiimide comme agent de condensation, et en présence de pyridine, 4-diméthylaminopyridine et/ou 4-pyrrolidinopyridine dans un solvant aprotique anhydre, choisi dans, le groupe constitué par le chloroforme, le dichlorométhane, un éther aliphatique inférieur ou un éther cycloaliphatique, le diméthylformamide et l'acétonitrile, à des températures comprises entre -30° et +30°.

22. Médicament contenant un ou plusieurs des composés selon l'une des revendications 1 à 7.

23. Composés selon l'une des revendications 1 à 7 destinés à l'application comme substances actives thérapeutiques et dans le traitement des convulsions et des maladies de type,épileptique chez les animaux homéothermes et chez l'homme.

24. Application des composés selon l'une des revendications 1 à 7 à la préparation d'agents anticonvulsifs et antiépileptiques.